# EUROPEAN PATENT APPLICATION

(11) **EP 2 116 603 A2**
(43) Date of publication of application: **11.11.2009**
(21) Application number: 09075385.6
(22) Date of filing: 14.11.2005
(51) Int. Cl.: C12N 15/11, C12Q 1/66, C12Q 1/18, C07K 7/08, C07K 7/06, A61P 31/04, A61K 38/10, A61K 38/08

(54) **Antimicrobial peptides**

(30) Priority: 12.11.2004 US 627356 P
(62) Divisional of application: 05803322.6
(71) Applicant: The University Of British Columbia, Vancover, BC V6T 1Z3 (CA)
(72) Inventor: Hancock, Robert E. W., Vancouver British Columbia V6J 4B3 (CA); Hilpert, Kai, Vancouver British Columbia V5V 1Z4 (CA)
(74) Representative: Goodfellow, Hugh Robin

(57) **Abstract**

A novel class of peptides having antimicrobial activity is provided. Also provided are methods for inhibiting the growth of bacteria utilizing the peptides of the invention. Pharmaceutical compositions comprising the novel class of peptides are also provided.

## Description

### CROSS-REFERENCE TO RELATED APPLICATIONS

This application claims priority, under 35 USC § 119(e), to U.S. Application No. 60/627,356, filed November 12, 2004, the disclosure of which is incorporated by reference in its entirety.

### FIELD

The present invention relates generally to peptides and more specifically to antimicrobial peptides.

### BACKGROUND

The treatment of bacterial infections with antibiotics is one of the mainstays of human medicine. Unfortunately the effectiveness of antibiotics has become limited due to an increase in bacterial antibiotic resistance in the face of a dearth of discovery of new classes of antibiotics. Today, nosocomial bacterial infections that are resistant to therapy lead to costs of more than $2 billion, and account for more than 80,000 direct and indirect deaths in North America.

A major limitation in antibiotic development has been the difficulty in finding new structures with the same assets as conventional antibiotics, namely low toxicity for the host and a broad action against bacterial pathogens. Recent novel antibiotic classes, including the oxazolidinones (linezolid), the streptogramins (synercid) and the glycolipids (daptomycin) have all been limited in their spectrum of activity to Gram-positive pathogens. It is therefore a difficult challenge for scientists to design antibiotics with novel structures and/or modes of action.

Cationic antimicrobial peptides represent good templates for a new generation of antimicrobials. They kill both Gram negative and Gram positive microorganisms rapidly and directly, do not easily select mutants, work against common clinically-resistant bacteria such as methicillin-resistant *Staphylococcus aureus* (MRSA) and vancomycin resistant *Enterococcus* (VRE), show a synergistic effect with conventional antibiotics, and can often activate host innate immunity without displaying immunogenicity. Moreover, they seem to counteract some of the more harmful aspects of inflammation (*e*.*g*., sepsis, endotoxaemia), which is extremely important since rapid killing of bacteria and subsequent liberation of bacterial components such as LPS or peptidoglycan can induce fatal immune dysregulation (Jarisch-Herxheimer reaction).

Cationic antimicrobial peptides comprising sequences of natural L-amino acids were discovered in the hemolymph of insects in the late 1970s. Today, more than 600 cationic peptides have been described in bacteria, fungi, insects, tunicates, amphibians, crustaceans, birds, fish and mammals including humans. They can be described through their physical chemical characteristics with a size ranging from 12 to 50 amino acids, a net positive charge exceeding +2, due to excess arginine and lysine residues, and approximately 50% hydrophobic amino acids. The multitude of cationic peptide sources, structures and spectra of activity is matched by a number of complex and controversial models attempting to describe and explain the modes of action of these peptides. Most antimicrobial peptides bind to the lipopolysaccharide (LPS) of Gram-negative bacteria or to lipoteichoic acid of Gram-positives, and subsequently associate with and either permeabilize the cytoplasmic membrane or cross that membrane and act on internal targets. The precise mechanisms as to how they bring about death in target cells are not fully understood to date.

Recently, cationic peptides containing a disulphide bond forming a looped structure were identified. One member of this group, bactenecin (*i*.*e*., dodecapeptide), is a twelve amino acid peptide isolated from bovine neutrophils. Bactenecin is the smallest known cationic antimicrobial peptide. Two cysteine residues form a disulphide bond to make bactenecin a loop molecule. This peptide is active against both Gram negative (*E. coli, P. aeruginosa*) and Gram positive bacteria (*S. pyogenes, C. xerosis*). It was demonstrated that the linear variant Bac2A shows a similar activity against Gram-negative bacteria and an improved activity against Gram-positive bacteria. These features, its small size, linearity and activity against both Gram-positive and Gram-negative bacteria make this peptide an ideal candidate for semi-random design methods such as spot peptide synthesis on cellulose membranes.

There is a need to develop peptides having a broad range of potent antimicrobial activity against a plurality of microorganisms, including Gram negative bacteria, Gram positive bacteria, fungi, protozoa, viruses and the like.

### SUMMARY

The present invention generally relates to peptides, and more specifically to antimicrobial peptides, analogs, derivatives, amidated variations and conservative variations thereof that have antimicrobial activity against a plurality of microorganisms, including Gram-negative bacteria, Gram-positive bacteria, fungi, protozoa and the like. The present invention provides peptide-based compositions, peptide variant compositions, and peptide mimetic compositions that inhibit, prevent, or destroy the growth or proliferation of microbes such as bacteria, fungi, protozoa, viruses, parasites and the like, and are, therefore, useful in a variety of therapeutic applications as well as in other applications, including protecting objects from bacterial colonization. The therapeutic applications include the treatment of microbial related diseases and conditions wherein the amount of peptide used is of sufficient quantity to decrease the numbers of bacteria, viruses, fungi, and parasites in the body of a subject. The present invention also provides polypeptide compositions, functional variants, and peptide mimetics thereof.

The present invention is based on the discovery that certain peptides originally identified from bactenecin have antimicrobial activity. Exemplary peptides of the invention include peptides having the amino acid sequences of SEQ ID NOS: 2-2166, and analogs, derivatives, amidated variations and conservative variations thereof.

Accordingly, the present invention provides methods for treating microbial diseases, disorders and conditions by administering therapeutic compounds, *e*.*g*., pharmaceutical compositions comprising one or more antimicrobial peptides or proteins of the invention, to a subject.

The invention also provides a method of inhibiting the growth of bacteria including contacting the bacteria with an inhibiting effective amount of at least one peptide of the invention alone, or in combination with at least one antibiotic. Classes of antibiotics that can be used in synergistic therapy with the peptides of the invention include, but are not limited to, aminoglycoside, penicillin, cephalosporin, fluoroquinolone, carbepenem, tetracycline and macrolide.

The invention provides polynucleotides that encode the peptides of the invention. Exemplary polynucleotides encode peptides having the amino acid sequences of SEQ ID NOS: 2-2166, and analogs, derivatives and conservative variations thereof.

In one aspect, the invention provides an isolated antimicrobial peptide having 8 to 12 amino acids, wherein the peptide has an amino acid sequence of SEQ ID NOS: 1-2166, or analogs, derivatives, amidated variations and conservative variations thereof. In some embodiments, an isolated polynucleotide encodes such peptides. In other embodiments, the peptide comprises any contiguous sequence of amino acids having the formula: R₁-L₂-A₃-R₄-I₅-V₆-V₇-I₈-R₉-V₁₀-A₁₁-R₁₂. wherein R₁ = R or W; L₂ = L, C, G, H, K, R, S, W, or Y; A₃ = A, C, F, H, I, K, L, Q, R, or W; I₅ =I, C, R, or W; V₆ =V, C, F, or W; V₇ = V, C, H, I, K, N, Q, R, or T; I₈ = I or C; R₉ =R or C; V₁₀ = V, C, or W; A₁₁ = A, C, G, H, I, K, L, M, R, S, or Y, and derivatives, substitutions, deletions and additions thereof. In some embodiments, the peptide has an amino acid sequence having the formula: AA₁ - AA₂ - AA₃ - AA₄ - AA₅ - AA₆ - AA₇ - AA₈ - AA₉ - AA₁₀ - AA₁₁ - AA₁₂, wherein AA₁ = A, G, I, K, L, P, R, or W; AA₂ = any residue except D, E, M, or N; AA₃ = any residue; AA₄ = K, M, or R; AA₅ = C, I, K, R, V, or W; AA₆ = C, F, K, R, V, W, or Y; AA₇ = C, F, G, H, I, K, L, N, Q, R, T, V, or Y; AA₈ = C, F, I, K, R, V, W, or Y; AA₉ = C, K, or R; AA₁₀ = C, I, K, L, R, V, W, or Y; AA₁₁ = any residue except D, E, or P; AA₁₂ = A, or R, and derivatives, substitutions, deletions and additions thereof. In other embodiments, the peptide has a sequence of 8 amino acids having the formula: AA₁ - AA₂ - AA₃ - V - I - AA₆ - AA₇ - R, wherein AA₁ = K or R; AA₂ = I or R; AA₃ = W or V; AA₆ = R or W; and AA₇ = R or W.

In another aspect, the invention provides a polypeptide X₁- A -X₂ or a functional variant or mimetic thereof, wherein A represents at least one peptide having an amino acid sequence of SEQ ID NOS: 1-2166 or analogs, derivatives, amidated variations and conservative variations thereof; and wherein each X₁ and X₂ independently of one another represents any amino acid sequence of n amino acids, n varying from 0 to 50, and n being identical or different in X₁ and X₂. In some embodiments, the functional variant or mimetic is a conservative amino acid substitution or peptide mimetic substitution. In other embodiments, the functional variant has about 70% or greater amino acid identity to X₁- A -X₂. In some embodiments, n is zero.

In another aspect, the invention provides a method of inhibiting the growth of bacteria comprising contacting the bacteria with an inhibiting effective amount of a peptide having an amino acid sequence of SEQ ID NOS: 2-2166, or any combination thereof, or analogs, derivatives, amidated variations and conservative variations thereof, with the proviso that the peptide having an amino acid sequence of SEQ ID NO: 1 is only used in combination with any peptide having an amino acid sequence of SEQ ID NO: 2-2166. In some embodiments, the contacting comprises a peptide in combination with at least one antibiotic or lysozome. In other embodiments, the antibiotic is selected from the group consisting of aminoglycosides, penicillins, cephalosporins, carbapenems, monobactams, quinolones, tetracyclines, and glycopeptides. In some embodiments, antibiotic is selected from the group consisting of amikacin, gentamicin, kanamycin, netilmicin, tobramycin, streptomycin, azithromycin, clarithromycin, erythromycin, erythromycin estolate/ethyl-succinate/gluceptate/lactobionate/stearate, penicillin G, penicillin V, methicillin, nafcillin, oxacillin, cloxacillin, dicloxacillin, ampicillin, amoxicillin, ticarcillin, carbenicillin, mezlocillin, azlocillin, piperacillin, cephalothin, cefazolin, cefaclor, cefamandole, cefoxitin, cefuroxime, cefonicid, cefmetazole, cefotetan, cefprozil, loracarbef, cefetamet, cefoperazone, cefotaxime, ceftizoxime, ceftriaxone, ceftazidime, cefepime, cefixime, cefpodoxime, cefsulodin, imipenem, aztreonam, fleroxacin, nalidixic acid, norfloxacin, ciprofloxacin, ofloxacin, enoxacin, lomefloxacin, cinoxacin, doxycycline, minocycline, tetracycline, vancomycin, chloramphenicol, clindamycin, trimethoprim, sulfamethoxazole, nitrofurantoin, rifampin and mupirocin and teicoplanin. In some embodiments, the bacteria is Gram positive. In some such embodiments, the bacteria is *Staphylococcus aureus, Staphylococcus epiderimidis*, or *Enterococcus faecaelis*. In other embodiments, the bacteria Gram negative. In some such embodiments, the bacteria is *Pseudomonas aeruginosa, Escherichia coli*, or *Salmonella enteritidis* ssp Typhimurium. In other embodiments, the peptide is covalently bound to a solid support.

In another aspect, the invention provides a method of identifying an antimicrobial peptide having 8 to 12 amino acids that is derived from Bac2A. The method includes contacting a test peptide with a microbe under conditions sufficient for antimicrobial activity, and detecting a change in growth or proliferation of the microbe as compared to the growth or proliferation of the microbe prior to contacting with the test peptide. In one embodiment, the peptide is synthesized in a multi-spot format on a solid support. The peptides of the invention will retain antimicrobial activity when cleaved from the solid support or retain activity when still associated with the solid support. In another embodiment, the peptide has a sequence of 12 amino acids including a consecutive stretch of 5 or more hydrophobic amino acid residues. The microbe can be a Gram negative bacterium, such as *Pseudomonas aeruginosa, Escherichia coli*, or *Salmonella enteritidis* ssp Typhimurium. In another embodiment, the microbe can be a Gram positive bacterium, such as *Staphylococcus aureus, Staphylococcus epidermidis*, or *Enterococcus faecaelis*. In yet another embodiment, the microbe can be a yeast, such as *Candida albicans*. The detection can include detecting luminescence in a microtiter plate luminescence reader over time. In this embodiment, the microbe contains a reporter system, such as a bacterial luciferase construct inserted into the chromosome. For example, the bacterial luciferase construct is inserted into the *fliC* gene in *Pseudomonas aeruginosa*.

In another aspect, the invention provides a pharmaceutical composition comprising the peptide(s) or polypeptide(s) of the invention and a pharmaceutically acceptable carrier.

In another aspect, the invention provides a method of modulating microbial activity in a subject, comprising administering to the subject a therapeutically effective amount of a pharmaceutical composition comprising the peptide(s) or polypeptide(s) of the invention and a pharmaceutically acceptable carrier.

In another aspect, the invention provides a method of treating a disease or disorder in a subject associated with microbial activity comprising administering to the subject a therapeutically effective amount of a pharmaceutical composition comprising the peptide(s) or polypeptide(s) of the invention and a pharmaceutically acceptable carrier. In some embodiments, the method of treating comprises the pharmaceutical composition of the invention in combination or in conjunction with other drugs or agents that can be used to for preventing or treating disease or disorder in a subject or organism.

In another aspect, the invention provides a method of protecting medical devices from colonization with pathogenic bacteria by coating at least one peptide of the invention on the surface of the medical device.

### BRIEF DESCRIPTION OF THE DRAWINGS

Fig. 1A is a graphical representation showing the results of a killing assay of *Pseudomonas aeruginosa* strain H1001 using luciferase as a reporter of residual ATP levels in the cell. These data demonstrate that the rate of killing (loss of luciferase activity as revealed by decreased light output in real time) is proportional to the amount of peptide added to the cells.

Fig. 1B is a graphical representation showing a conventional killing curve measuring residual colony counts by plating survivors on nutrient agar and growing overnight at 37oC.

Fig. 2 is a graphical representation showing the detection of the luminescence of *Pseudomonas aeruginosa* strain H1001 with cellulose bound peptides and controls after three hours incubation at 37°C.

Fig. 3A is a graphical representation showing the structural characterization of peptides by circular dichroism in buffer.

Fig. 3B is a graphical representation showing the structural characterization of peptides by circular dichroism in a lipid mimic, SDS.

Fig. 3C is a graphical representation showing the structural characterization of different scrambled peptides by circular dichroism spectroscopy in liposomes at increasing lipid to peptide ratios.

Fig. 4 is a graphical representation showing the mechanism of action of peptides assessed by measuring the ability to depolarize *Staphylococcus aureus* cells using the membrane potential-sensitive dye diSC35. Either 20 µg/ml of peptides or, as a control, 29 µg/ml of gramicidin S (Gram S) were added. Lines are smoothed using a poloynomial (to the 6th power) function.

Fig. 5 is a graphical representation showing the cytotoxicity on THP-1 macrophage-like cells as assessed by tryphan blue staining.

Fig. 6A is a graphical representation showing length analysis with bound peptides - longer peptides.

Fig. 6B is a graphical representation showing length analysis with bound peptides - shorter peptides.

Fig. 7 is a graphical representation showing complete substitution analysis of Bac2A.

Fig. 8 is a graphical representation showing assessment of the ability of peptides to bind to LPS as reflected by their suppression of *P. aeruginosa* LPS-stimulated TNFα production in THP1 cells. Presented results are the mean values for 4 wells performed on 2 separate occasions.

### DETAILED DESCRIPTION

### A. INTRODUCTION

The present invention provides peptides having antimicrobial activity. These peptides are useful for inhibiting microbial infection or growth and are often synergistic with conventional antibiotics and/or lysozyme. In addition, such peptides are useful as antifungal agents, antitumor agents, or antiviral agents. Many of the peptides of the invention are cationic in nature.

A method of synthesizing an array of peptides in parallel on cellulose sheets was developed by Ronald Frank in 1992. Frank, Tetrahedron 48: 9217-9232, 1992. This technique was first carried out manually and used for the identification of antibody epitopes. Now, with the help of pipetting robots, up to 8000 peptides can be synthesized on one cellulose sheet (20x30 cm). Kramer et al., Cell 91: 799-809, 1997. Today, the applications of this technology include characterizing homodimer interfaces, screening for kinase recognition sites, optimizing protease inhibitors, and screening for DNA binding sites of proteins.

The present invention adapts this methodology to create a large number of variants through sequence scrambling, truncations and systematic modifications of peptide sequence, and uses a luciferase-based screen to investigate their ability to kill *Pseudomonas aeruginosa.* This broad screening program represents a rapid and efficient method to investigate antimicrobial peptide activity. It has permitted for the first time a systematic and highly detailed investigation of the determinants of peptide activity in very small peptides. Previous attempts to make smaller peptides have tended to create molecules with modest activities or with good activities only when measured in dilute medium.

The peptides of the invention retain activities in the typical media used to test *in vitro* antibiotic activity, making them candidates for clinical therapeutic usage. In addition some of the peptides remain effective when bound to cellulose sheets, indicating that they have huge potential for use in coating medical devices, including catheters, to prevent them from becoming colonized with pathogenic bacteria.

The invention provides a number of methods, reagents, and compounds that can be used for inhibiting microbial infection or growth. It is to be understood that this invention is not limited to particular methods, reagents, compounds, compositions, or biological systems, which can, of course, vary. It is also to be understood that the terminology used herein is for the purpose of describing particular embodiments only, and is not intended to be limiting. As used in this specification and the appended claims, the singular forms "a", "an", and "the" include plural referents unless the content clearly dictates otherwise. Thus, for example, reference to "a peptide" includes a combination of two or more peptides, and the like.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

"Antimicrobial" as used herein means that the peptides of the present invention inhibit, prevent, or destroy the growth or proliferation of microbes such as bacteria, fungi, viruses, parasites or the like. "Antiviral" as used herein means that the peptides of the present invention inhibit, prevent or destroy the growth or proliferation of viruses or of virally-infected cells. "Anti-tumor" as used herein means that the peptides of the present invention may be used to inhibit the growth of or destroy tumors. "Antifungal" as used herein means that the peptides of the present invention may be used to inhibit the growth of or destroy fungi. "Antiparasite" as used herein means that the peptides of the present invention inhibit, prevent, or destroy the growth or proliferation of any organism that lives at the expense of a host organism.

As used herein, "microbe" or "microbial agent" is meant to include any organism comprised of the phylogenetic domains bacteria and archaea, as well as unicellular and filamentous fungi (such as yeasts and molds), unicellular and filamentous algae, unicellular and multicellular parasites, and viruses that causes a disease, disorder or condition in a subject. Accordingly, such microbial agents include, but are not limited to, bacterial, viral, fungal, or protozoan pathogens.

The compositions of the present invention possess activity toward microbes, *i*.*e*., antimicrobial activity. "Prevention" can be considered to be the obstruction or hindrance of any potential microbial growth. "Termination" can be considered to be actual killing of the microbes by the presence of the composition. "Inhibition" can be considered to be a reduction in microbial growth or inhibiting virulence factor expression or function of the microbe. Preferably, the compositions of the present invention will inhibit virulence factor expression or function of a microbe by greater than 30%, 50%, 65%, 70%, 75%, 80%, 85%, 90%, more preferably by greater than 91%, 92%, 93%, 94%, 95%, 96%, 97%, 98% or 99%.

The present invention is effective against bacteria including Gram-positive and Gram-negative cocci, Gram-positive and Gram-negative straight, curved and helical/vibroid and branched rods, sheathed bacteria, sulfur-oxidizing bacteria, sulfur or sulfate-reducing bacteria, spirochetes, actinomycetes and related genera, myxobacteria, mycoplasmas, rickettsias and chlamydias, cyanobacteria, archea, fungi, parasites, viruses and algae.

The "amino acid" residues identified herein are in the natural L-configuration. In keeping with standard polypeptide nomenclature, J. Biol. Chem. 243: 3557-59, 1968, abbreviations for amino acid residues are as shown in the following table.

| 1-Letter | 3-Letter | Amino Acid |
|---|---|---|
| Y | Tyr | L-tyrosine |
| G | Gly | L-glycine |
| F | Phe | L- |
| M | Met | L-methionine |
| A | Ala | L-alanine |
| S | Ser | L-serine |
| I | He | L-isoleucine |
| L | Leu | L-leucine |
| T | Thr | L-threonine |
| V | Val | L-valine |
| P | Pro | L-proline |
| K | Lys | L-lysine |
| H | His | L-histidine |
| Q | Gin | L-glutamine |
| E | Glu | L-glutamic acid |
| W | Trp | L-tryptohan |
| R | Arg | L-arginine |
| D | Asp | L-aspartic acid |
| N | Asn | L-asparagine |
| C | Cys | L-cysteine |

It should be noted that all amino acid residue sequences are represented herein by formulae whose left to right orientation is in the conventional direction of amino-terminus to carboxy-terminus.

Except when noted, the terms "subject" or "patient" are used interchangeably and refer to mammals such as human patients and non-human primates, as well as experimental animals such as rabbits, rats, and mice, and other animals. Accordingly, the term "subject" or "patient" as used herein means any mammalian patient or subject to which the compositions of the invention can be administered. In an exemplary embodiment of the present invention, to identify subject patients for treatment with a pharmaceutical composition comprising one or more antimicrobial peptides and/or proteins according to the methods of the invention, accepted screening methods are employed to determine the status of an existing disease or condition in a subject or risk factors associated with a targeted or suspected disease or condition. These screening methods include, for example, examinations to determine whether a subject is suffering from a microbial-based disease or disorder. These and other routine methods allow the clinician to select subjects in need of therapy.

### B. PEPTIDES

The invention provides an isolated antimicrobial peptide. Exemplary peptides of the invention have an amino acid sequence including those listed in Table 1, and analogs, derivatives, amidated variations and conservative variations thereof, wherein the peptides have antimicrobial activity. The peptides of the invention include SEQ ID NOS: 2-2166, as well as the broader groups of peptides having hydrophilic and hydrophobic substitutions, and conservative variations thereof.

"Isolated" when used in reference to a peptide, refers to a peptide substantially free of proteins, lipids, nucleic acids, for example, with which it can be naturally associated. Those of skill in the art can make similar substitutions to achieve peptides with greater antimicrobial activity and a broader host range. For example, the invention includes the peptides depicted in SEQ ID NOS: 1-2166, as well as analogs or derivatives thereof, as long as the bioactivity (*e*.*g*., antimicrobial) of the peptide remains. Minor modifications of the primary amino acid sequence of the peptides of the invention may result in peptides that have substantially equivalent activity as compared to the specific peptides described herein. Such modifications may be deliberate, as by site-directed mutagenesis, or may be spontaneous. All of the peptides produced by these modifications are included herein as long as the biological activity of the original peptide still exists.

Further, deletion of one or more amino acids can also result in a modification of the structure of the resultant molecule without significantly altering its biological activity. This can lead to the development of a smaller active molecule that would also have utility. For example, amino or carboxy terminal amino acids that may not be required for biological activity of the particular peptide can be removed. Peptides of the invention include any analog, homolog, mutant, isomer or derivative of the peptides disclosed in the present invention, so long as the bioactivity as described herein remains. All peptides were synthesized using L amino acids, however, all D forms of the peptides can be synthetically produced. In addition, C-terminal derivatives can be produced, such as C-terminal methyl esters and C-terminal amidates, in order to increase the antimicrobial activity of a peptide of the invention. The peptide can be synthesized such that the sequence is reversed whereby the last amino acid in the sequence becomes the first amino acid, and the penultimate amino acid becomes the second amino acid, and so on. It is well known that such reversed peptides usually have similar antimicrobial activities to the original sequence.

In certain embodiments, the peptides of the invention include peptide analogs and peptide mimetics. Indeed, the peptides of the invention include peptides having any of a variety of different modifications, including those described herein.

Peptide analogs of the invention are generally designed and produced by chemical modifications of a lead peptide, including, *e*.*g*., any of the particular peptides described herein, such as any of the following sequences disclosed in the tables. The present invention clearly establishes that these peptides in their entirety and derivatives created by modifying any side chains of the constituent amino acids have the ability to inhibit, prevent, or destroy the growth or proliferation of microbes such as bacteria, fungi, viruses, parasites or the like. The present invention further encompasses polypeptides up to about 50 amino acids in length that include the amino acid sequences and functional variants or peptide mimetics of the sequences described herein.

In another embodiment, a peptide of the present invention is a pseudopeptide. Pseudopeptides or amide bond surrogates refers to peptides containing chemical modifications of some (or all) of the peptide bonds. The introduction of amide bond surrogates not only decreases peptide degradation but also may significantly modify some of the biochemical properties of the peptides, particularly the conformational flexibility and hydrophobicity.

To improve or alter the characteristics of polypeptides of the present invention, protein engineering can be employed. Recombinant DNA technology known to those skilled in the art can be used to create novel mutant proteins or muteins including single or multiple amino acid substitutions, deletions, additions, or fusion proteins. Such modified polypeptides can show, *e*.*g*., increased/decreased biological activity or increased/decreased stability. In addition, they can be purified in higher yields and show better solubility than the corresponding natural polypeptide, at least under certain purification and storage conditions. Further, the polypeptides of the present invention can be produced as multimers including dimers, trimers and tetramers. Multimerization can be facilitated by linkers, introduction of cysteines to permit creation of interchain disulphide bonds, or recombinantly though heterologous polypeptides such as F_{c} regions.

It is known in the art that one or more amino acids can be deleted from the N-terminus or C-terminus without substantial loss of biological function. See, *e*.*g*., Ron et al., Biol Chem. 268: 2984-2988, 1993. Accordingly, the present invention provides polypeptides having one or more residues deleted from the amino terminus. Similarly, many examples of biologically functional C-terminal deletion mutants are known (see, *e*.*g*., Dobeli et al., J. Biotechnology 7: 199-216, 1988). Accordingly, the present invention provides polypeptides having one or more residues deleted from the carboxy terminus. The invention also provides polypeptides having one or more amino acids deleted from both the amino and the carboxyl termini as described below.

Other mutants in addition to N- and C-terminal deletion forms of the protein discussed above are included in the present invention. Thus, the invention further includes variations of the polypeptides which show substantial chaperone polypeptide activity. Such mutants include deletions, insertions, inversions, repeats, and substitutions selected according to general rules known in the art so as to have little effect on activity.

There are two main approaches for studying the tolerance of an amino acid sequence to change, see, Bowie et al., Science 247: 1306-1310, 1994. The first method relies on the process of evolution, in which mutations are either accepted or rejected by natural selection. The second approach uses genetic engineering to introduce amino acid changes at specific positions of a cloned gene and selections or screens to identify sequences that maintain functionality. These studies have revealed that proteins are surprisingly tolerant of amino acid substitutions.

Typically seen as conservative substitutions are the replacements, one for another, among the aliphatic amino acids Ala, Val, Leu and Phe; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr. Thus, the polypeptide of the present invention can be, for example: (i) one in which one or more of the amino acid residues are substituted with a conserved or non-conserved amino acid residue (preferably a conserved amino acid residue) and such substituted amino acid residue can or cannot be one encoded by the genetic code; or (ii) one in which one or more of the amino acid residues includes a substituent group; or (iii) one in which the polypeptide is fused with another compound, such as a compound to increase the half-life of the polypeptide (for example, polyethylene glycol); or (iv) one in which the additional amino acids are fused to the above form of the polypeptide, such as an IgG F_{c} fusion region peptide or leader or secretory sequence or a sequence which is employed for purification of the above form of the polypeptide or a pro-protein sequence.

Thus, the polypeptides of the present invention can include one or more amino acid substitutions, deletions, or additions, either from natural mutations or human manipulation. As indicated, changes are preferably of a minor nature, such as conservative amino acid substitutions that do not significantly affect the folding or activity of the protein. The following groups of amino acids represent equivalent changes: (1) Ala, Pro, Gly, Glu, Asp, Gln, Asn, Ser, Thr; (2) Cys, Ser, Tyr, Thr; (3) Val, Ile, Leu, Met, Ala, Phe; (4) Lys, Arg, His; (5) Phe, Tyr, Trp, His.

Furthermore, polypeptides of the present invention can include one or more amino acid substitutions that mimic modified amino acids. An example of this type of substitution includes replacing amino acids that are capable of being phosphorylated (*e*.*g*., serine, threonine, or tyrosine) with a negatively charged amino acid that resembles the negative charge of the phosphorylated amino acid (*e*.*g*., aspartic acid or glutamic acid). Also included is substitution of amino acids that are capable of being modified by hydrophobic groups (*e*.*g*., arginine) with amino acids carrying bulky hydrophobic side chains, such as tryptophan or phenylalanine. Therefore, a specific embodiment of the invention includes polypeptides that include one or more amino acid substitutions that mimic modified amino acids at positions where amino acids that are capable of being modified are normally positioned. Further included are polypeptides where any subset of modifiable amino acids is substituted. For example, a polypeptide that includes three serine residues can be substituted at any one, any two, or all three of said serines. Furthermore, any polypeptide amino acid capable of being modified can be excluded from substitution with a modification-mimicking amino acid.

The present invention is further directed to fragments of the polypeptides of the present invention. More specifically, the present invention embodies purified, isolated, and recombinant polypeptides comprising at least any one integer between 6 and 504 (or the length of the polypeptides amino acid residues minus 1 if the length is less than 1000) of consecutive amino acid residues. Preferably, the fragments are at least 6, preferably at least 8 to 10, more preferably 12, 15, 20, 25, 30, 35, 40, 50 or more consecutive amino acids of a polypeptide of the present invention.

The present invention also provides for the exclusion of any species of polypeptide fragments of the present invention specified by 5' and 3' positions or sub-genuses of polypeptides specified by size in amino acids as described above. Any number of fragments specified by 5' and 3' positions or by size in amino acids, as described above, can be excluded.

In addition, it should be understood that in certain embodiments, the peptides of the present invention include two or more modifications, including, but not limited to those described herein. By taking into the account the features of the peptide drugs on the market or under current development, it is clear that most of the peptides successfully stabilized against proteolysis consist of a mixture of several types of the above described modifications. This conclusion is understood in the light of the knowledge that many different enzymes are implicated in peptide degradation.

### C. PEPTIDES, PEPTIDE VARIANTS, AND PEPTIDE MIMETICS

"Polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Amino acid mimetics refers to chemical compounds that have a structure that is different from the general chemical structure of an amino acid, but which functions in a manner similar to a naturally occurring amino acid. Non-natural residues are well described in the scientific and patent literature; a few exemplary non-natural compositions useful as mimetics of natural amino acid residues and guidelines are described below. Mimetics of aromatic amino acids can be generated by replacing by, *e*.*g*., D- or L-naphylalanine; D- or L-phenylglycine; D- or L-2 thieneylalanine; D- or L-1, -2,3-, or 4-pyreneylalanine; D- or L-3 thieneylalanine; D- or L-(2-pyridinyl)-alanine; D- or L-(3-pyridinyl)-alanine; D- or L-(2-pyrazinyl)-alanine; D- or L-(4-isopropyl)-phenylglycine; D-(trifluoromethyl)-phenylglycine; D-(trifluoromethyl)-phenylalanine; D-p-fluoro-phenylalanine; D- or L-p-biphenylphenylalanine; K- or L-p-methoxy-biphenylphenylalanine; D- or L-2-indole(alkyl)alanines; and, D- or L-alkylainines, where alkyl can be substituted or unsubstituted methyl, ethyl, propyl, hexyl, butyl, pentyl, isopropyl, iso-butyl, sec-isotyl, iso-pentyl, or a non-acidic amino acids. Aromatic rings of a non-natural amino acid include, *e*.*g*., thiazolyl, thiophenyl, pyrazolyl, benzimidazolyl, naphthyl, furanyl, pyrrolyl, and pyridyl aromatic rings.

"Peptide" as used herein includes peptides that are conservative variations of those peptides specifically exemplified herein. "Conservative variation" as used herein denotes the replacement of an amino acid residue by another, biologically similar residue. Examples of conservative variations include, but are not limited to, the substitution of one hydrophobic residue such as isoleucine, valine, leucine, alanine, cysteine, glycine, phenylalanine, proline, tryptophan, tyrosine, norleucine or methionine for another, or the substitution of one polar residue for another, such as the substitution of arginine for lysine, glutamic for aspartic acids, or glutamine for asparagine, and the like. Neutral hydrophilic amino acids that can be substituted for one another include asparagine, glutamine, serine and threonine. The term "conservative variation" also includes the use of a substituted amino acid in place of an unsubstituted parent amino acid provided that antibodies raised to the substituted polypeptide also immunoreact with the unsubstituted polypeptide. Such conservative substitutions are within the definition of the classes of the peptides of the invention. "Cationic" as is used to refer to any peptide that possesses sufficient positively charged amino acids to have a pI (isoelectric point) greater than about 9.0.

The biological activity of the peptides can be determined by standard methods known to those of skill in the art, such as "minimal inhibitory concentration (MIC)" assay described in the present examples, whereby the lowest concentration at which no change in OD is observed for a given period of time is recorded as MIC.

The peptides and polypeptides of the invention, as defined above, include all "mimetic" and "peptidomimetic" forms. The terms "mimetic" and "peptidomimetic" refer to a synthetic chemical compound that has substantially the same structural and/or functional characteristics of the polypeptides of the invention. The mimetic can be either entirely composed of synthetic, non-natural analogues of amino acids, or, is a chimeric molecule of partly natural peptide amino acids and partly non-natural analogs of amino acids. The mimetic can also incorporate any amount of natural amino acid conservative substitutions as long as such substitutions also do not substantially alter the mimetic's structure and/or activity. As with polypeptides of the invention which are conservative variants, routine experimentation will determine whether a mimetic is within the scope of the invention, *i*.*e*., that its structure and/or function is not substantially altered. Thus, a mimetic composition is within the scope of the invention if, when administered to or expressed in a cell, *e*.*g*., a polypeptide fragment of an animicrobial protein having antimicrobial activity.

Polypeptide mimetic compositions can contain any combination of non-natural structural components, which are typically from three structural groups: a) residue linkage groups other than the natural amide bond ("peptide bond") linkages; b) non-natural residues in place of naturally occurring amino acid residues; or c) residues which induce secondary structural mimicry, *i*.*e*., to induce or stabilize a secondary structure, *e*.*g*., a beta turn, gamma turn, beta sheet, alpha helix conformation, and the like. For example, a polypeptide can be characterized as a mimetic when all or some of its residues are joined by chemical means other than natural peptide bonds. Individual peptidomimetic residues can be joined by peptide bonds, other chemical bonds or coupling means, such as, *e*.*g*., glutaraldehyde, N-hydroxysuccinimide esters, bifunctional maleimides, N,N'-dicyclohexylcarbodiimide (DCC) or N,N'-diisopropylcarbodiimide (DIC). Linking groups that can be an alternative to the traditional amide bond ("peptide bond") linkages include, *e*.*g*., ketomethylene (*e*.*g*., --C(=O)-CH₂-for -C(=O)-NH--), aminomethylene (CH₂-NH), ethylene, olefin (CH=CH), ether (CH₂-O), thioether (CH₂-S), tetrazole (CN₄-), thiazole, retroamide, thioamide, or ester (see, *e*.*g*., Spatola, 1983, Chemistry and Biochemistry of Amino Acids, Peptides and Proteins 7: 267-357).

Mimetics of acidic amino acids can be generated by substitution by, *e*.*g*., non-carboxylate amino acids while maintaining a negative charge; (phosphono)alanine; sulfated threonine. Carboxyl side groups (*e*.*g*., aspartyl or glutamyl) can also be selectively modified by reaction with carbodiimides (R'-N-C-N-R') such as, *e*.*g*., 1-cyclohexyl-3(2-morpholin-yl-(4-ethyl) carbodiimide or 1-ethyl-3(4-azonia-4,4-dimetholpentyl) carbodiimide. Aspartyl or glutamyl can also be converted to asparaginyl and glutaminyl residues by reaction with ammonium ions.

Mimetics of basic amino acids can be generated by substitution with, *e*.*g*., (in addition to lysine and arginine) the amino acids ornithine, citrulline, guanidino-acetic acid, or (guanidino)alkyl-acetic acid, where alkyl is defined above. Nitrile derivative (*e*.*g*., containing the CN-moiety in place of COOH) can be substituted for asparagine or glutamine. Asparaginyl and glutaminyl residues can be deaminated to the corresponding aspartyl or glutamyl residues.

Arginine residue mimetics can be generated by reacting arginyl with, *e*.*g*., one or more conventional reagents, including, *e*.*g*., phenylglyoxal, 2,3-butanedione, 1,2-cyclohexanedione, or ninhydrin, preferably under alkaline conditions. Tyrosine residue mimetics can be generated by reacting tyrosyl with, *e*.*g*., aromatic diazonium compounds or tetranitromethane. N-acetylimidizol and tetranitromethane can be used to form O-acetyl tyrosyl species and 3-nitro derivatives, respectively. Cysteine residue mimetics can be generated by reacting cysteinyl residues with, *e*.*g*., alpha-haloacetates such as 2-chloroacetic acid or chloroacetamide and corresponding amines; to give carboxymethyl or carboxyamidomethyl derivatives. Cysteine residue mimetics can also be generated by reacting cysteinyl residues with, *e*.*g*., bromo-trifluoroacetone, alpha-bromo-beta-(5-imidozoyl) propionic acid; chloroacetyl phosphate, N-alkylmaleimides, 3-nitro-2-pyridyl disulfide; methyl 2-pyridyl disulfide; p-chloromercuribenzoate; 2-chloromercuri-4 nitrophenol; or, chloro-7-nitrobenzo-oxa-1,3-diazole. Lysine mimetics can be generated (and amino terminal residues can be altered) by reacting lysinyl with, *e*.*g*., succinic or other carboxylic acid anhydrides. Lysine and other alpha-amino-containing residue mimetics can also be generated by reaction with imidoesters, such as methyl picolinimidate, pyridoxal phosphate, pyridoxal, chloroborohydride, trinitrobenzenesulfonic acid, O-methylisourea, 2,4, pentanedione, and transamidase-catalyzed reactions with glyoxylate. Mimetics of methionine can be generated by reaction with, *e*.*g*., methionine sulfoxide. Mimetics of proline include, *e*.*g*., pipecolic acid, thiazolidine carboxylic acid, 3- or 4-hydroxy proline, dehydroproline, 3- or 4-methylproline, or 3,3,-dimethylproline. Histidine residue mimetics can be generated by reacting histidyl with, *e*.*g*., diethylprocarbonate or para-bromophenacyl bromide. Other mimetics include, *e*.*g*., those generated by hydroxylation of proline and lysine; phosphorylation of the hydroxyl groups of seryl or threonyl residues; methylation of the alpha-amino groups of lysine, arginine and histidine; acetylation of the N-terminal amine; methylation of main chain amide residues or substitution with N-methyl amino acids; or amidation of C-terminal carboxyl groups.

A component of a polypeptide of the invention can also be replaced by an amino acid (or peptidomimetic residue) of the opposite chirality. Thus, any amino acid naturally occurring in the L-configuration (which can also be referred to as the R or S, depending upon the structure of the chemical entity) can be replaced with the amino acid of the same chemical structural type or a peptidomimetic, but of the opposite chirality, referred to as the D-amino acid, but which can additionally be referred to as the R- or S-form

The invention also provides polypeptides that are "substantially identical" to an exemplary polypeptide of the invention. A "substantially identical" amino acid sequence is a sequence that differs from a reference sequence by one or more conservative or non-conservative amino acid substitutions, deletions, or insertions, particularly when such a substitution occurs at a site that is not the active site of the molecule, and provided that the polypeptide essentially retains its functional properties. A conservative amino acid substitution, for example, substitutes one amino acid for another of the same class (*e*.*g*., substitution of one hydrophobic amino acid, such as isoleucine, valine, leucine, or methionine, for another, or substitution of one polar amino acid for another, such as substitution of arginine for lysine, glutamic acid for aspartic acid or glutamine for asparagine). One or more amino acids can be deleted, for example, from an antimicrobial polypeptide having antimicrobial activity of the invention, resulting in modification of the structure of the polypeptide, without significantly altering its biological activity. For example, amino- or carboxyl-terminal, or internal, amino acids that are not required for antimicrobial activity can be removed.

The skilled artisan will recognize that individual synthetic residues and polypeptides incorporating these mimetics can be synthesized using a variety of procedures and methodologies, which are well described in the scientific and patent literature, *e*.*g*., Organic Syntheses Collective Volumes, Gilman, et al. (Eds) John Wiley & Sons, Inc., NY. Peptides and peptide mimetics of the invention can also be synthesized using combinatorial methodologies. Various techniques for generation of peptide and peptidomimetic libraries are well known, and include, *e*.*g*., multipin, tea bag, and split-couple-mix techniques; see, *e*.*g*., al-Obeidi, Mol. Biotechnol. 9: 205-223, 1998; Hruby, Curr. Opin. Chem. Biol. 1: 114-119, 1997; Ostergaard, Mol. Divers. 3: 17-27, 1997; Ostresh, Methods Enzymol. 267: 220-234, 1996. Modified peptides of the invention can be further produced by chemical modification methods, see, *e*.*g.,* Belousov, Nucleic Acids Res. 25: 3440-3444, 1997; Frenkel, Free Radic. Biol. Med. 19: 373-380, 1995; Blommers, Biochemistry 33: 7886-7896, 1994.

Polypeptides and peptides of the invention can be isolated from natural sources, be synthetic, or be recombinantly generated polypeptides. Peptides and proteins can be recombinantly expressed *in vitro* or *in vivo.* The peptides and polypeptides of the invention can be made and isolated using any method known in the art. Polypeptide and peptides of the invention can also be synthesized, whole or in part, using chemical methods well known in the art. See *e*.*g*., Caruthers, Nucleic Acids Res. Symp. Ser. 215-223, 1980; Horn, Nucleic Acids Res. Symp. Ser. 225-232, 1980; Banga, 1995, Therapeutic Peptides and Proteins, Formulation, Processing and Delivery Systems. For example, peptide synthesis can be performed using various solid-phase techniques (see *e.g.,* Roberge, Science 269: 202, 1995; Merrifield, Methods Enzymol. 289: 3-13, 1997) and automated synthesis can be achieved, *e*.*g*., using the ABI 431A Peptide Synthesizer (Perkin Elmer) in accordance with the instructions provided by the manufacturer.

Peptides of the invention can be synthesized by such commonly used methods as t-BOC or FMOC protection of alpha-amino groups. Both methods involve stepwise syntheses whereby a single amino acid is added at each step starting from the C terminus of the peptide (See, Coligan et al., Current Protocols in Immunology, Wiley Interscience, 1991, Unit 9). Peptides of the invention can also be synthesized by the well known solid phase peptide synthesis methods described in Merrifield, J. Am. Chem. Soc. 85: 2149, 1962, and Stewart and Young, 1969, Solid Phase Peptides Synthesis 27-62, using a copoly(styrene-divinylbenzene) containing 0.1-1.0 mMol amines/g polymer. On completion of chemical synthesis, the peptides can be deprotected and cleaved from the polymer by treatment with liquid HF-10% anisole for about 1/4-1 hours at 0°C. After evaporation of the reagents, the peptides are extracted from the polymer with 1% acetic acid solution which is then lyophilized to yield the crude material. This can normally be purified by such techniques as gel filtration on Sephadex G-15 using 5% acetic acid as a solvent. Lyophilization of appropriate fractions of the column will yield the homogeneous peptide or peptide derivatives, which can then be characterized by such standard techniques as amino acid analysis, thin layer chromatography, high performance liquid chromatography, ultraviolet absorption spectroscopy, molar rotation, solubility, and quantitated by the solid phase Edman degradation.

Analogs, polypeptide fragment of antimicrobial protein having antimicrobial activity, are generally designed and produced by chemical modifications of a lead peptide, including, *e*.*g*., any of the particular peptides described herein, such as any of the sequences including SEQ ID NOS:1-2166.

The terms "identical" or percent "identity", in the context of two or more nucleic acids or polypeptide sequences, refers to two or more sequences or subsequences that are the same or have a specified percentage of amino acid residues or nucleotides that are the same (*i*.*e*., about 60% identity, preferably 65%, 70%, 75%, 80%, 85%, 90%, 91%, 92%, 93%, 94%, 95%, 96%, 97%, 98%, 99%, or higher identity over a specified region (*e*.*g*., nucleotide sequence encoding an antibody described herein or amino acid sequence of an antibody described herein), when compared and aligned for maximum correspondence over a comparison window or designated region) as measured using a BLAST or BLAST 2.0 sequence comparison algorithms with default parameters described below, or by manual alignment and visual inspection. Such sequences are then said to be "substantially identical." This term also refers to, or can be applied to, the compliment of a test sequence. The term also includes sequences that have deletions and/or additions, as well as those that have substitutions. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence can be compared to a reference sequence of the same number of contiguous positions after the two sequences are optimally aligned. Methods of alignment of sequences for comparison are well-known in the art. Optimal alignment of sequences for comparison can be conducted, *e*.*g*., by the local homology algorithm of Smith & Waterman, Adv. Appl. Math. 2: 482, 1981, by the homology alignment algorithm of Needleman & Wunsch, J. Mol. Biol. 48: 443, 1970, by the search for similarity method of Pearson & Lipman, Proc. Nat'l. Acad. Sci. USA 85: 2444, 1988, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, *e*.*g*., Ausubel et al., 1995 supplement, Current Protocols in Molecular Biology).

Programs for searching for alignments are well known in the art, *e*.*g*., BLAST and the like. For example, if the target species is human, a source of such amino acid sequences or gene sequences (germline or rearranged antibody sequences) can be found in any suitable reference database such as Genbank, the NCBI protein databank (http://ncbi.nlm.nih.gov/BLAST/), VBASE, a database of human antibody genes (http://www.mrc-cpe.cam.ac.uk/imt-doc), and the Kabat database of immunoglobulins (http://www.immuno.bme.nwu.edu) or translated products thereof. If the alignments are done based on the nucleotide sequences, then the selected genes should be analyzed to determine which genes of that subset have the closest amino acid homology to the originating species antibody. It is contemplated that amino acid sequences or gene sequences which approach a higher degree homology as compared to other sequences in the database can be utilized and manipulated in accordance with the procedures described herein. Moreover, amino acid sequences or genes which have lesser homology can be utilized when they encode products which, when manipulated and selected in accordance with the procedures described herein, exhibit specificity for the predetermined target antigen. In certain embodiments, an acceptable range of homology is greater than about 50%. It should be understood that target species can be other than human.

A preferred example of algorithm that is suitable for determining percent sequence identity and sequence similarity are the BLAST and BLAST 2.0 algorithms, which are described in Altschul et al., Nuc. Acids Res. 25: 3389-3402, 1977 and Altschul et al., J. Mol. Biol. 215: 403-410, 1990, respectively. BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold. These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff & Henikoff, Proc. Natl. Acad. Sci. USA 89: 10915, 1989) alignments (B) of 50, expectation (E) of 10, M=5, N=-4, and a comparison of both strands.

### D. POLYPEPTIDES AND FUNCTIONAL VARIANTS THEREOF

"Polypeptide" includes proteins, fusion proteins, oligopeptides and polypeptide derivatives, with the exception that peptidomimetics are considered to be small molecules herein.

A "protein" is a molecule having a sequence of amino acids that are linked to each other in a linear molecule by peptide bonds. The term protein refers to a polypeptide that is isolated from a natural source, or produced from an isolated cDNA using recombinant DNA technology; and has a sequence of amino acids having a length of at least about 200 amino acids.

A "fusion protein" is a type of recombinant protein that has an amino acid sequence that results from the linkage of the amino acid sequences of two or more normally separate polypeptides.

A "protein fragment" is a proteolytic fragment of a larger polypeptide, which may be a protein or a fusion protein. A proteolytic fragment may be prepared by *in vivo* or *in vitro* proteolytic cleavage of a larger polypeptide, and is generally too large to be prepared by chemical synthesis. Proteolytic fragments have amino acid sequences having a length from about 200 to about 1,000 amino acids.

An "oligopeptide" or "peptide" is a polypeptide having a short amino acid sequence (*i*.*e*., 2 to about 200 amino acids). An oligopeptide is generally prepared by chemical synthesis.

Although oligopeptides and protein fragments may be otherwise prepared, it is possible to use recombinant DNA technology and/or *in vitro* biochemical manipulations. For example, a nucleic acid encoding an amino acid sequence may be prepared and used as a template for *in vitro* transcription/translation reactions. In such reactions, an exogenous nucleic acid encoding a preselected polypeptide is introduced into a mixture that is essentially depleted of exogenous nucleic acids that contains all of the cellular components required for transcription and translation. One or more radiolabeled amino acids are added before or with the exogenous DNA, and transcription and translation are allowed to proceed. Because the only nucleic acid present in the reaction mix is the exogenous nucleic acid added to the reaction, only polypeptides encoded thereby are produced, and incorporate the radiolabeled amino acid(s). In this manner, polypeptides encoded by a preselected exogenous nucleic acid are radiolabeled. Although other proteins are present in the reaction mix, the preselected polypeptide is the only one that is produced in the presence of the radiolabeled amino acids and is thus uniquely labeled.

As is explained in detail below, "polypeptide derivatives" include without limitation mutant polypeptides, chemically modified polypeptides, and peptidomimetics.

The polypeptides of this invention, including the analogs and other modified variants, may generally be prepared following known techniques. Preferably, synthetic production of the polypeptide of the invention may be according to the solid phase synthetic method. For example, the solid phase synthesis is well understood and is a common method for preparation of polypeptides, as are a variety of modifications of that technique. Merrifield, J. Am. Chem. Soc., 85: 2149, 1964; Stewart and Young, 1984, Solid Phase polypeptide Synthesis; Bodansky and Bodanszky, 1984, The Practice of polypeptide Synthesis; Atherton and Sheppard, 1989, Solid Phase polypeptide Synthesis: A Practical Approach. See, also, the specific method described in Example 1 below.

Alternatively, polypeptides of this invention may be prepared in recombinant systems using polynucleotide sequences encoding the polypeptides.

A "variant" or "functional variant" of a polypeptide is a compound that is not, by definition, a polypeptide, *i*.*e*., it contains at least one chemical linkage that is not a peptide bond. Thus, polypeptide derivatives include without limitation proteins that naturally undergo post-translational modifications such as, *e*.*g*., glycosylation. It is understood that a polypeptide of the invention may contain more than one of the following modifications within the same polypeptide. Preferred polypeptide derivatives retain a desirable attribute, which may be biological activity; more preferably, a polypeptide derivative is enhanced with regard to one or more desirable attributes, or has one or more desirable attributes not found in the parent polypeptide. Although they are described in this section, peptidomimetics are taken as small molecules in the present disclosure.

A polypeptide having an amino acid sequence identical to that found in a protein prepared from a natural source is a "wild type" polypeptide. Functional variants of polypeptides can be prepared by chemical synthesis, including without limitation combinatorial synthesis.

Functional variants of polypeptides larger than oligopeptides can be prepared using recombinant DNA technology by altering the nucleotide sequence of a nucleic acid encoding a polypeptide. Although some alterations in the nucleotide sequence will not alter the amino acid sequence of the polypeptide encoded thereby ("silent" mutations), many will result in a polypeptide having an altered amino acid sequence that is altered relative to the parent sequence. Such altered amino acid sequences may comprise substitutions, deletions and additions of amino acids, with the proviso that such amino acids are naturally occurring amino acids.

Thus, subjecting a nucleic acid that encodes a polypeptide to mutagenesis is one technique that can be used to prepare Functional variants of polypeptides, particularly ones having substitutions of amino acids but no deletions or insertions thereof. A variety of mutagenic techniques are known that can be used *in vitro* or *in vivo* including without limitation chemical mutagenesis and PCR-mediated mutagenesis. Such mutagenesis may be randomly targeted (*i*.*e*., mutations may occur anywhere within the nucleic acid) or directed to a section of the nucleic acid that encodes a stretch of amino acids of particular interest. Using such techniques, it is possible to prepare randomized, combinatorial or focused compound libraries, pools and mixtures.

Polypeptides having deletions or insertions of naturally occurring amino acids may be synthetic oligopeptides that result from the chemical synthesis of amino acid sequences that are based on the amino acid sequence of a parent polypeptide but which have one or more amino acids inserted or deleted relative to the sequence of the parent polypeptide. Insertions and deletions of amino acid residues in polypeptides having longer amino acid sequences may be prepared by directed mutagenesis.

As contemplated by this invention, "polypeptide" includes those having one or more chemical modification relative to another polypeptide, *i*.*e*., chemically modified polypeptides. The polypeptide from which a chemically modified polypeptide is derived may be a wild type protein, a functional variant protein or a functional variant polypeptide, or polypeptide fragments thereof; an antibody or other polypeptide ligand according to the invention including without limitation single-chain antibodies, crystalline proteins and polypeptide derivatives thereof; or polypeptide ligands prepared according to the disclosure. Preferably, the chemical modification(s) confer(s) or improve(s) desirable attributes of the polypeptide but does not substantially alter or compromise the biological activity thereof. Desirable attributes include but are limited to increased shelf-life; enhanced serum or other *in vivo* stability; resistance to proteases; and the like. Such modifications include by way of non-limiting example N-terminal acetylation, glycosylation, and biotinylation.

An effective approach to confer resistance to peptidases acting on the N-terminal or C-terminal residues of a polypeptide is to add chemical groups at the polypeptide termini, such that the modified polypeptide is no longer a substrate for the peptidase. One such chemical modification is glycosylation of the polypeptides at either or both termini. Certain chemical modifications, in particular N-terminal glycosylation, have been shown to increase the stability of polypeptides in human serum. Powell et al., Pharma. Res. 10: 1268-1273, 1993. Other chemical modifications which enhance serum stability include, but are not limited to, the addition of an N-terminal alkyl group, consisting of a lower alkyl of from 1 to 20 carbons, such as an acetyl group, and/or the addition of a C-terminal amide or substituted amide group.

The presence of an N-terminal D-amino acid increases the serum stability of a polypeptide that otherwise contains L-amino acids, because exopeptidases acting on the N-terminal residue cannot utilize a D-amino acid as a substrate. Similarly, the presence of a C-terminal D-amino acid also stabilizes a polypeptide, because serum exopeptidases acting on the C-terminal residue cannot utilize a D-amino acid as a substrate. With the exception of these terminal modifications, the amino acid sequences of polypeptides with N-terminal and/or C-terminal D-amino acids are usually identical to the sequences of the parent L-amino acid polypeptide.

Substitution of unnatural amino acids for natural amino acids in a subsequence of a polypeptide can confer or enhance desirable attributes including biological activity. Such a substitution can, for example, confer resistance to proteolysis by exopeptidases acting on the N-terminus. The synthesis of polypeptides with unnatural amino acids is routine and known in the art (see, for example, Coller, *et al*. 1993, cited above).

Different host cells will contain different post-translational modification mechanisms that may provide particular types of post-translational modification of a fusion protein if the amino acid sequences required for such modifications is present in the fusion protein. A large number (about 100) of post-translational modifications have been described, a few of which are discussed herein. One skilled in the art will be able to choose appropriate host cells, and design chimeric genes that encode protein members comprising the amino acid sequence needed for a particular type of modification.

Glycosylation is one type of post-translational chemical modification that occurs in many eukaryotic systems, and may influence the activity, stability, pharmacogenetics, immunogenicity and/or antigenicity of proteins. However, specific amino acids must be present at such sites to recruit the appropriate glycosylation machinery, and not all host cells have the appropriate molecular machinery. Saccharomyces cerevisieae and Pichia pastoris provide for the production of glycosylated proteins, as do expression systems that utilize insect cells, although the pattern of glyscoylation may vary depending on which host cells are used to produce the fusion protein.

Another type of post-translation modification is the phosphorylation of a free hydroxyl group of the side chain of one or more Ser, Thr or Tyr residues, Protein kinases catalyze such reactions. Phosphorylation is often reversible due to the action of a protein phosphatase, an enzyme that catalyzes the dephosphorylation of amino acid residues.

Differences in the chemical structure of amino terminal residues result from different host cells, each of which may have a different chemical version of the methionine residue encoded by a start codon, and these will result in amino termini with different chemical modifications.

For example, many or most bacterial proteins are synthesized with an amino terminal amino acid that is a modified form of methionine, *i*.*e*., N-formylmethionine (fMet). Although the statement is often made that all bacterial proteins are synthesized with an fMet initiator amino acid; although this may be true for *E. coli*, recent studies have shown that it is not true in the case of other bacteria such as *Pseudomonas aeruginosa*. Newton et al., J. Biol. Chem. 274: 22143-22146, 1999. In any event, in E. coli, the formyl group of fMet is usually enzymatically removed after translation to yield an amino terminal methionine residue, although the entire fMet residue is sometimes removed (see Hershey, 1987, Escherichia coli and Salmonella Typhimurium: Cellular and Molecular Biology 1: 613-647, and references cited therein.). *E. coli* mutants that lack the enzymes (such as, *e*.*g*., formylase) that catalyze such post-translational modifications will produce proteins having an amino terminal fMet residue (Guillon et al., J. Bacteriol. 174: 4294-4301, 1992).

In eukaryotes, acetylation of the initiator methionine residue, or the penultimate residue if the initiator methionine has been removed, typically occurs co- or post-translationally. The acetylation reactions are catalyzed by N-terminal acetyltransferases (NATs, a.k.a. N-alpha-acetyltransferases), whereas removal of the initiator methionine residue is catalyzed by methionine aminopeptidases (for reviews, see Bradshaw et al., Trends Biochem. Sci. 23: 263-267, 1998; and Driessen et al., CRC Crit. Rev. Biochem. 18: 281-325, 1985). Amino terminally acetylated proteins are said to be "N-acetylated," "N alpha acetylated" or simply "acetylated."

Another post-translational process that occurs in eukaryotes is the alpha-amidation of the carboxy terminus. For reviews, see Eipper et al., Annu. Rev. Physiol. 50: 333-344, 1988, and Bradbury et al., Lung Cancer 14: 239-251, 1996. About 50% of known endocrine and neuroendocrine peptide hormones are alpha-amidated (Treston et al., Cell Growth Differ. 4: 911-920, 1993). In most cases, carboxy alpha-amidation is required to activate these peptide hormones.

### E. POLYPEPTIDE MIMETIC

In general, a polypeptide mimetic ("peptidomimetic") is a molecule that mimics the biological activity of a polypeptide but is no longer peptidic in chemical nature. By strict definition, a peptidomimetic is a molecule that contains no peptide bonds (that is, amide bonds between amino acids). However, the term peptidomimetic is sometimes used to describe molecules that are no longer completely peptidic in nature, such as pseudo-peptides, semi-peptides and peptoids. Examples of some peptidomimetics by the broader definition (where part of a polypeptide is replaced by a structure lacking peptide bonds) are described below. Whether completely or partially non-peptide, peptidomimetics according to this invention provide a spatial arrangement of reactive chemical moieties that closely resembles the three-dimensional arrangement of active groups in the polypeptide on which the peptidomimetic is based. As a result of this similar active-site geometry, the peptidomimetic has effects on biological systems that are similar to the biological activity of the polypeptide.

There are several potential advantages for using a mimetic of a given polypeptide rather than the polypeptide itself. For example, polypeptides may exhibit two undesirable attributes, *i*.*e*., poor bioavailability and short duration of action. Peptidomimetics are often small enough to be both orally active and to have a long duration of action. There are also problems associated with stability, storage and immunoreactivity for polypeptides that are not experienced with peptidomimetics.

Candidate, lead and other polypeptides having a desired biological activity can be used in the development of peptidomimetics with similar biological activities. Techniques of developing peptidomimetics from polypeptides are known. Peptide bonds can be replaced by non-peptide bonds that allow the peptidomimetic to adopt a similar structure, and therefore biological activity, to the original polypeptide. Further modifications can also be made by replacing chemical groups of the amino acids with other chemical groups of similar structure. The development of peptidomimetics can be aided by determining the tertiary structure of the original polypeptide, either free or bound to a ligand, by NMR spectroscopy, crystallography and/or computer-aided molecular modeling. These techniques aid in the development of novel compositions of higher potency and/or greater bioavailability and/or greater stability than the original polypeptide (Dean, BioEssays 16: 683-687, 1994; Cohen and Shatzmiller, J. Mol. Graph. 11: 166-173, 1993; Wiley and Rich, Med. Res. Rev. 13: 327-384, 1993; Moore, Trends Pharmacol. Sci. 15: 124-129, 1994; Hruby, Biopolymers 33: 1073-1082, 1993; Bugg et al., Sci. Am. 269: 92-98, 1993, all incorporated herein by reference].

Thus, through use of the methods described above, the present invention provides compounds exhibiting enhanced therapeutic activity in comparison to the polypeptides described above. The peptidomimetic compounds obtained by the above methods, having the biological activity of the above named polypeptides and similar three-dimensional structure, are encompassed by this invention. It will be readily apparent to one skilled in the art that a peptidomimetic can be generated from any of the modified polypeptides described in the previous section or from a polypeptide bearing more than one of the modifications described from the previous section. It will furthermore be apparent that the peptidomimetics of this invention can be further used for the development of even more potent non-peptidic compounds, in addition to their utility as therapeutic compounds.

Specific examples of peptidomimetics derived from the polypeptides described in the previous section are presented below. These examples are illustrative and not limiting in terms of the other or additional modifications.

Proteases act on peptide bonds. It therefore follows that substitution of peptide bonds by pseudopeptide bonds confers resistance to proteolysis. A number of pseudopeptide bonds have been described that in general do not affect polypeptide structure and biological activity. The reduced isosteric pseudopeptide bond is a suitable pseudopeptide bond that is known to enhance stability to enzymatic cleavage with no or little loss of biological activity (Couder et al., Int. J. Polypeptide Protein Res. 41: 181-184, 1993, incorporated herein by reference). Thus, the amino acid sequences of these compounds may be identical to the sequences of their parent L-amino acid polypeptides, except that one or more of the peptide bonds are replaced by an isosteric pseudopeptide bond. Preferably the most N-terminal peptide bond is substituted, since such a substitution would confer resistance to proteolysis by exopeptidases acting on the N-terminus.

To confer resistance to proteolysis, peptide bonds may also be substituted by retro-inverso pseudopeptide bonds (Dalpozzo et al., Int. J. Polypeptide Protein Res. 41: 561-566, incorporated herein by reference). According to this modification, the amino acid sequences of the compounds may be identical to the sequences of their L-amino acid parent polypeptides, except that one or more of the peptide bonds are replaced by a retro-inverso pseudopeptide bond. Preferably the most N-terminal peptide bond is substituted, since such a substitution will confer resistance to proteolysis by exopeptidases acting on the N-terminus.

Peptoid derivatives of polypeptides represent another form of modified polypeptides that retain the important structural determinants for biological activity, yet eliminate the peptide bonds, thereby conferring resistance to proteolysis (Simon et al., Proc. Natl. Acad. Sci. USA 89: 9367-9371, 1992, and incorporated herein by reference). Peptoids are oligomers of N-substituted glycines. A number of N-alkyl groups have been described, each corresponding to the side chain of a natural amino acid.

### F. POLYNUCLEOTIDES

The invention includes polynucleotides encoding peptides of the invention. Exemplary polynucleotides encode peptides including those listed in Table 1, and analogs, derivatives, amidated variations and conservative variations thereof, wherein the peptides have antimicrobial activity. The peptides of the invention include SEQ ID NOS:1-2166, as well as the broader groups of peptides having hydrophilic and hydrophobic substitutions, and conservative variations thereof.

"Isolated" when used in reference to a polynucleotide, refers to a polynucleotide substantially free of proteins, lipids, nucleic acids, for example, with which it is naturally associated. As used herein, "polynucleotide" refers to a polymer of deoxyribonucleotides or ribonucleotides, in the form of a separate fragment or as a component of a larger construct. DNA encoding a peptide of the invention can be assembled from cDNA fragments or from oligonucleotides which provide a synthetic gene which is capable of being expressed in a recombinant transcriptional unit. Polynucleotide sequences of the invention include DNA, RNA and cDNA sequences. A polynucleotide sequence can be deduced from the genetic code, however, the degeneracy of the code must be taken into account. Polynucleotides of the invention include sequences which are degenerate as a result of the genetic code. Such polynucleotides are useful for the recombinant production of large quantities of a peptide of interest, such as the peptide of SEQ ID NOS: 1-2166.

"Recombinant" when used with reference, *e*.*g*., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

In the present invention, the polynucleotides encoding the peptides of the invention may be inserted into a recombinant "expression vector". The term "expression vector" refers to a plasmid, virus or other vehicle known in the art that has been manipulated by insertion or incorporation of genetic sequences. Such expression vectors of the invention are preferably plasmids that contain a promoter sequence that facilitates the efficient transcription of the inserted genetic sequence in the host. The expression vector typically contains an origin of replication, a promoter, as well as specific genes that allow phenotypic selection of the transformed cells. For example, the expression of the peptides of the invention can be placed under control of E. coli chromosomal DNA comprising a lactose or lac operon which mediates lactose utilization by elaborating the enzyme beta-galactosidase. The lac control system can be induced by IPTG. A plasmid can be constructed to contain the lac Iq repressor gene, permitting repression of the lac promoter until IPTG is added. Other promoter systems known in the art include beta lactamase, lambda promoters, the protein A promoter, and the tryptophan promoter systems. While these are the most commonly used, other microbial promoters, both inducible and constitutive, can be utilized as well. The vector contains a replicon site and control sequences which are derived from species compatible with the host cell. In addition, the vector may carry specific gene(s) which are capable of providing phenotypic selection in transformed cells. For example, the beta-lactamase gene confers ampicillin resistance to those transformed cells containing the vector with the beta-lactamase gene. An exemplary expression system for production of the peptides of the invention is described in U.S. Pat. No. 5,707,855.

Transformation of a host cell with the polynucleotide may be carried out by conventional techniques known to those skilled in the art. For example, where the host is prokaryotic, such as E. coli, competent cells that are capable of DNA uptake can be prepared from cells harvested after exponential growth and subsequently treated by the CaCl₂ method using procedures known in the art. Alternatively, MgCl₂ or RbCl could be used.

In addition to conventional chemical methods of transformation, the plasmid vectors of the invention may be introduced into a host cell by physical means, such as by electroporation or microinjection. Electroporation allows transfer of the vector by high voltage electric impulse, which creates pores in the plasma membrane of the host and is performed according to methods known in the art. Additionally, cloned DNA can be introduced into host cells by protoplast fusion, using methods known in the art.

DNA sequences encoding the peptides can be expressed *in vivo* by DNA transfer into a suitable host cell. "Host cells" of the invention are those in which a vector can be propagated and its DNA expressed. The term also includes any progeny of the subject host cell. It is understood that not all progeny are identical to the parental cell, since there may be mutations that occur during replication. However, such progeny are included when the terms above are used. Preferred host cells of the invention include *E. coli, S. aureus* and *P. aeruginosa*, although other Gram-negative and Gram-positive organisms known in the art can be utilized as long as the expression vectors contain an origin of replication to permit expression in the host.

The polynucleotide sequence encoding the peptide used according to the method of the invention can be isolated from an organism or synthesized in the laboratory. Specific DNA sequences encoding the peptide of interest can be obtained by: 1) isolation of a double-stranded DNA sequence from the genomic DNA; 2) chemical manufacture of a DNA sequence to provide the necessary codons for the peptide of interest; and 3) *in vitro* synthesis of a double-stranded DNA sequence by reverse transcription of mRNA isolated from a donor cell. In the latter case, a double-stranded DNA complement of mRNA is eventually formed that is generally referred to as cDNA.

The synthesis of DNA sequences is frequently the method of choice when the entire sequence of amino acid residues of the desired peptide product is known. In the present invention, the synthesis of a DNA sequence has the advantage of allowing the incorporation of codons that are more likely to be recognized by a bacterial host, thereby permitting high level expression without difficulties in translation. In addition, virtually any peptide can be synthesized, including those encoding natural peptides, variants of the same, or synthetic peptides.

When the entire sequence of the desired peptide is not known, the direct synthesis of DNA sequences is not possible and the method of choice is the formation of cDNA sequences. Among the standard procedures for isolating cDNA sequences of interest is the formation of plasmid or phage containing cDNA libraries that are derived from reverse transcription of mRNA that is abundant in donor cells that have a high level of genetic expression. When used in combination with polymerase chain reaction technology, even rare expression products can be cloned. In those cases where significant portions of the amino acid sequence of the peptide are known, the production of labeled single or double-stranded DNA or RNA probe sequences duplicating a sequence putatively present in the target cDNA may be employed in DNA/DNA hybridization procedures which are carried out on cloned copies of the cDNA which have been denatured into a single stranded form. Jay et al., Nuc. Acid Res. 11: 2325, 1983.

### G. METHODS OF USE

The invention also provides a method of inhibiting the growth of bacteria including contacting the bacteria with an inhibiting effective amount of a peptide of the invention, including SEQ ID NOS: 1-2166, and analogs, derivatives, amidated variations and conservative variations thereof, wherein the peptides have antimicrobial activity.

The term "contacting" refers to exposing the bacteria to the peptide so that the peptide can effectively inhibit, kill, or lyse bacteria, bind endotoxin (LPS), or permeabilize Gram-negative bacterial outer membranes. Contacting may be *in vitro*, for example by adding the peptide to a bacterial culture to test for susceptibility of the bacteria to the peptide. Contacting may be *in vivo,* for example administering the peptide to a subject with a bacterial disorder, such as septic shock or infection. Contacting may further involve coating an object (*e*.*g*., medical device) such as a catheter to inhibit bacteria with which it comes into contact, thus preventing it from becoming colonized with the bacteria. "Inhibiting" or "inhibiting effective amount" refers to the amount of peptide that is required to cause a bacteriostatic or bactericidal effect. Examples of bacteria that may be inhibited include Escherichia coli, *Pseudomonas aeruginosa, Enterobacter cloacae, Salmonella enteritidis* subspecies Typhimurium, *Staphylococcus aureus, Enterococcus facaelis, Listeria monocytogenes, Corynebacterium xerosis, Streptococcus pyogenes, Streptococcus pneumoniae, Streptococcus mitis* and *Staphylococcuus epidermidis*.

The method of inhibiting the growth of bacteria may further include the addition of antibiotics for combination or synergistic therapy. The appropriate antibiotic administered will typically depend on the susceptibility of the bacteria such as whether the bacteria is Gram negative or Gram positive, and will be easily discernable by one of skill in the art. Examples of particular classes of antibiotics useful for synergistic therapy with the peptides of the invention include aminoglycosides (*e*.*g*., tobramycin), penicillins (*e*.*g*., piperacillin), cephalosporins (*e*.*g*., ceftazidime), fluoroquinolones (*e*.*g*., ciprofloxacin), carbapenems (*e*.*g*., imipenem), tetracyclines and macrolides (*e*.*g*., erythromycin and clarithromycin). The method of inhibiting the growth of bacteria may further include the addition of antibiotics for combination or synergistic therapy. The appropriate antibiotic administered will typically depend on the susceptibility of the bacteria such as whether the bacteria is Gram negative or Gram positive, and will be easily discernable by one of skill in the art. Further to the antibiotics listed above, typical antibiotics include aminoglycosides (amikacin, gentamicin, kanamycin, netilmicin, t-obramycin, streptomycin), macrolides ( azithromycin, clarithromycin, erythromycin, erythromycin estolate/ethylsuccinate/ gluceptate/lactobionate/stearate), beta-lactams such as penicillins (*e*.*g*., penicillin G, penicillin V, methicillin, nafcillin, oxacillin, cloxacillin, dicloxacillin, ampicillin, amoxicillin, ticarcillin, carbenicillin, mezlocillin, azlocillin and piperacillin), or cephalosporins (*e*.*g*., cephalothin, cefazolin, cefaclor, cefamandole, cefoxitin, cefuroxime, cefonicid, cefmetazole, cefotetan, cefprozil, loracarbef, cefetamet, cefoperazone, cefotaxime, ceftizoxime, ceftriaxone, ceftazidime, cefepime, cefixime, cefpodoxime, and cefsulodin) or carbapenems (*e*.*g*., imipenem, meropenem, panipenem), or monobactams (*e*.*g*., aztreonam). Other classes of antibiotics include quinolones (*e*.*g*., fleroxacin, nalidixic acid, norfloxacin, ciprofloxacin, ofloxacin, enoxacin, lomefloxacin and cinoxacin), tetracyclines (*e*.*g*., doxycycline, minocycline, tetracycline), and glycopeptides (*e*.*g*., vancomycin, teicoplanin), for example. Other antibiotics include chloramphenicol, clindamycin, trimethoprim, sulfamethoxazole, nitrofurantoin, rifampin, linezolid, synercid, polymyxin B, colisitin, colimycin, methotrexate, daptomycin, phosphonomycin and mupirocin.

The peptides and/or analogs or derivatives thereof may be administered to any host, including a human or non-human animal, in an amount effective to inhibit not only growth of a bacterium, but also a virus, parasite or fungus. These peptides are useful as antimicrobial agents, antiviral agents, and antifungal agents. The peptides and/or analogs or derivatives thereof may be administered to any host, including a human or non-human animal, in an amount effective to inhibit not only growth of a bacterium, but also a virus or fungus. These peptides are useful as antimicrobial agents, antiviral agents, and antifungal agents.

In addition to being active against a broad range of pathogens, bactenecin has been shown to be cytotoxic to rat embryonic neurons, fetal rat astrocytes and human glioblastoma cells. Radermacher et al., J. Neuro. Res. 36: 657, 1993. Thus, it is envisioned that the peptides of the present invention can be used to inhibit the growth of a eukaryotic cell by contacting the eukaryotic cell with an inhibiting effective amount of a peptide of the invention. Such a method would be useful, for example, for inhibiting a cell proliferation-associated disorder in a subject having or at risk of having such a disorder. The method can involve, for example, administering to the subject a therapeutically effective amount of a peptide of the present invention to inhibit the over-growth of cells in a subject in need of such treatment. Such disorders would include, for example, neurological related disorders.

The invention further provides a method of protecting objects from bacterial colonization. The peptides of the invention remain active when conjugated to solid surfaces. Thus, the peptides may be used for protecting objects such as medical devices from colonization with pathogenic bacteria by chemically conjugating, or coating by any other means, at least one peptide of the invention to the surface of the medical device. Such medical devices include indwelling catheters, and the like.

### H. TREATMENT REGIMES

The invention provides pharmaceutical compositions comprising one or a combination of antimicrobial peptides, for example, formulated together with a pharmaceutically acceptable carrier. Some compositions include a combination of multiple (*e*.*g*., two or more) peptides of the invention. Some compositions include a combination of a peptide and/or peptides of the invention together with other drugs or agents (*i*.*e*., antimicrobial drugs and/or antimicrobial agents).

As used herein "pharmaceutically acceptable carrier" and "pharmaceutically acceptable excipient" include any and all solvents, dispersion media, coatings, antibacterial and antifungal agents, isotonic and absorption delaying agents, and the like that are physiologically compatible. In one embodiment, the carrier is suitable for parenteral administration. Alternatively, the carrier can be suitable for intravenous, intraperitoneal or intramuscular administration. In another embodiment, the carrier is suitable for oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is compatible with the active compound, use thereof in the pharmaceutical compositions is contemplated. Supplementary active compounds can also be incorporated into the compositions.

"Pharmaceutically acceptable salts and esters" means salts and esters that are pharmaceutically acceptable and have the desired pharmacological properties. Such salts include salts that can be formed where acidic protons present in the compounds are capable of reacting with inorganic or organic bases. Suitable inorganic salts include those formed with the alkali metals, *e*.*g*. sodium and potassium, magnesium, calcium, and aluminum. Suitable organic salts include those formed with organic bases such as the amine bases, *e*.*g*. ethanolamine, diethanolamine, triethanolamine, tromethamine, N methylglucamine, and the like. Such salts also include acid addition salts formed with inorganic acids (*e*.*g*., hydrochloric and hydrobromic acids) and organic acids (*e*.*g*. acetic acid, citric acid, maleic acid, and the alkane- and arene-sulfonic acids such as methanesulfonic acid and benzenesulfonic acid). Pharmaceutically acceptable esters include esters formed from carboxy, sulfonyloxy, and phosphonoxy groups present in the compounds, *e*.*g*. C1-6 alkyl esters. When there are two acidic groups present, a pharmaceutically acceptable salt or ester can be a mono-acid-mono-salt or ester or a di-salt or ester; and similarly where there are more than two acidic groups present, some or all of such groups can be salified or esterified. Compounds named in this invention can be present in unsalified or unesterified form, or in salified and/or esterified form, and the naming of such compounds is intended to include both the original (unsalified and unesterified) compound and its pharmaceutically acceptable salts and esters. Also, certain compounds named in this invention may be present in more than one stereoisomeric form, and the naming of such compounds is intended to include all single stereoisomers and all mixtures (whether racemic or otherwise) of such stereoisomers.

"Pharmaceutically acceptable", "physiologically tolerable" and grammatical variations thereof, as they refer to compositions, carriers, diluents and reagents, are used interchangeably and represent that the materials are capable of administration to or upon a human without the production of undesirable physiological effects to a degree that would prohibit administration of the composition.

In prophylactic applications, pharmaceutical compositions or medicaments are administered to a patient susceptible to, or otherwise at risk of a disease or condition (*i*.*e*., as a result of bacteria, fungi, viruses, parasites or the like) in an amount sufficient to eliminate or reduce the risk, lessen the severity, or delay the outset of the disease, including biochemical, histologic and/or behavioral symptoms of the disease, its complications and intermediate pathological phenotypes presenting during development of the disease. In therapeutic applications, compositions or medicants are administered to a patient suspected of, or already suffering from such a disease or condition in an amount sufficient to cure, or at least partially arrest, the symptoms of the disease or condition (*e*.*g*., biochemical and/or histologic), including its complications and intermediate pathological phenotypes in development of the disease or condition. An amount adequate to accomplish therapeutic or prophylactic treatment is defined as a therapeutically- or prophylactically-effective dose. In both prophylactic and therapeutic regimes, agents are usually administered in several dosages until a sufficient response has been achieved. Typically, the response is monitored and repeated dosages are given if the response starts to wane.

The pharmaceutical composition of the present invention should be sterile and fluid to the extent that the composition is deliverable by syringe. In addition to water, the carrier can be an isotonic buffered saline solution, ethanol, polyol (for example, glycerol, propylene glycol, and liquid polyetheylene glycol, and the like), and suitable mixtures thereof. Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition. Long-term absorption of the injectable compositions can be brought about by including in the composition an agent which delays absorption, for example, aluminum monostearate or gelatin.

When the active compound is suitably protected, as described above, the compound can be orally administered, for example, with an inert diluent or an assimilable edible carrier.

Pharmaceutical compositions of the invention also can be administered in combination therapy, *i*.*e*., combined with other agents. For example, in treatment of bacteria, the combination therapy can include a composition of the present invention with at least one agent or other conventional therapy. "Concomitant administration" of a known antimicrobial drug with a pharmaceutical composition of the present invention means administration of the drug and the peptide and/or protein composition at such time that both the known drug and the composition of the present invention will have a therapeutic effect. Such concomitant administration can involve concurrent (*i*.*e*., at the same time), prior, or subsequent administration of the antimicrobial drug with respect to the administration of a compound of the present invention. A person of ordinary skill in the art, would have no difficulty determining the appropriate timing, sequence and dosages of administration for particular drugs and compositions of the present invention

### I. ROUTES OF ADMINISTRATION

A composition of the present invention can be administered by a variety of methods known in the art. The route and/or mode of administration vary depending upon the desired results. The phrases "parenteral administration" and "administered parenterally" mean modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. The peptide of the invention can be administered parenterally by injection or by gradual infusion over time. The peptide can also be prepared with carriers that protect the compound against rapid release, such as a controlled release formulation, including implants, transdermal patches, and microencapsulated delivery systems Further methods for delivery of the peptide include orally, by encapsulation in microspheres or proteinoids, by aerosol delivery to the lungs, or transdermally by iontophoresis or transdermal electroporation. To administer a peptide of the invention by certain routes of administration, it can be necessary to coat the compound with, or co-administer the compound with, a material to prevent its inactivation. The method of the invention also includes delivery systems such as microencapsulation of peptides into liposomes or a diluent. Microencapsulation also allows co-entrapment of antimicrobial molecules along with the antigens, so that these molecules, such as antibiotics, may be delivered to a site in need of such treatment in conjunction with the peptides of the invention. Liposomes in the blood stream are generally taken up by the liver and spleen. Pharmaceutically acceptable diluents include saline and aqueous buffer solutions. Liposomes include water-in-oil-in-water CGF emulsions as well as conventional liposomes. Strejan et al., J. Neuroimmunol. 7: 27, 1984. Thus, the method of the invention is particularly useful for delivering antimicrobial peptides to such organs. Biodegradable, biocompatible polymers can be used, such as ethylene vinyl acetate, polyanhydrides, polyglycolic acid, collagen, polyorthoesters, and polylactic acid. Many methods for the preparation of such formulations are described by *e*.*g*., Robinson, 1978, Sustained and Controlled Release Drug Delivery Systems. Other methods of administration will be known to those skilled in the art.

Preparations for parenteral administration of a peptide of the invention include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Therapeutic compositions typically must be sterile, substantially isotonic, and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it is preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof. Therapeutic compositions can also be administered with medical devices known in the art. For example, in a preferred embodiment, a therapeutic composition of the invention can be administered with a needleless hypodermic injection device, such as the devices disclosed in, *e*.*g*., U.S. Patent Nos. 5,399,163, 5,383,851, 5,312,335, 5,064,413, 4,941,880, 4,790,824, or 4,596,556. Examples of implants and modules useful in the present invention include: U.S. Patent No. 4,487,603, which discloses an implantable micro-infusion pump for dispensing medication at a controlled rate; U.S. Patent No. 4,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. Many other such implants, delivery systems, and modules are known.

When the peptides of the present invention are administered as pharmaceuticals, to humans and animals, they can be given alone or as a pharmaceutical composition containing, for example, 0.01 to 99.5% (or 0.1 to 90%) of active ingredient in combination with a pharmaceutically acceptable carrier.

### J. EFFECTIVE DOSAGES

"Therapeutically effective amount" as used herein for treatment of antimicrobial related diseases and conditions refers to the amount of peptide used that is of sufficient quantity to decrease the numbers of bacteria, viruses, fungi, and parasites in the body of a subject. The dosage ranges for the administration of peptides are those large enough to produce the desired effect. The amount of peptide adequate to accomplish this is defined as a "therapeutically effective dose." The dosage schedule and amounts effective for this use, *i*.*e*., the "dosing regimen," will depend upon a variety of factors, including the stage of the disease or condition, the severity of the disease or condition, the general state of the patient's health, the patient's physical status, age, pharmaceutical formulation and concentration of active agent, and the like. In calculating the dosage regimen for a patient, the mode of administration also is taken into consideration. The dosage regimen must also take into consideration the pharmacokinetics, *i*.*e*., the pharmaceutical composition's rate of absorption, bioavailability, metabolism, clearance, and the like. See, *e*.*g*., the latest Remington's (Remington's Pharmaceutical Science, Mack Publishing Company, Easton, PA); Egleton, Peptides 18: 1431-1439, 1997; Langer, Science 249: 1527-1533, 1990. The dosage regimen can be adjusted by the individual physician in the event of any contraindications.

Dosage regimens of the pharmaceutical compositions of the present invention are adjusted to provide the optimum desired response (*e*.*g*., a therapeutic response). For example, a single bolus can be administered, several divided doses can be administered over time or the dose can be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

Actual dosage levels of the active ingredients in the pharmaceutical compositions of the present invention can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

A physician or veterinarian can start doses of the compounds of the invention employed in the pharmaceutical composition at levels lower than that required to achieve the desired therapeutic effect and gradually increase the dosage until the desired effect is achieved. In general, a suitable daily dose of a compound of the invention is that amount of the compound which is the lowest dose effective to produce a therapeutic effect. Such an effective dose generally depends upon the factors described above. It is preferred that administration be intravenous, intramuscular, intraperitoneal, or subcutaneous, or administered proximal to the site of the target. If desired, the effective daily dose of a therapeutic composition can be administered as two, three, four, five, six or more sub-doses administered separately at appropriate intervals throughout the day, optionally, in unit dosage forms. While it is possible for a compound of the present invention to be administered alone, it is preferable to administer the compound as a pharmaceutical formulation (composition).

An effective dose of each of the peptides disclosed herein as potential therapeutics for use in treating microbial diseases and conditions is from about 1 µg to 500 mg/kg body weight, per single administration, which can readily be determined by one skilled in the art. As discussed above, the dosage depends upon the age, sex, health, and weight of the recipient, kind of concurrent therapy, if any, and frequency of treatment. Other effective dosage range upper limits are 100 mg/kg body weight, 50 mg/kg body weight, 25 mg/kg body weight, and 10 mg/kg body weight.

The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patent can be administered a prophylactic regime.

Some compounds of the invention can be formulated to ensure proper distribution *in vivo*. For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, See, *e*.*g.,* U.S. Patents 4,522,811; 5,374,548; and 5,399,331. The liposomes can comprise one or more moieties which are selectively transported into specific cells or organs, thus enhance targeted drug delivery (See, *e*.*g*., Ranade, J. Clin. Pharmacol. 29: 685, 1989). Exemplary targeting moieties include folate or biotin (See, *e*.*g*., U.S. Patent 5,416,016 to Low et al.,); mannosides (Umezawa et al., Biochem. Biophys. Res. Commun. 153: 1038, 1988); antibodies (Bloeman et al., FEBS Lett. 357: 140, 1995; Owais et al., Antimicrob. Agents Chemother. 39: 180, 1995); surfactant protein A receptor (Briscoe et al., Am. J. Physiol. 1233: 134, 1995), different species of which can comprise the formulations of the inventions, as well as components of the invented molecules; p120 (Schreier et al., J. Biol. Chem. 269: 9090, 1994); See also Keinanen et al., FEBS Lett. 346: 123, 1994; Killion et al., Immunomethods 4: 273, 1994. In some methods, the therapeutic compounds of the invention are formulated in liposomes; in a more preferred embodiment, the liposomes include a targeting moiety. In some methods, the therapeutic compounds in the liposomes are delivered by bolus injection to a site proximal to the tumor or infection. The composition should be fluid to the extent that easy syringability exists. It should be stable under the conditions of manufacture and storage and should be preserved against the contaminating action of microorganisms such as bacteria and fungi.

"Bactericidal amount" as used herein refers to an amount sufficient to achieve a bacteria-killing blood concentration in the subject receiving the treatment. The bactericidal amount of antibiotic generally recognized as safe for administration to a human is well known in the art, and as is known in the art, varies with the specific antibiotic and the type of bacterial infection being treated.

Because of the antibiotic, antimicrobial, and antiviral properties of the peptides, they may also be used as preservatives or sterillants of materials susceptible to microbial or viral contamination. The peptides of the invention can be utilized as broad spectrum antimicrobial agents directed toward various specific applications. Such applications include use of the peptides as preservatives in processed foods (organisms including Salmonella, Yersinia, Shigella), either alone or in combination with antibacterial food additives such as lysozymes; as a topical agent (Pseudomonas, Streptococcus) and to kill odor producing microbes (Micrococci). The relative effectiveness of the peptides of the invention for the applications described can be readily determined by one of skill in the art by determining the sensitivity of any organism to one of the peptides.

### K. FORMULATION

Typically, compositions are prepared as injectables, either as liquid solutions or suspensions; solid forms suitable for solution in, or suspension in, liquid vehicles prior to injection can also be prepared. The preparation also can be emulsified or encapsulated in liposomes or micro particles such as polylactide, polyglycolide, or copolymer for enhanced adjuvant effect, as discussed above. Langer, Science 249: 1527, 1990 and Hanes, Advanced Dnig Delivery Reviews 28: 97-119, 1997. The agents of this invention can be administered in the form of a depot injection or implant preparation which can be formulated in such a manner as to permit a sustained or pulsatile release of the active ingredient.

Additional formulations suitable for other modes of administration include oral, intranasal, and pulmonary formulations, suppositories, and transdermal applications.

For suppositories, binders and carriers include, for example, polyalkylene glycols or triglycerides; such suppositories can be formed from mixtures containing the active ingredient in the range of 0.5% to 10%, preferably 1%-2%. Oral formulations include excipients, such as pharmaceutical grades of mannitol, lactose, starch, magnesium stearate, sodium saccharine, cellulose, and magnesium carbonate. These compositions take the form of solutions, suspensions, tablets, pills, capsules, sustained release formulations or powders and contain 10%-95% of active ingredient, preferably 25%-70%.

Topical application can result in transdermal or intradermal delivery. Topical administration can be facilitated by co-administration of the agent with cholera toxin or detoxified derivatives or subunits thereof or other similar bacterial toxins. Glenn et al., Nature 391: 851, 1998. Co-administration can be achieved by using the components as a mixture or as linked molecules obtained by chemical crosslinking or expression as a fusion protein.

Alternatively, transdermal delivery can be achieved using a skin patch or using transferosomes. Paul et al., Eur. J. Immunol. 25: 3521-24, 1995; Cevc et al., Biochem. Biophys. Acta 1368: 201-15, 1998.

The pharmaceutical compositions are generally formulated as sterile, substantially isotonic and in full compliance with all Good Manufacturing Practice (GMP) regulations of the U.S. Food and Drug Administration.

From the foregoing description, various modifications and changes in the compositions and methods will occur to those skilled in the art. All such modifications coming within the scope of the appended claims are intended to be included therein. Each recited range includes all combinations and sub-combinations of ranges, as well as specific numerals contained therein.

All publications and patent documents cited above are hereby incorporated by reference in their entirety for all purposes to the same extent as if each were so individually denoted.

Although the foregoing invention has been described in detail by way of example for purposes of clarity of understanding, it will be apparent to the artisan that certain changes and modifications are comprehended by the disclosure and can be practiced without undue experimentation within the scope of the appended claims, which are presented by way of illustration not limitation.

### EXEMPLARY EMBODIMENTS

### EXAMPLE 1

### MATERIALS AND METHODS AND PEPTIDES

Peptide Synthesis - Peptide syntheses on cellulose were performed using a pipetting robot (Abimed, Langenfeld, Germany) and Whatman 50 cellulose membranes (Whatman, Maidstone, United Kingdom) as described previously. Kramer et al., Comp. Meth. Enzymol. 6: 388-395, 1994; Kramer et al., Cell 91: 799-809, 1997. The HPLC purified peptides used for further characterization (CD, membrane permeability, MIC) were purchased from Thermo Electron Corperation (Ulm, Germany).

**TABLE 1**

| | | |
|---|---|---|
| Bac001 = Bac2A | RLARIVVIRVAR | SEQ ID NO: 1 |
| Bac002 | RRIARVIVAVLR | SEQ ID NO: 2 |
| Bac003 | ARRLIVRVRVIA | SEQ ID NO: 3 |
| Bac004 | IRARIAVRRVVL | SEQ ID NO: 4 |
| Bac005 | IVRVAVALRRIR | SEQ ID NO: 5 |
| Bac006 | VRIRARRVILVA | SEQ ID NO: 6 |
| Bac007 | RRLVAIVAVRRI | SEQ ID NO: 7 |
| Bac008 | VLIRIRRVARAV | SEQ ID NO: 8 |
| Bac009 | IIRAALRRVRVV | SEQ ID NO: 9 |
| Bac010 | AAVRRRVRLVII | SEQ ID NO: 10 |
| Bac011 | AVRVRRRAILVI | SEQ ID NO: 11 |
| Bac012 | IAARRLIRVVRV | SEQ ID NO: 12 |
| Bac013 | VARIVVRLIRAR | SEQ ID NO: 13 |
| Bac014 | RAVAVIIRLRRV | SEQ ID NO: 14 |
| Bac015 | AVRAIRVLRVIR | SEQ ID NO: 15 |
| Bac016 | RARIVRVRVILA | SEQ ID NO: 16 |
| Bac017 | VILARRRVRIAV | SEQ ID NO: 17 |
| Bac018 | RRVAIVVIARLR | SEQ ID NO: 18 |
| Bac019 | ILVARVIRRRVA | SEQ ID NO: 19 |
| Bac020 | RRAAVVLIVIRR | SEQ ID NO: 20 |
| Bac021 | ARIARRVRILVV | SEQ ID NO: 21 |
| Bac022 | ILRRVRVRAVAI | SEQ ID NO: 22 |
| Bac023 | RRRAIVRVVAIL | SEQ ID NO: 23 |
| Bac024 | RAIIRRVLVRVA | SEQ ID NO: 24 |
| Bac025 | ARAILIRVVRRV | SEQ ID NO: 25 |
| Bac026 | IARRIVAVRLRV | SEQ ID NO: 26 |
| Bac027 | RVLIARVVRAIR | SEQ ID NO: 27 |
| Bac028 | VIVRLAARRVRI | SEQ ID NO: 28 |
| Bac029 | IILAVRAVRRVR | SEQ ID NO: 29 |
| Bac030 | IVVRRRRAALVI | SEQ ID NO: 30 |
| B ac031 | LAIVRRARVRIV | SEQ ID NO: 31 |
| Bac032 | ARRARIRILVVV | SEQ ID NO: 32 |
| Bac033 | IRVRRLVAAVIR | SEQ ID NO: 33 |
| Bac034 | VRLRIRVAVIRA | SEQ ID NO: 34 |
| Bac035 | RVLRVVRAAIRI | SEQ ID NO: 35 |
| Bac036 | RARRVRVLIAIV | SEQ ID NO: 36 |
| Bac037 | RAIRVRRIVLAV | SEQ ID NO: 37 |
| Bac038 | VVIRAAIRRVRL | SEQ ID NO: 38 |
| Bac039 | RIVLRRAAVIRV | SEQ ID NO: 39 |
| Bac040 | VLARVVARRIRI | SEQ ID NO: 40 |
| Bac041 | RLRVAIVAIVRR | SEQ ID NO: 41 |
| Bac042 | ILVIVRRRARAV | SEQ ID NO: 42 |
| Bac043 | RVLIVIRARRVA | SEQ ID NO: 43 |
| Bac044 | VIRRRRILAAVV | SEQ ID NO: 44 |
| Bac045 | VIALRIAVRRVR | SEQ ID NO: 45 |
| Bac046 | RRRVIVAVLARI | SEQ ID NO: 46 |
| Bac047 | RVLIAARVIRRV | SEQ ID NO: 47 |
| Bac048 | VVRALRRIIARV | SEQ ID NO: 48 |
| Bac049 | VIALVRARVRRI | SEQ ID NO: 49 |
| Bac050 | RRVIAIAVRRLV | SEQ ID NO: 50 |
| Bac101 | RLARIVVIRVA | SEQ ID NO: 51 |
| Bac102 | LARIVVIRVAR | SEQ ID NO: 52 |
| Bac103 | RLARIVVIRV | SEQ ID NO: 53 |
| Bac104 | LARIVVIRVA | SEQ ID NO: 54 |
| Bac105 | ARIVVIRVAR | SEQ ID NO: 55 |
| Bac106 | RLARIVVIR | SEQ ID NO: 56 |
| Bac107 | LARIVVIRV | SEQ ID NO: 57 |
| Bac108 | ARIVVIRVA | SEQ ID NO: 58 |
| Bac109 | RIVVIRVAR | SEQ ID NO: 59 |
| Bac110 | RLARIVVI | SEQ ID NO: 60 |
| Bac111 | LARIVVIR | SEQ ID NO: 61 |
| Bac112 | ARIVVIRV | SEQ ID NO: 62 |
| Bac113 | RIVVIRVA | SEQ ID NO: 63 |
| Bac114 | IVVIRVAR | SEQ ID NO: 64 |
| Bac115 | RLARIVV | SEQ ID NO: 65 |
| Bac116 | LARIVVI | SEQ ID NO: 66 |
| Bac117 | ARIVVIR | SEQ ID NO: 67 |
| Bac118 | RIVVIRV | SEQ ID NO: 68 |
| Bac119 | IVVIRVA | SEQ ID NO: 69 |
| Bac120 | VVIRVAR | SEQ ID NO: 70 |
| Bac121 | RLARIV | SEQ ID NO: 71 |
| Bac122 | LARIVV | SEQ ID NO: 72 |
| Bac123 | ARIVVI | SEQ ID NO: 73 |
| Bac124 | RIVVIR | SEQ ID NO: 74 |
| Bac125 | IVVIRV | SEQ ID NO: 75 |
| Bac126 | VVIRVA | SEQ ID NO: 76 |
| Bac127 | VIRVAR | SEQ ID NO: 77 |
| Bac128 | RLARIV | SEQ ID NO: 78 |
| Bac129 | LARIVV | SEQ ID NO: 79 |
| Bac130 | ARIVVI | SEQ ID NO: 80 |
| Bac131 | RIVVIR | SEQ ID NO: 81 |
| Bac132 | IVVIRV | SEQ ID NO: 82 |
| Bac133 | VVIRVA | SEQ ID NO: 83 |
| Bac134 | VIRVAR | SEQ ID NO: 84 |
| Bac135 | RLARI | SEQ ID NO: 85 |
| Bac136 | LARIV | SEQ ID NO: 86 |
| Bac137 | ARIVV | SEQ ID NO: 87 |
| Bac138 | RIVVI | SEQ ID NO: 88 |
| Bac139 | IVVIR | SEQ ID NO: 89 |
| Bac140 | VVIRV | SEQ ID NO: 90 |
| Bac141 | VIRVA | SEQ ID NO: 91 |
| Bac142 | IRVAR | SEQ ID NO: 92 |
| Bac201 | ALARIVVIRVAR | SEQ ID NO: 93 |
| Bac202 | CLARIVVIRVAR | SEQ ID NO: 94 |
| Bac203 | DLARIVVIRVAR | SEQ ID NO: 95 |
| Bac204 | ELARIVVIRVAR | SEQ ID NO: 96 |
| Bac205 | FLARIVVIRVAR | SEQ ID NO: 97 |
| Bac206 | GLARIVVIRVAR | SEQ ID NO: 98 |
| Bac207 | HLARIVVIRVAR | SEQ ID NO: 99 |
| Bac208 | ILARIVVIRVAR | SEQ ID NO: 100 |
| Bac209 | KLARIVVIRVAR | SEQ ID NO: 101 |
| Bac210 | LLARIVVIRVAR | SEQ ID NO: 102 |
| Bac211 | MLARIVVIRVAR | SEQ ID NO: 103 |
| Bac212 | NLARIVVIRVAR | SEQ ID NO: 104 |
| Bac213 | PLARIVVIRVAR | SEQ ID NO: 105 |
| Bac214 | QLARIVVIRVAR | SEQ ID NO: 106 |
| Bac215 | SLARIVVIRVAR | SEQ ID NO: 107 |
| Bac216 | TLARIVVIRVAR | SEQ ID NO: 108 |
| Bac217 | VLARIVVIRVAR | SEQ ID NO: 109 |
| Bac218 | WLARIVVIRVAR | SEQ ID NO: 110 |
| Bac219 | YLARIVVIRVAR | SEQ ID NO: 111 |
| Bac220 | RAARIVVIRVAR | SEQ ID NO: 112 |
| Bac221 | RCARIVVIRVAR | SEQ ID NO: 113 |
| Bac222 | RDARIVVIRVAR | SEQ ID NO: 114 |
| Bac223 | REARIVVIRVAR | SEQ ID NO: 115 |
| Bac224 | RFARIVVIRVAR | SEQ ID NO: 116 |
| Bac225 | RGARIVVIRVAR | SEQ ID NO: 117 |
| Bac226 | RHARIVVIRVAR | SEQ ID NO: 118 |
| Bac227 | RIARIVVIRVAR | SEQ ID NO: 119 |
| Bac228 | RKARIVVIRVAR | SEQ ID NO: 120 |
| Bac229 | RMARIVVIRVAR | SEQ ID NO: 121 |
| Bac230 | RNARIVVIRVAR | SEQ ID NO: 122 |
| Bac231 | RPARIVVIRVAR | SEQ ID NO: 123 |
| Bac232 | RQARIVVIRVAR | SEQ ID NO: 124 |
| Bac233 | RRARIVVIRVAR | SEQ ID NO: 125 |
| Bac234 | RSARIVVIRVAR | SEQ ID NO: 126 |
| Bac235 | RTARIVVIRVAR | SEQ ID NO: 127 |
| Bac236 | RVARIVVIRVAR | SEQ ID NO: 128 |
| Bac237 | RWARIVVIRVAR | SEQ ID NO: 129 |
| Bac238 | RYARIVVIRVAR | SEQ ID NO: 130 |
| Bac239 | RLCRIVVIRVAR | SEQ ID NO: 131 |
| Bac240 | RLDRIVVIRVAR | SEQ ID NO: 132 |
| Bac241 | RLERIVVIRVAR | SEQ ID NO: 133 |
| Bac242 | RLFRIVVIRVAR | SEQ ID NO: 134 |
| Bac243 | RLGRIVVIRVAR | SEQ ID NO: 135 |
| Bac244 | RLHRIVVIRVAR | SEQ ID NO: 136 |
| Bac245 | RLIRIVVIRVAR | SEQ ID NO: 137 |
| Bac246 | RLKRIVVIRVAR | SEQ ID NO: 138 |
| Bac247 | RLLRIVVIRVAR | SEQ ID NO: 139 |
| Bac248 | RLMRIVVIRVAR | SEQ ID NO: 140 |
| Bac249 | RLNRIVVIRVAR | SEQ ID NO: 141 |
| Bac250 | RLPRIVVIRVAR | SEQ ID NO: 142 |
| Bac251 | RLQRIVVIRVAR | SEQ ID NO: 143 |
| Bac252 | RLRRIVVIRVAR | SEQ ID NO: 144 |
| Bac253 | RLSRIVVIRVAR | SEQ ID NO: 145 |
| Bac254 | RLTRIVVIRVAR | SEQ ID NO: 146 |
| Bac255 | RLVRIVVIRVAR | SEQ ID NO: 147 |
| Bac256 | RLWRIVVIRVAR | SEQ ID NO: 148 |
| Bac257 | RLYRIVVIRVAR | SEQ ID NO: 149 |
| Bac258 | RLAAIVVIRVAR | SEQ ID NO: 150 |
| Bac259 | RLACIVVIRVAR | SEQ ID NO: 151 |
| Bac260 | RLADIVVIRVAR | SEQ ID NO: 152 |
| Bac261 | RLAEIVVIRVAR | SEQ ID NO: 153 |
| Bac262 | RLAFIVVIRVAR | SEQ ID NO: 154 |
| Bac263 | RLAGIVVIRVAR | SEQ ID NO: 155 |
| Bac264 | RLAHIVVIRVAR | SEQ ID NO: 156 |
| Bac265 | RLAIIVVIRVAR | SEQ ID NO: 157 |
| Bac266 | RLAKIVVIRVAR | SEQ ID NO: 158 |
| Bac267 | RLALIVVIRVAR | SEQ ID NO: 159 |
| Bac268 | RLAMIVVIRVAR | SEQ ID NO: 160 |
| Bac269 | RLANIVVIRVAR | SEQ ID NO: 161 |
| Bac270 | RLAPIVVIRVAR | SEQ ID NO: 162 |
| Bac271 | RLAQIVVIRVAR | SEQ ID NO: 163 |
| Bac272 | RLASIVVIRVAR | SEQ ID NO: 164 |
| Bac273 | RLATIVVIRVAR | SEQ ID NO: 165 |
| Bac274 | RLAVIVVIRVAR | SEQ ID NO: 166 |
| Bac275 | RLAWIVVIRVAR | SEQ ID NO: 167 |
| Bac276 | RLAYIVVIRVAR | SEQ ID NO: 168 |
| Bac277 | RLARAVVIRVAR | SEQ ID NO: 169 |
| Bac278 | RLARCVVIRVAR | SEQ ID NO: 170 |
| Bac279 | RLARDVVIRVAR | SEQ ID NO: 171 |
| Bac280 | RLAREVVIRVAR | SEQ ID NO: 172 |
| Bac281 | RLARFVVIRVAR | SEQ ID NO: 173 |
| Bac282 | RLARGVVIRVAR | SEQ ID NO: 174 |
| Bac283 | RLARHVVIRVAR | SEQ ID NO: 175 |
| Bac284 | RLARKVVIRVAR | SEQ ID NO: 176 |
| Bac285 | RLARLVVIRVAR | SEQ ID NO: 177 |
| Bac286 | RLARMVVIRVAR | SEQ ID NO: 178 |
| Bac287 | RLARNVVIRVAR | SEQ ID NO: 179 |
| Bac288 | RLARPVVIRVAR | SEQ ID NO: 180 |
| Bac289 | RLARQVVIRVAR | SEQ ID NO: 181 |
| Bac290 | RLARRVVIRVAR | SEQ ID NO: 182 |
| Bac291 | RLARSVVIRVAR | SEQ ID NO: 183 |
| Bac292 | RLARTVVIRVAR | SEQ ID NO: 184 |
| Bac293 | RLARVVVIRVAR | SEQ ID NO: 185 |
| Bac294 | RLARWVVIRVAR | SEQ ID NO: 186 |
| Bac295 | RLARYVVIRVAR | SEQ ID NO: 187 |
| Bac296 | RLARIAVIRVAR | SEQ ID NO: 188 |
| Bac297 | RLARICVIRVAR | SEQ ID NO: 189 |
| Bac298 | RLARIDVIRVAR | SEQ ID NO: 190 |
| Bac299 | RLARIEVIRVAR | SEQ ID NO: 191 |
| Bac300 | RLARIFVIRVAR | SEQ ID NO: 192 |
| Bac301 | RLARIGVIRVAR | SEQ ID NO: 193 |
| Bac302 | RLARIHVIRVAR | SEQ ID NO: 194 |
| Bac303 | RLARIIVIRVAR | SEQ ID NO: 195 |
| Bac304 | RLARIKVIRVAR | SEQ ID NO: 196 |
| Bac305 | RLARILVIRVAR | SEQ ID NO: 197 |
| Bac306 | RLARIMVIRVAR | SEQ ID NO: 198 |
| Bac307 | RLARINVIRVAR | SEQ ID NO: 199 |
| Bac308 | RLARIPVIRVAR | SEQ ID NO: 200 |
| Bac309 | RLARIQVIRVAR | SEQ ID NO: 201 |
| Bac310 | RLARIRVIRVAR | SEQ ID NO: 202 |
| Bac311 | RLARISVIRVAR | SEQ ID NO: 203 |
| Bac312 | RLARITVIRVAR | SEQ ID NO: 204 |
| Bac313 | RLARIWVIRVAR | SEQ ID NO: 205 |
| Bac314 | RLARIYVIRVAR | SEQ ID NO: 206 |
| Bac315 | RLARIVAIRVAR | SEQ ID NO: 207 |
| Bac316 | RLARIVCIRVAR | SEQ ID NO: 208 |
| Bac317 | RLARIVDIRVAR | SEQ ID NO: 209 |
| Bac318 | RLARIVEIRVAR | SEQ ID NO: 210 |
| Bac319 | RLARIVFIRVAR | SEQ ID NO: 211 |
| Bac320 | RLARIVGIRVAR | SEQ ID NO: 212 |
| Bac321 | RLARIVHIRVAR | SEQ ID NO: 213 |
| Bac322 | RLARIVIIRVAR | SEQ ID NO: 214 |
| Bac323 | RLARIVKIRVAR | SEQ ID NO: 215 |
| Bac324 | RLARIVLIRVAR | SEQ ID NO: 216 |
| Bac325 | RLARIVMIRVAR | SEQ ID NO: 217 |
| Bac326 | RLARIVNIRVAR | SEQ ID NO: 218 |
| Bac327 | RLARIVPIRVAR | SEQ ID NO: 219 |
| Bac328 | RLARIVQIRVAR | SEQ ID NO: 220 |
| Bac329 | RLARIVRIRVAR | SEQ ID NO: 221 |
| Bac330 | RLARIVSIRVAR | SEQ ID NO: 222 |
| Bac331 | RLARIVTIRVAR | SEQ ID NO: 223 |
| Bac332 | RLARIVWIRVAR | SEQ ID NO: 224 |
| Bac333 | RLARIVYIRVAR | SEQ ID NO: 225 |
| Bac334 | RLARIVVARVAR | SEQ ID NO: 226 |
| Bac335 | RLARIVVCRVAR | SEQ ID NO: 227 |
| Bac336 | RLARIVVDRVAR | SEQ ID NO: 228 |
| Bac337 | RLARIVVERVAR | SEQ ID NO: 229 |
| Bac338 | RLARIVVFRVAR | SEQ ID NO: 230 |
| Bac339 | RLARIVVGRVAR | SEQ ID NO: 231 |
| Bac340 | RLARIVVHRVAR | SEQ ID NO: 232 |
| Bac341 | RLARIVVKRVAR | SEQ ID NO: 233 |
| Bac342 | RLARIVVLRVAR | SEQ ID NO: 234 |
| Bac343 | RLARIVVMRVAR | SEQ ID NO: 235 |
| Bac344 | RLARIVVNRVAR | SEQ ID NO: 236 |
| Bac345 | RLARIVVPRVAR | SEQ ID NO: 237 |
| Bac346 | RLARIVVQRVAR | SEQ ID NO: 238 |
| Bac347 | RLARIVVRRVAR | SEQ ID NO: 239 |
| Bac348 | RLARIVVSRVAR | SEQ ID NO: 240 |
| Bac349 | RLARIVVTRVAR | SEQ ID NO: 241 |
| Bac350 | RLARIVVVRVAR | SEQ ID NO: 242 |
| Bac351 | RLARIVVWRVAR | SEQ ID NO: 243 |
| Bac352 | RLARIVVYRVAR | SEQ ID NO: 244 |
| Bac353 | RLARIVVIAVAR | SEQ ID NO: 245 |
| Bac354 | RLARIVVICVAR | SEQ ID NO: 246 |
| Bac355 | RLARIVVIDVAR | SEQ ID NO: 247 |
| Bac356 | RLARIVVIEVAR | SEQ ID NO: 248 |
| Bac357 | RLARIVVIFVAR | SEQ ID NO: 249 |
| Bac358 | RLARIVVIGVAR | SEQ ID NO: 250 |
| Bac359 | RLARIVVIHVAR | SEQ ID NO: 251 |
| Bac360 | RLARIVVIIVAR | SEQ ID NO: 252 |
| Bac361 | RLARIVVIKVAR | SEQ ID NO: 253 |
| Bac362 | RLARIVVILVAR | SEQ ID NO: 254 |
| Bac363 | RLARIVVIMVAR | SEQ ID NO: 255 |
| Bac364 | RLARIVVINVAR | SEQ ID NO: 256 |
| Bac365 | RLARIVVIPVAR | SEQ ID NO: 257 |
| Bac366 | RLARIVVIQVAR | SEQ ID NO: 258 |
| Bac367 | RLARIVVISVAR | SEQ ID NO: 259 |
| Bac368 | RLARIVVITVAR | SEQ ID NO: 260 |
| Bac369 | RLARIVVIVVAR | SEQ ID NO: 261 |
| Bac370 | RLARIVVIWVAR | SEQ ID NO: 262 |
| Bac371 | RLARIVVIYVAR | SEQ ID NO: 263 |
| Bac372 | RLARIVVIRAAR | SEQ ID NO: 264 |
| Bac373 | RLARIVVIRCAR | SEQ ID NO: 265 |
| Bac374 | RLARIVVIRDAR | SEQ ID NO: 266 |
| Bac375 | RLARIVVIREAR | SEQ ID NO: 267 |
| Bac376 | RLARIVVIRFAR | SEQ ID NO: 268 |
| Bac377 | RLARIVVIRGAR | SEQ ID NO: 269 |
| Bac378 | RLARIVVIRHAR | SEQ ID NO: 270 |
| Bac379 | RLARIVVIRIAR | SEQ ID NO: 271 |
| Bac380 | RLARIVVIRKAR | SEQ ID NO: 272 |
| Bac381 | RLARIVVIRLAR | SEQ ID NO: 273 |
| Bac382 | RLARIVVIRMAR | SEQ ID NO: 274 |
| Bac383 | RLARIVVIRNAR | SEQ ID NO: 275 |
| Bac384 | RLARIVVIRPAR | SEQ ID NO: 276 |
| Bac385 | RLARIVVIRQAR | SEQ ID NO: 277 |
| Bac386 | RLARIVVIRRAR | SEQ ID NO: 278 |
| Bac387 | RLARIVVIRSAR | SEQ ID NO: 279 |
| Bac388 | RLARIVVIRTAR | SEQ ID NO: 280 |
| Bac389 | RLARIVVIRWAR | SEQ ID NO: 281 |
| Bac390 | RLARIVVIRYAR | SEQ ID NO: 282 |
| Bac391 | RLARIVVIRVCR | SEQ ID NO: 283 |
| Bac392 | RLARIVVIRVDR | SEQ ID NO: 284 |
| Bac393 | RLARIVVIRVER | SEQ ID NO: 285 |
| Bac394 | RLARIVVIRVFR | SEQ ID NO: 286 |
| Bac395 | RLARIVVIRVGR | SEQ ID NO: 287 |
| Bac396 | RLARIVVIRVHR | SEQ ID NO: 288 |
| Bac397 | RLARIVVIRVIR | SEQ ID NO: 289 |
| Bac398 | RLARIVVIRVKR | SEQ ID NO: 290 |
| Bac399 | RLARIVVIRVLR | SEQ ID NO: 291 |
| Bac400 | RLARIVVIRVMR | SEQ ID NO: 292 |
| Bac401 | RLARIVVIRVNR | SEQ ID NO: 293 |
| Bac402 | RLARIVVIRVPR | SEQ ID NO: 294 |
| Bac403 | RLARIVVIRVQR | SEQ ID NO: 295 |
| Bac404 | RLARIVVIRVRR | SEQ ID NO: 296 |
| Bac405 | RLARIVVIRVSR | SEQ ID NO: 297 |
| Bac406 | RLARIVVIRVTR | SEQ ID NO: 298 |
| Bac407 | RLARIVVIRVVR | SEQ ID NO: 299 |
| Bac408 | RLARIVVIRVWR | SEQ ID NO: 300 |
| Bac409 | RLARIVVIRVYR | SEQ ID NO: 301 |
| Bac410 | RLARIVVIRVAA | SEQ ID NO: 302 |
| Bac411 | RLARIVVIRVAC | SEQ ID NO: 303 |
| Bac412 | RLARIVVIRVAD | SEQ ID NO: 304 |
| Bac413 | RLARIVVIRVAE | SEQ ID NO: 305 |
| Bac414 | RLARIVVIRVAF | SEQ ID NO: 306 |
| Bac415 | RLARIVVIRVAG | SEQ ID NO: 307 |
| Bac416 | RLARIVVIRVAH | SEQ ID NO: 308 |
| Bac417 | RLARIVVIRVAI | SEQ ID NO: 309 |
| Bac418 | RLARIVVIRVAK | SEQ ID NO: 310 |
| Bac419 | RLARIVVIRVAL | SEQ ID NO: 311 |
| Bac420 | RLARIVVIRVAM | SEQ ID NO: 312 |
| Bac421 | RLARIVVIRVAN | SEQ ID NO: 313 |
| Bac422 | RLARIVVIRVAP | SEQ ID NO: 314 |
| Bac423 | RLARIVVIRVAQ | SEQ ID NO: 315 |
| Bac424 | RLARIVVIRVAS | SEQ ID NO: 316 |
| Bac425 | RLARIVVIRVAT | SEQ ID NO: 317 |
| Bac426 | RLARIVVIRVAV | SEQ ID NO: 318 |
| Bac427 | RLARIVVIRVAW | SEQ ID NO: 319 |
| Bac428 | RLARIVVIRVAY | SEQ ID NO: 320 |
| Bac429 | GATPEDLNQKLS | SEQ ID NO: 321 |
| Bac430 | RRWRIVVIRVRR | SEQ ID NO: 322 |
| Bac431 | RRWKIVVIRWRR | SEQ ID NO: 323 |
| Bac432 | RWWKIWVIRWWR | SEQ ID NO: 324 |
| Bac433 | RLRRIVVIRVRR | SEQ ID NO: 325 |
| Bac434 | KIWVIRWR | SEQ ID NO: 326 |
| Bac435 | RIWVIRWR | SEQ ID NO: 327 |
| Bac436 | RIWVIWRR | SEQ ID NO: 328 |
| Bac437 | RRWVIWRR | SEQ ID NO: 329 |
| Bac500 | PNGKIIWRM | SEQ ID NO: 330 |
| Bac501 | RRLYMKFKN | SEQ ID NO: 331 |
| Bac502 | SRNGGASIR | SEQ ID NO: 332 |
| Bac503 | KWKSIRGHG | SEQ ID NO: 333 |
| Bac504 | VRRRWIRFW | SEQ ID NO: 334 |
| Bac505 | RIKIWGGGP | SEQ ID NO: 335 |
| Bac506 | VKPTRAWRV | SEQ ID NO: 336 |
| Bac507 | RTKQTTKVR | SEQ ID NO: 337 |
| Bac508 | KWYRWNNAR | SEQ ID NO: 338 |
| Bac509 | HYKPNYWKW | SEQ ID NO: 339 |
| Bac510 | KWSLKHWVV | SEQ ID NO: 340 |
| Bac511 | VWVIRGLGK | SEQ ID NO: 341 |
| Bac512 | GQRVYVRMW | SEQ ID NO: 342 |
| Bac513 | RYWMRTRPW | SEQ ID NO: 343 |
| Bac514 | RGTMLRMFQ | SEQ ID NO: 344 |
| Bac515 | RVGRRHTGK | SEQ ID NO: 345 |
| Bac516 | VDKYRVRFR | SEQ ID NO: 346 |
| Bac517 | WSMPLWKRY | SEQ ID NO: 347 |
| Bac518 | RSIMTQRWW | SEQ ID NO: 348 |
| Bac519 | RWKPTHHLW | SEQ ID NO: 349 |
| Bac520 | WQWKVRIWR | SEQ ID NO: 350 |
| Bac521 | FKGYHWYRR | SEQ ID NO: 351 |
| Bac522 | KKKIIMMMR | SEQ ID NO: 352 |
| Bac523 | KRNMGHWMH | SEQ ID NO: 353 |
| Bac524 | KWSKGVVTN | SEQ ID NO: 354 |
| Bac525 | WGQTHKSRM | SEQ ID NO: 355 |
| Bac526 | KHRKWWKRQ | SEQ ID NO: 356 |
| Bac527 | FYVIWKKGQ | SEQ ID NO: 357 |
| Bac528 | IQIKYIYKS | SEQ ID NO: 358 |
| Bac529 | KRNWVGVRG | SEQ ID NO: 359 |
| Bac530 | YRPWNKGWN | SEQ ID NO: 360 |
| Bac531 | VKPVRVWKF | SEQ ID NO: 361 |
| Bac532 | HPQHFRRWR | SEQ ID NO: 362 |
| Bac533 | KRPHMHHWM | SEQ ID NO: 363 |
| Bac534 | RKLWRWKRT | SEQ ID NO: 364 |
| Bac535 | YHQHKGWIR | SEQ ID NO: 365 |
| Bac536 | PVKRKQRRM | SEQ ID NO: 366 |
| Bac537 | SRTMQNAMR | SEQ ID NO: 367 |
| Bac538 | VYKRLQRGL | SEQ ID NO: 368 |
| Bac539 | TRSVVRKKL | SEQ ID NO: 369 |
| Bac540 | RAGFVMRMR | SEQ ID NO: 370 |
| Bac541 | RRYYWKKGV | SEQ ID NO: 371 |
| Bac542 | WKGRWYKTT | SEQ ID NO: 372 |
| Bac543 | RWIRVAMRD | SEQ ID NO: 373 |
| Bac544 | RPRWWAGFY | SEQ ID NO: 374 |
| Bac545 | WIKWGYRTG | SEQ ID NO: 375 |
| Bac546 | RNVFQRMAG | SEQ ID NO: 376 |
| Bac547 | AGRKRVWHK | SEQ ID NO: 377 |
| Bac548 | MQWGATKIR | SEQ ID NO: 378 |
| Bac549 | LIMGWQRKP | SEQ ID NO: 379 |
| Bac550 | ARSWRNPWF | SEQ ID NO: 380 |
| Bac551 | KVPRPGVMI | SEQ ID NO: 381 |
| Bac552 | MMWRRIGIK | SEQ ID NO: 382 |
| Bac553 | VVRKHSLIK | SEQ ID NO: 383 |
| Bac554 | VWWRGFNRM | SEQ ID NO: 384 |
| Bac555 | QQRRWWSYV | SEQ ID NO: 385 |
| Bac556 | RSAQKRGYI | SEQ ID NO: 386 |
| Bac557 | TRVTRKVTW | SEQ ID NO: 387 |
| Bac558 | IWSWRWWRM | SEQ ID NO: 388 |
| Bac559 | KLTRVYRKY | SEQ ID NO: 389 |
| Bac560 | IKMWRALIR | SEQ ID NO: 390 |
| Bac561 | RWGKWSWRK | SEQ ID NO: 391 |
| Bac562 | WKAVRWKKV | SEQ ID NO: 392 |
| Bac563 | RRIKPVWAW | SEQ ID NO: 393 |
| Bac564 | SVWMKGRYA | SEQ ID NO: 394 |
| Bac565 | VSIMARMKW | SEQ ID NO: 395 |
| Bac566 | RWVGIRVRI | SEQ ID NO: 396 |
| Bac567 | RIQHKKNGY | SEQ ID NO: 397 |
| Bac568 | NMGMRWRAK | SEQ ID NO: 398 |
| Bac569 | RNIQPTRMH | SEQ ID NO: 399 |
| Bac570 | AKLVSRYKR | SEQ ID NO: 400 |
| Bac571 | TRRQRHKPQ | SEQ ID NO: 401 |
| Bac572 | SWMINRYRR | SEQ ID NO: 402 |
| Bac573 | WYRVIHHYK | SEQ ID NO: 403 |
| Bac574 | FTMNIRNRM | SEQ ID NO: 404 |
| Bac575 | WHMSWHKRK | SEQ ID NO: 405 |
| Bac576 | RLFNSKKYK | SEQ ID NO: 406 |
| Bac577 | WKQIVVGKY | SEQ ID NO: 407 |
| Bac578 | KMKEGAKGF | SEQ ID NO: 408 |
| Bac579 | VWGIRKNSS | SEQ ID NO: 409 |
| Bac580 | RGIAVIKMV | SEQ ID NO: 410 |
| Bac581 | YVIIGRGRR | SEQ ID NO: 411 |
| Bac582 | RVKKLRIQP | SEQ NO: 412 |
| Bac583 | KQWSGIKNT | SEQ ID NO: 413 |
| Bac584 | KRRWMWVKR | SEQ ID NO: 414 |
| Bac585 | PHKWNFSKY | SEQ ID NO: 415 |
| Bac586 | FGRWPKLFR | SEQ ID NO: 416 |
| Bac587 | KVYRRHAGY | SEQ ID NO: 417 |
| Bac588 | TYWRRTGPY | SEQ ID NO: 418 |
| Bac589 | KRSKPKYKA | SEQ ID NO: 419 |
| Bac590 | MIRRWKKQW | SEQ ID NO: 420 |
| Bac591 | HQRSRLWHK | SEQ ID NO: 421 |
| Bac592 | WNIFVKGWR | SEQ ID NO: 422 |
| Bac593 | PKTRKKGLY | SEQ ID NO: 423 |
| Bac594 | RSNKGHWYV | SEQ ID NO: 424 |
| Bac595 | MRIWRHWRK | SEQ ID NO: 425 |
| Bac596 | IRGRWKKLR | SEQ ID NO: 426 |
| Bac597 | VKKVRKGIV | SEQ ID NO: 427 |
| Bac598 | MGFFRRRPN | SEQ ID NO: 428 |
| Bac599 | MMKGFMGRW | SEQ ID NO: 429 |
| Bac600 | GRRRLVWTR | SEQ ID NO: 430 |
| Bac601 | VAKRTKAYW | SEQ ID NO: 431 |
| Bac602 | KYKQRVQHI | SEQ ID NO: 432 |
| Bac603 | KKRTYKYPF | SEQ ID NO: 433 |
| Bac604 | FRWKWVKHI | SEQ ID NO: 434 |
| Bac605 | KWRWRVKKR | SEQ ID NO: 435 |
| Bac606 | IIIWGLRRA | SEQ ID NO: 436 |
| Bac607 | NIARRKGFR | SEQ ID NO: 437 |
| Bac608 | SHMHLRKVR | SEQ ID NO: 438 |
| Bac609 | RIGAGNKQG | SEQ ID NO: 439 |
| Bac610 | GGHLRWKNA | SEQ ID NO: 440 |
| Bac611 | RINKVVPRV | SEQ ID NO: 441 |
| Bac612 | WSPRKKQKI | SEQ ID NO: 442 |
| Bac613 | VYSRYMKGG | SEQ ID NO: 443 |
| Bac614 | GKMWRNNYL | SEQ ID NO: 444 |
| Bac615 | KRHWHNSIW | SEQ ID NO: 445 |
| Bac616 | PWTTKNFWR | SEQ ID NO: 446 |
| Bac617 | FPNAVHRRR | SEQ ID NO: 447 |
| Bac618 | PFRAGNTKR | SEQ ID NO: 448 |
| Bac619 | WGRISKRMR | SEQ ID NO: 449 |
| Bac620 | GWRKVRVVV | SEQ ID NO: 450 |
| Bac621 | WGKIVGKGR | SEQ ID NO: 451 |
| Bac622 | MWPGARGAR | SEQ ID NO: 452 |
| Bac623 | IAGRNIWPR | SEQ ID NO: 453 |
| Bac624 | VRTMVKVPM | SEQ ID NO: 454 |
| Bac625 | HKRGWRKGA | SEQ ID NO: 455 |
| Bac626 | SGRWHSKFW | SEQ ID NO: 456 |
| Bac627 | ITWTKIKKF | SEQ ID NO: 457 |
| Bac628 | KRSLMKMWP | SEQ ID NO: 458 |
| Bac629 | VWSRYNKPR | SEQ ID NO: 459 |
| Bac630 | PHVKKGGVA | SEQ ID NO: 460 |
| Bac631 | AYGGTKMRV | SEQ ID NO: 461 |
| Bac632 | HPVKWRRKK | SEQ ID NO: 462 |
| Bac633 | RWVRFVMGI | SEQ ID NO: 463 |
| Bac634 | SIVQKGWFR | SEQ ID NO: 464 |
| Bac635 | NSKTHGFRY | SEQ ID NO: 465 |
| Bac636 | RVVRSARGI | SEQ ID NO: 466 |
| Bac637 | LGKNKATKT | SEQ ID NO: 467 |
| Bac638 | KGFPKSMSG | SEQ ID NO: 468 |
| Bac639 | GKKFWNFWG | SEQ ID NO: 469 |
| Bac640 | MKMKPRKVS | SEQ ID NO: 470 |
| Bac641 | RNVRNNNTR | SEQ ID NO: 471 |
| Bac642 | WRGKPKGLF | SEQ ID NO: 472 |
| Bac643 | KIRNRKIKW | SEQ ID NO: 473 |
| Bac644 | RRRRARGHW | SEQ ID NO: 474 |
| Bac645 | GNMFQWRKG | SEQ ID NO: 475 |
| Bac646 | VRISIMRWW | SEQ ID NO: 476 |
| Bac647 | RVRHGFKWW | SEQ ID NO: 477 |
| Bac648 | WKWKWSKWI | SEQ ID NO: 478 |
| Bac649 | GKNVMMGRI | SEQ ID NO: 479 |
| Bac650 | YWKVKTKHH | SEQ ID NO: 480 |
| Bac651 | IKQWMRRKN | SEQ ID NO: 481 |
| Bac652 | HKKNPWNGK | SEQ ID NO: 482 |
| Bac653 | KRSRIRIGV | SEQ ID NO: 483 |
| Bac654 | QGKWQRPRM | SEQ ID NO: 484 |
| Bac655 | QRSTQRSWW | SEQ ID NO: 485 |
| Bac656 | NRSMRRKPR | SEQ ID NO: 486 |
| Bac657 | HWKRISWGR | SEQ ID NO: 487 |
| Bac658 | RRRWFRTRG | SEQ ID NO: 488 |
| Bac659 | VKSPKWWIG | SEQ ID NO: 489 |
| Bac660 | RIWVWRHKY | SEQ ID NO: 490 |
| Bac661 | WVRPRGFWS | SEQ ID NO: 491 |
| Bac662 | LRSRGSGVH | SEQ ID NO: 492 |
| Bac663 | FHRWWYFFK | SEQ ID NO: 493 |
| Bac664 | RGVFKAMST | SEQ ID NO: 494 |
| Bac665 | WRGGWTHRR | SEQ ID NO: 495 |
| Bac666 | SRMKFWVIK | SEQ ID NO: 496 |
| Bac667 | YMTNRKVSG | SEQ ID NO: 497 |
| Bac668 | WQIGKIWRT | SEQ ID NO: 498 |
| Bac669 | RQVKYINHY | SEQ ID NO: 499 |
| Bac670 | FMWRVKMI | SEQ ID NO: 500 |
| Bac671 | FDRAGRKLI | SEQ ID NO: 501 |
| Bac672 | VKRVKWWWS | SEQ ID NO: 502 |
| Bac673 | RVVFRSQPF | SEQ ID NO: 503 |
| Bac674 | RVKVWSKKF | SEQ ID NO: 504 |
| Bac675 | NAMWKYVWR | SEQ ID NO: 505 |
| Bac676 | RTQQSRPSV | SEQ ID NO: 506 |
| Bac677 | VRYIGAIYR | SEQ ID NO: 507 |
| Bac678 | SIRKWIFWI | SEQ ID NO: 508 |
| Bac679 | NKIVTLPKL | SEQ ID NO: 509 |
| Bac680 | IMARVFPRW | SEQ ID NO: 510 |
| Bac681 | TIRKKWKGS | SEQ ID NO: 511 |
| Bac682 | RRIEKSLRW | SEQ ID NO: 512 |
| Bac683 | RWKSRGRTL | SEQ ID NO: 513 |
| Bac684 | EGNIRKRVY | SEQ ID NO: 514 |
| Bac685 | ILKSFQRGH | SEQ ID NO: 515 |
| Bac686 | GINFKHKRF | SEQ ID NO: 516 |
| Bac687 | KNMRLSNWQ | SEQ ID NO: 517 |
| Bac688 | GRNFKVPVR | SEQ ID NO: 518 |
| Bac689 | IKKKFKWNT | SEQ ID NO: 519 |
| Bac690 | DRRRVQKGL | SEQ ID NO: 520 |
| Bac691 | GQRGRPWAT | SEQ ID NO: 521 |
| Bac692 | HRKNKKHYM | SEQ ID NO: 522 |
| Bac693 | INPGTAGKK | SEQ ID NO: 523 |
| Bac694 | KFIVVKRVV | SEQ ID NO: 524 |
| Bac695 | RHKTWYPGK | SEQ ID NO: 525 |
| Bac696 | RPLRKKVKL | SEQ ID NO: 526 |
| Bac697 | SKRWILVRR | SEQ ID NO: 527 |
| Bac698 | TKRWQTKFM | SEQ ID NO: 528 |
| Bac699 | WPKTRTVAK | SEQ ID NO: 529 |
| Bac700 | FKPVRIVFS | SEQ ID NO: 530 |
| Bac701 | MRVITVRIL | SEQ ID NO: 531 |
| Bac702 | RHWQQKVRS | SEQ ID NO: 532 |
| Bac703 | NQAKKKVGA | SEQ ID NO: 533 |
| Bac704 | MKYQGKGMQ | SEQ ID NO: 534 |
| Bac705 | RGWPRNWPT | SEQ ID NO: 535 |
| Bac706 | LKISGYVKH | SEQ ID NO: 536 |
| Bac707 | RWKLFKVWW | SEQ ID NO: 537 |
| Bac708 | RRANFFFSV | SEQ ID NO: 538 |
| Bac709 | RYNWYGQLR | SEQ ID NO: 539 |
| Bac710 | RGFWMKRWW | SEQ ID NO: 540 |
| Bac711 | GGGRRSQWK | SEQ ID NO: 541 |
| Bac712 | WYGFKRKIV | SEQ ID NO: 542 |
| Bac713 | WSAIRKKGK | SEQ ID NO: 543 |
| Bac714 | HRQPWRGRI | SEQ ID NO: 544 |
| Bac715 | KNMVTKWNK | SEQ ID NO: 545 |
| Bac716 | HIRGRFWRW | SEQ ID NO: 546 |
| Bac717 | IIPPKWYRS | SEQ ID NO: 547 |
| Bac718 | IPRQWWTFK | SEQ ID NO: 548 |
| Bac719 | WSNIIRKFM | SEQ ID NO: 549 |
| Bac720 | RVTYRRNVT | SEQ ID NO: 550 |
| Bac721 | KWWRIRGWI | SEQ ID NO: 551 |
| Bac722 | VEKHIRQRV | SEQ ID NO: 552 |
| Bac723 | RISSVPRMP | SEQ ID NO: 553 |
| Bac724 | ANNPLRVRL | SEQ ID NO: 554 |
| Bac725 | QRWIRIKPW | SEQ ID NO: 555 |
| Bac726 | RGWPRQIYY | SEQ ID NO: 556 |
| Bac727 | VKLGLGYQR | SEQ ID NO: 557 |
| Bac728 | VHQLKKRHW | SEQ ID NO: 558 |
| Bac729 | RWWQVRMYI | SEQ ID NO: 559 |
| Bac730 | IVLRNIKFI | SEQ ID NO: 560 |
| Bac731 | VRTRHWSPS | SEQ ID NO: 561 |
| Bac732 | WFRWHNRLV | SEQ ID NO: 562 |
| Bac733 | NRLWRYGRL | SEQ ID NO: 563 |
| Bac734 | HPWNRYKWG | SEQ ID NO: 564 |
| Bac735 | QVRVRRRII | SEQ ID NO: 565 |
| Bac736 | VRRRPSMFM | SEQ ID NO: 566 |
| Bac737 | RKYQIGRHI | SEQ ID NO: 567 |
| Bac738 | AKRRSRMKR | SEQ ID NO: 568 |
| Bac739 | KLWWMIRRW | SEQ ID NO: 569 |
| Bac740 | HNLHDIKRK | SEQ ID NO: 570 |
| Bac741 | RGVGVTFKL | SEQ ID NO: 571 |
| Bac742 | VPKLHYVVR | SEQ ID NO: 572 |
| Bac743 | VHWRGAKVT | SEQ ID NO: 573 |
| Bac744 | NGRWRFWSG | SEQ ID NO: 574 |
| Bac745 | KPVHWKKLQ | SEQ ID NO: 575 |
| Bac746 | KRNRGGWKV | SEQ ID NO: 576 |
| Bac747 | TRNKTGYWW | SEQ ID NO: 577 |
| Bac748 | YQQRLRHIY | SEQ ID NO: 578 |
| Bac749 | GVVVWRRRV | SEQ ID NO: 579 |
| Bac750 | IMTRWKMHT | SEQ ID NO: 580 |
| Bac751 | TVHKRAAYP | SEQ ID NO: 581 |
| Bac752 | VKHKRGFYR | SEQ ID NO: 582 |
| Bac753 | RWTISFKRS | SEQ ID NO: 583 |
| Bac754 | GRMRHKRFT | SEQ ID NO: 584 |
| Bac755 | HSKSVLWIK | SEQ ID NO: 585 |
| Bac756 | KQMGRRISG | SEQ ID NO: 586 |
| Bac757 | RRMHSKIKG | SEQ ID NO: 587 |
| Bac758 | RINPKIYRS | SEQ ID NO: 588 |
| Bac759 | TIKRYIWIK | SEQ ID NO: 589 |
| Bac760 | WLIRPGAKL | SEQ ID NO: 590 |
| Bac761 | NTFRRAWRM | SEQ ID NO: 591 |
| Bac762 | KTRARWKNK | SEQ ID NO: 592 |
| Bac763 | HRPKIGFAG | SEQ ID NO: 593 |
| Bac764 | MSHKMRQKR | SEQ ID NO: 594 |
| Bac765 | FRGRWPLAR | SEQ ID NO: 595 |
| Bac766 | VIKQVGPHK | SEQ ID NO: 596 |
| Bac767 | AGFKRMWRV | SEQ ID NO: 597 |
| Bac768 | YRAVNKNPI | SEQ ID NO: 598 |
| Bac769 | AIWIPSKWR | SEQ ID NO: 599 |
| Bac770 | KNGAWWVLR | SEQ ID NO: 600 |
| Bac771 | MKMKRRMGV | SEQ ID NO: 601 |
| Bac772 | RTIKRWWWW | SEQ ID NO: 602 |
| Bac773 | AWYKKKRWW | SEQ ID NO: 603 |
| Bac774 | PRVLSRLIK | SEQ ID NO: 604 |
| Bac775 | WMFPKATRV | SEQ ID NO: 605 |
| Bac776 | MQVSKVKQI | SEQ ID NO: 606 |
| Bac777 | SWKRINQIN | SEQ ID NO: 607 |
| Bac778 | NRWRLINAQ | SEQ ID NO: 608 |
| Bac779 | RPSWHKWHH | SEQ ID NO: 609 |
| Bac780 | RVPINKWHR | SEQ ID NO: 610 |
| Bac781 | ILKIVRIKR | SEQ ID NO: 611 |
| Bac782 | DVWWKRLPR | SEQ ID NO: 612 |
| Bac783 | WIYWKVRGG | SEQ ID NO: 613 |
| Bac784 | HHRPPFRFQ | SEQ ID NO: 614 |
| Bac785 | MQRNFRRSI | SEQ ID NO: 615 |
| Bac786 | LMVRVLKNR | SEQ ID NO: 616 |
| Bac787 | HKWTQKYKA | SEQ ID NO: 617 |
| Bac788 | LWRRKWRTG | SEQ ID NO: 618 |
| Bac789 | KYVRRWKSG | SEQ ID NO: 619 |
| Bac790 | VAGWWSRRM | SEQ ID NO: 620 |
| Bac791 | IRQRWIWWY | SEQ ID NO: 621 |
| Bac792 | FRVRRWVRM | SEQ ID NO: 622 |
| Bac793 | FVMFLRQFK | SEQ ID NO: 623 |
| Bac794 | RNFVPRMIG | SEQ ID NO: 624 |
| Bac795 | RPTRQKNMN | SEQ ID NO: 625 |
| Bac796 | RRYIKWHIV | SEQ ID NO: 626 |
| Bac797 | WGKMNVRIH | SEQ ID NO: 627 |
| Bac798 | KITFRRYNP | SEQ ID NO: 628 |
| Bac799 | QAQQWWFKR | SEQ ID NO: 629 |
| Bac800 | IKSMFWRGP | SEQ ID NO: 630 |
| Bac801 | QPMRGIRMT | SEQ ID NO: 631 |
| Bac802 | HMILIRLFR | SEQ ID NO: 632 |
| Bac803 | SMNAPRVKR | SEQ ID NO: 633 |
| Bac804 | WKSKLSVNK | SEQ ID NO: 634 |
| Bac805 | ISNMRVSAK | SEQ ID NO: 635 |
| Bac806 | VRMGWWAHR | SEQ ID NO: 636 |
| Bac807 | RWPRVSWQA | SEQ ID NO: 637 |
| Bac808 | LSRTGVTRG | SEQ ID NO: 638 |
| Bac809 | RQLKPQNWS | SEQ ID NO: 639 |
| Bac810 | LHVRHKQHM | SEQ ID NO: 640 |
| Bac811 | QFRKIKAVS | SEQ ID NO: 641 |
| Bac812 | HWFNGNKKK | SEQ ID NO: 642 |
| Bac813 | RRVKIVRKI | SEQ ID NO: 643 |
| Bac814 | SMGKRTWMR | SEQ ID NO: 644 |
| Bac815 | KYSWVKKNI | SEQ ID NO: 645 |
| Bac816 | FPIRFKIWI | SEQ ID NO: 646 |
| Bac817 | KFFTYSRFR | SEQ ID NO: 647 |
| Bac818 | LGRKRMGHW | SEQ ID NO: 648 |
| Bac819 | NWRKLYRRK | SEQ ID NO: 649 |
| Bac820 | RGKIVVATL | SEQ ID NO: 650 |
| Bac821 | GWRRPGHNK | SEQ ID NO: 651 |
| Bac822 | IIAGTGLKR | SEQ ID NO: 652 |
| Bac823 | RRMKRRSIM | SEQ ID NO: 653 |
| Bac824 | KVFGRRYRK | SEQ ID NO: 654 |
| Bac825 | LQMFWYLRR | SEQ ID NO: 655 |
| Bac826 | RAYHKMRKK | SEQ ID NO: 656 |
| Bac827 | NPARWRPRV | SEQ ID NO: 657 |
| Bac828 | YVGKDRRKP | SEQ ID NO: 658 |
| Bac829 | LRWWTNTRW | SEQ ID NO: 659 |
| Bac830 | MYKQMMRVG | SEQ ID NO: 660 |
| Bac831 | PRRPWMRHR | SEQ ID NO: 661 |
| Bac832 | LNKFVKQRR | SEQ ID NO: 662 |
| Bac833 | ITINSRKWT | SEQ ID NO: 663 |
| Bac834 | RQFGMRKWT | SEQ ID NO: 664 |
| Bac835 | RIAVRHWHM | SEQ ID NO: 665 |
| Bac836 | HAVSQHRGK | SEQ ID NO: 666 |
| Bac837 | KSMYFRRSM | SEQ ID NO: 667 |
| Bac838 | VGTIRLGRG | SEQ ID NO: 668 |
| Bac839 | MRVGMRTKF | SEQ ID NO: 669 |
| Bac840 | LRRIWRMWS | SEQ ID NO: 670 |
| Bac841 | RFHRRAMFR | SEQ ID NO: 671 |
| Bac842 | MRPRHYIKN | SEQ ID NO: 672 |
| Bac843 | MRKRMKVYS | SEQ ID NO: 673 |
| Bac844 | MKHRHFGII | SEQ ID NO: 674 |
| Bac845 | VRGKVYAWW | SEQ ID NO: 675 |
| Bac846 | KIRLIRGKI | SEQ ID NO: 676 |
| Bac847 | KWINGPRKA | SEQ ID NO: 677 |
| Bac848 | GKNRITYSK | SEQ ID NO: 678 |
| Bac849 | GRAKRQHVI | SEQ ID NO: 679 |
| Bac850 | GIRFTRWLK | SEQ ID NO: 680 |
| Bac851 | AKMAWYKNP | SEQ ID NO: 681 |
| Bac852 | TRAKYIILR | SEQ ID NO: 682 |
| Bac853 | RSIVRWFGR | SEQ ID NO: 683 |
| Bac854 | RSEYKQIYR | SEQ ID NO: 684 |
| Bac855 | KRRMRWIIM | SEQ ID NO: 685 |
| Bac856 | YTKRWPVFR | SEQ ID NO: 686 |
| Bac857 | PYVSRWYKK | SEQ ID NO: 687 |
| Bac858 | RRFIKWGHV | SEQ ID NO: 688 |
| Bac859 | YVHKRKSMW | SEQ ID NO: 689 |
| Bac860 | QIGYIYRVR | SEQ ID NO: 690 |
| Bac861 | ARHWKMLWY | SEQ ID NO: 691 |
| Bac862 | VAVKKKRIS | SEQ ID NO: 692 |
| Bac863 | RKYGRSVPH | SEQ ID NO: 693 |
| Bac864 | TTGRGKIKR | SEQ ID NO: 694 |
| Bac865 | VWTIHTKMK | SEQ ID NO: 695 |
| Bac866 | RRWQEKGWR | SEQ ID NO: 696 |
| Bac867 | RQSGTFFKW | SEQ ID NO: 697 |
| Bac868 | WRITRGGII | SEQ ID NO: 698 |
| Bac869 | VAGRYKMQA | SEQ ID NO: 699 |
| Bac870 | KRGTHMRNV | SEQ ID NO: 700 |
| Bac871 | IGYHKIPMR | SEQ ID NO: 701 |
| Bac872 | YRFWGKKGF | SEQ ID NO: 702 |
| Bac873 | HYKRRGSRW | SEQ ID NO: 703 |
| Bac874 | GAKGGQIVR | SEQ ID NO: 704 |
| Bac875 | MIIIRSRRV | SEQ ID NO: 705 |
| Bac876 | NLGAWYKWK | SEQ ID NO: 706 |
| Bac877 | GPRVRKIWS | SEQ ID NO: 707 |
| Bac878 | RGISTYHKR | SEQ ID NO: 708 |
| Bac879 | IRAIKKVRK | SEQ ID NO: 709 |
| Bac880 | FGYRIKKRN | SEQ ID NO: 710 |
| Bac881 | PKNYMKIFP | SEQ ID NO: 711 |
| Bac882 | QAAQKKKRS | SEQ ID NO: 712 |
| Bac883 | MFGIPRHMR | SEQ ID NO: 713 |
| Bac884 | HAGVGIRHR | SEQ ID NO: 714 |
| Bac885 | LKWQAKSWI | SEQ ID NO: 715 |
| Bac886 | RYRRKRRWA | SEQ ID NO: 716 |
| Bac887 | LRGLVLKSG | SEQ ID NO: 717 |
| Bac888 | RRRKIHGPW | SEQ ID NO: 718 |
| Bac889 | RTARGGKFK | SEQ ID NO: 719 |
| Bac890 | MGNKIVWKN | SEQ ID NO: 720 |
| Bac891 | WRITIIKIT | SEQ ID NO: 721 |
| Bac892 | FRWRVSGRW | SEQ ID NO: 722 |
| Bac893 | TRVIPRMYY | SEQ ID NO: 723 |
| Bac894 | KKQARKYIK | SEQ ID NO: 724 |
| Bac895 | WIYIARSVK | SEQ ID NO: 725 |
| Bac896 | RVFTLARHV | SEQ ID NO: 726 |
| Bac897 | NYIVYRRVF | SEQ ID NO: 727 |
| Bac898 | NRFSKKHWK | SEQ ID NO: 728 |
| Bac899 | NSRGWQRRW | SEQ ID NO: 729 |
| Bac900 | TSSGNRKVT | SEQ ID NO: 730 |
| Bac901 | WIKAGWRSW | SEQ ID NO: 731 |
| Bac902 | TWIRLSRRV | SEQ ID NO: 732 |
| Bac903 | IRKWWVRNV | SEQ ID NO: 733 |
| Bac904 | YKKPPWQFK | SEQ ID NO: 734 |
| Bac905 | QQSILRKNN | SEQ ID NO: 735 |
| Bac906 | GHKFWKINR | SEQ ID NO: 736 |
| Bac907 | RVRWYRIFY | SEQ ID NO: 737 |
| Bac908 | WWRVVYKGV | SEQ ID NO: 738 |
| Bac909 | SKGIIAKWW | SEQ ID NO: 739 |
| Bac910 | QIKKQFVKQ | SEQ ID NO: 740 |
| Bac911 | KGTVNFYRQ | SEQ ID NO: 741 |
| Bac912 | LMMRHYWIR | SEQ ID NO: 742 |
| Bac913 | WWNRRKVDQ | SEQ ID NO: 743 |
| Bac914 | RYRIRKKRG | SEQ ID NO: 744 |
| Bac915 | KRQMHIHSR | SEQ ID NO: 745 |
| Bac916 | HKSMLRIWG | SEQ ID NO: 746 |
| Bac917 | WKILIIFGR | SEQ ID NO: 747 |
| Bac918 | KKVSLHREA | SEQ ID NO: 748 |
| Bac919 | PLSRVRNRW | SEQ ID NO: 749 |
| Bac920 | GWSHRVKGI | SEQ NO: 750 |
| Bac921 | RTIRRNWIE | SEQ ID NO: 751 |
| Bac922 | ITMPSKRRV | SEQ NO: 752 |
| Bac923 | KRKPPVHQK | SEQ NO: 753 |
| Bac924 | IWPKHRSKW | SEQ NO: 754 |
| Bac925 | QKAFWMWWR | SEQ ID NO: 755 |
| Bac926 | WRQHWGRHR | SEQ NO: 756 |
| Bac927 | VMGKARFWR | SEQ NO: 757 |
| Bac928 | WIVVKKQNR | SEQ ID NO: 758 |
| Bac929 | PRYQGWWRV | SEQ NO: 759 |
| Bac930 | KMRRPNIWL | SEQ ID NO: 760 |
| Bac931 | GGKRVFVRS | SEQ ID NO: 761 |
| Bac932 | GRGHPKYVT | SEQ ID NO: 762 |
| Bac933 | KAARNWKVW | SEQ ID NO: 763 |
| Bac934 | KKMSWGHTR | SEQ ID NO: 764 |
| Bac935 | KKTYWYNRA | SEQ ID NO: 765 |
| Bac936 | KNRRRRFWF | SEQ ID NO: 766 |
| Bac937 | RRRQKRGVW | SEQ ID NO: 767 |
| Bac938 | GRRNHQRAK | SEQ ID NO: 768 |
| Bac939 | QKMGHRWRI | SEQ ID NO: 769 |
| Bac940 | PKWKIQKGH | SEQ ID NO: 770 |
| Bac941 | AMRRFMRRP | SEQ ID NO: 771 |
| Bac942 | GKGMGRRAW | SEQ ID NO: 772 |
| Bac943 | HKRRKAKIM | SEQ ID NO: 773 |
| Bac944 | IMMNHRKWQ | SEQ ID NO: 774 |
| Bac945 | RWMRMKWVT | SEQ ID NO: 775 |
| Bac946 | RWGYRYNFI | SEQ ID NO: 776 |
| Bac947 | MWKKNNWNL | SEQ ID NO: 777 |
| Bac948 | WKRVGRKRL | SEQ ID NO: 778 |
| Bac949 | WLHGVKKMW | SEQ ID NO: 779 |
| Bac950 | EYWNWKRKV | SEQ ID NO: 780 |
| Bac951 | TKVTRGWPW | SEQ ID NO: 781 |
| Bac952 | QRWIQNTRW | SEQ ID NO: 782 |
| Bac953 | GKYIFKWWQ | SEQ ID NO: 783 |
| Bac954 | IVWKLRFQP | SEQ ID NO: 784 |
| Bac955 | WWAVNRNRK | SEQ ID NO: 785 |
| Bac956 | SIRMTKGKM | SEQ ID NO: 786 |
| Bac957 | NIKWGFFGK | SEQ ID NO: 787 |
| Bac958 | WMRQWRHWY | SEQ ID NO: 788 |
| Bac959 | PKVKRIWPK | SEQ ID NO: 789 |
| Bac960 | RRRKIAHKM | SEQ ID NO: 790 |
| Bac961 | RKYIKYVWN | SEQ ID NO: 791 |
| Bac962 | QPKRYVQTP | SEQ ID NO: 792 |
| Bac963 | LVGNPKWGR | SEQ ID NO: 793 |
| Bac964 | YKRAYSPIS | SEQ ID NO: 794 |
| Bac965 | KHKWGTFRF | SEQ ID NO: 795 |
| Bac966 | KIFLKYKGW | SEQ ID NO: 796 |
| Bac967 | RNVRHHWFR | SEQ ID NO: 797 |
| Bac968 | VIAVKRHLG | SEQ ID NO: 798 |
| Bac969 | RWKVGILRR | SEQ ID NO: 799 |
| Bac970 | WIQGIKMVR | SEQ ID NO: 800 |
| Bac971 | GWFKVTYKK | SEQ ID NO: 801 |
| Bac972 | YVMTKWRYV | SEQ ID NO: 802 |
| Bac973 | PRIFWKHIN | SEQ ID NO: 803 |
| Bac974 | VSPRWIWLK | SEQ ID NO: 804 |
| Bac975 | WVAIPGRSR | SEQ ID NO: 805 |
| Bac976 | NGFRKWWSR | SEQ ID NO: 806 |
| Bac977 | QFYIMKRYY | SEQ ID NO: 807 |
| Bac978 | ARHGRKTKK | SEQ ID NO: 808 |
| Bac979 | KRGRAKHIK | SEQ ID NO: 809 |
| Bac980 | NLWKIKTPI | SEQ ID NO: 810 |
| Bac981 | VPKGWKFLS | SEQ ID NO: 811 |
| Bac982 | RFMKEWFAK | SEQ ID NO: 812 |
| Bac983 | MGARKWIWW | SEQ ID NO: 813 |
| Bac984 | GLNHRRRPI | SEQ ID NO: 814 |
| Bac985 | VLMRKRIWH | SEQ ID NO: 815 |
| Bac986 | PGPYKKRQF | SEQ ID NO: 816 |
| Bac987 | WKYLYKPNP | SEQ ID NO: 817 |
| Bac988 | RKMRWWIRR | SEQ ID NO: 818 |
| Bac989 | AWRNSFRRG | SEQ ID NO: 819 |
| Bac990 | WKVGIIRAG | SEQ ID NO: 820 |
| Bac991 | WGRKTHYWH | SEQ ID NO: 821 |
| Bac992 | RIQHRYFLM | SEQ ID NO: 822 |
| Bac993 | FSWKVRIGV | SEQ ID NO: 823 |
| Bac994 | MPTKYGRHR | SEQ ID NO: 824 |
| Bac995 | RSPWRHRQF | SEQ ID NO: 825 |
| Bac996 | KRIWHIWRW | SEQ ID NO: 826 |
| Bac997 | KVVWLHRWQ | SEQ ID NO: 827 |
| Bac998 | IYRRKMIFQ | SEQ ID NO: 828 |
| Bac999 | HRYQYWKLT | SEQ ID NO: 829 |
| Bac1000 | AYKYFSQKR | SEQ ID NO: 830 |
| Bac1001 | KLVHKVTMT | SEQ ID NO: 831 |
| Bac1002 | VRNRRFIWR | SEQ ID NO: 832 |
| Bac1003 | RVLLVARPN | SEQ ID NO: 833 |
| Bac1004 | YKKHLRWGL | SEQ ID NO: 834 |
| Bac1005 | RKVYSARPG | SEQ ID NO: 835 |
| Bac1006 | QVMRWVQKL | SEQ ID NO: 836 |
| Bac1007 | IKILVLRVI | SEQ ID NO: 837 |
| Bac1008 | WHKRYFRSP | SEQ ID NO: 838 |
| Bac1009 | KMGNAKWRH | SEQ ID NO: 839 |
| Bac1010 | FGHHRFRLA | SEQ ID NO: 840 |
| Bac1011 | GHPSKIVRR | SEQ ID NO: 841 |
| Bac1012 | WRVWVRVKR | SEQ ID NO: 842 |
| Bac1013 | RSMTLWRKH | SEQ ID NO: 843 |
| Bac1014 | IRLYITRWL | SEQ ID NO: 844 |
| Bac1015 | IVKMRRRHA | SEQ ID NO: 845 |
| Bac1016 | FWRRQRWKQ | SEQ ID NO: 846 |
| Bac1017 | QQMYSRQRK | SEQ ID NO: 847 |
| Bac1018 | KPMKTWAKG | SEQ ID NO: 848 |
| Bac1019 | KYFVTKWGT | SEQ ID NO: 849 |
| Bac1020 | IRRKGTKRR | SEQ ID NO: 850 |
| Bac1021 | GMRHFKWGI | SEQ ID NO: 851 |
| Bac1022 | KKQVAIVRT | SEQ ID NO: 852 |
| Bac1023 | NGFKYVRSM | SEQ ID NO: 853 |
| Bac1024 | LWVGRLVYK | SEQ ID NO: 854 |
| Bac1025 | RWVNKITWI | SEQ ID NO: 855 |
| Bac1026 | AARIFRRYS | SEQ ID NO: 856 |
| Bac1027 | SQRWPTRGR | SEQ ID NO: 857 |
| Bac1028 | IRAKRWRQI | SEQ ID NO: 858 |
| Bac1029 | VKKPGWRLY | SEQ ID NO: 859 |
| Bac1030 | KRKTKLNPA | SEQ ID NO: 860 |
| Bac1031 | HVKGWTKFR | SEQ ID NO: 861 |
| Bac1032 | RPPVFHKHN | SEQ ID NO: 862 |
| Bac1033 | NVMTMRLKK | SEQ ID NO: 863 |
| Bac1034 | QWIKIRFSR | SEQ ID NO: 864 |
| Bac1035 | WYRRWSNVR | SEQ ID NO: 865 |
| Bac1036 | VFARRIWGI | SEQ ID NO: 866 |
| Bac1037 | HFTRPKFWR | SEQ ID NO: 867 |
| Bac1038 | WVQVKMASK | SEQ ID NO: 868 |
| Bac1039 | SWRSVKKVN | SEQ ID NO: 869 |
| Bac1040 | WYYVRYRWW | SEQ ID NO: 870 |
| Bac1041 | MHDRKWAVR | SEQ ID NO: 871 |
| Bac1042 | GGKGGRYRG | SEQ ID NO: 872 |
| Bac1043 | YVTYKTWRS | SEQ ID NO: 873 |
| Bac1044 | QRRNNQRVV | SEQ ID NO: 874 |
| Bac1045 | WRWVFMIVR | SEQ ID NO: 875 |
| Bac1046 | FYSMTYIRK | SEQ ID NO: 876 |
| Bac1047 | HPKKMAVVR | SEQ ID NO: 877 |
| Bac1048 | YRLTRYKGA | SEQ ID NO: 878 |
| Bac1049 | VKARFRIQW | SEQ ID NO: 879 |
| Bac1050 | IRRAKLRGR | SEQ ID NO: 880 |
| Bac1051 | KGINIKWKP | SEQ ID NO: 881 |
| Bac1052 | LWKYLRHGV | SEQ ID NO: 882 |
| Bac1053 | THAMWKGKN | SEQ ID NO: 883 |
| Bac1054 | IKKIHYRNK | SEQ ID NO: 884 |
| Bac1055 | RIMGWVHIK | SEQ ID NO: 885 |
| Bac1056 | KIRHIWIVG | SEQ ID NO: 886 |
| Bac1057 | PRQLFWPRW | SEQ ID NO: 887 |
| Bac1058 | SHARWMKIH | SEQ ID NO: 888 |
| Bac1059 | KISKKIKVV | SEQ ID NO: 889 |
| Bac1060 | HGKVVGQRI | SEQ ID NO: 890 |
| Bac1061 | RQVIWRWIT | SEQ ID NO: 891 |
| Bac1062 | TKIRARRVL | SEQ ID NO: 892 |
| Bac1063 | YRRVRQRDY | SEQ ID NO: 893 |
| Bac1064 | YKRKMWYIW | SEQ ID NO: 894 |
| Bac1065 | WKYGGPRQR | SEQ ID NO: 895 |
| Bac1066 | ATWPHGKKV | SEQ ID NO: 896 |
| Bac1067 | RGGKHRKKA | SEQ ID NO: 897 |
| Bac1068 | WKRWIRVMQ | SEQ ID NO: 898 |
| Bac1069 | WIIKEVRKP | SEQ ID NO: 899 |
| Bac1070 | YRQIMRWVQ | SEQ ID NO: 900 |
| Bac1071 | FKRRGGWLR | SEQ ID NO: 901 |
| Bac1072 | IVWNNSRVR | SEQ ID NO: 902 |
| Bac1073 | RRGGYVMYV | SEQ ID NO: 903 |
| Bac1074 | TSVKMFWRA | SEQ ID NO: 904 |
| Bac1075 | KHIWWKLHM | SEQ ID NO: 905 |
| Bac1076 | RLWGIIRKT | SEQ ID NO: 906 |
| Bac1077 | MVRPNRIRR | SEQ ID NO: 907 |
| Bac1078 | WPRAKNPSA | SEQ ID NO: 908 |
| Bac1079 | RRWLRAIIY | SEQ ID NO: 909 |
| Bac1080 | IGNRRNTGI | SEQ ID NO: 910 |
| Bac1081 | YIRSLIGKP | SEQ ID NO: 911 |
| Bac1082 | TGGWNIRMR | SEQ ID NO: 912 |
| Bac1083 | VVAVNKERK | SEQ ID NO: 913 |
| Bac1084 | NAHNKRYYR | SEQ ID NO: 914 |
| Bac1085 | WWRIAFKLT | SEQ ID NO: 915 |
| Bac1086 | NGQRKYIYI | SEQ ID NO: 916 |
| Bac1087 | RGWGWRRLY | SEQ ID NO: 917 |
| Bac1088 | RIAFPMKGG | SEQ ID NO: 918 |
| Bac1089 | RAKNVLGTY | SEQ ID NO: 919 |
| Bac1090 | NRRIKGQWV | SEQ ID NO: 920 |
| Bac1091 | RTSWMNRIW | SEQ ID NO: 921 |
| Bac1092 | TWIKQLINK | SEQ ID NO: 922 |
| Bac1093 | QKRKPRWPW | SEQ ID NO: 923 |
| Bac1094 | SKLLVRMWK | SEQ ID NO: 924 |
| Bac1095 | YKQGWWKWL | SEQ ID NO: 925 |
| Bac1096 | TWAPRHKSQ | SEQ ID NO: 926 |
| Bac1097 | KWVRTGYQW | SEQ ID NO: 927 |
| Bac1098 | QARRKQVWI | SEQ ID NO: 928 |
| Bac1099 | WKIGRIKMR | SEQ ID NO: 929 |
| Bac1100 | FIIRGRWAN | SEQ ID NO: 930 |
| Bac1101 | MLRKMGAPQ | SEQ ID NO: 931 |
| Bac1102 | KRRPVKSYK | SEQ ID NO: 932 |
| Bac1103 | IYWVNFRLR | SEQ ID NO: 933 |
| Bac1104 | MRIRKWQLS | SEQ ID NO: 934 |
| Bac1105 | WGRKQKQWS | SEQ ID NO: 935 |
| Bac1106 | RNWWTGHWR | SEQ ID NO: 936 |
| Bac1107 | VGRQQRYMK | SEQ ID NO: 937 |
| Bac1108 | GRKRNVEGR | SEQ ID NO: 938 |
| Bac1109 | ALGRIRGKR | SEQ ID NO: 939 |
| Bac1110 | MNKWINKLM | SEQ ID NO: 940 |
| Bac1111 | PKRWWGIRN | SEQ ID NO: 941 |
| Bac1112 | RHYRYTGII | SEQ ID NO: 942 |
| Bac1113 | PVRRWGWTL | SEQ ID NO: 943 |
| Bac1114 | RNSGWGWR | SEQ ID NO: 944 |
| Bac1115 | RIKLLTIWK | SEQ ID NO: 945 |
| Bac1116 | KWVKNWRYR | SEQ ID NO: 946 |
| Bac1117 | YQKGVRVIT | SEQ ID NO: 947 |
| Bac1118 | VRGGAKGGS | SEQ ID NO: 948 |
| Bac1119 | AIWGRIRKR | SEQ ID NO: 949 |
| Bac1120 | RIWKTATFG | SEQ ID NO: 950 |
| Bac1121 | HISRGARHK | SEQ ID NO: 951 |
| Bac1122 | YVTRKMIHQ | SEQ ID NO: 952 |
| Bac1123 | LMIRVGWRW | SEQ ID NO: 953 |
| Bac1124 | WSQYMFKRW | SEQ ID NO: 954 |
| Bac1125 | RNIITYRFQ | SEQ ID NO: 955 |
| Bac1126 | KWRWAKGRQ | SEQ ID NO: 956 |
| Bac1127 | PGRTKIHRG | SEQ ID NO: 957 |
| Bac1128 | MNVYARLRH | SEQ ID NO: 958 |
| Bac1129 | WFNYKRHVL | SEQ ID NO: 959 |
| Bac1130 | RQMRSIRGR | SEQ ID NO: 960 |
| Bac1131 | RGFRKIYKR | SEQ ID NO: 961 |
| Bac1132 | YSGPIRQAR | SEQ ID NO: 962 |
| Bac1133 | IVATVWRKN | SEQ ID NO: 963 |
| Bac1134 | WGKRYPKYW | SEQ ID NO: 964 |
| Bac1135 | ITWPRGGGK | SEQ ID NO: 965 |
| Bac1136 | WKFKKIGMQ | SEQ ID NO: 966 |
| Bac1137 | RQGRIWWVR | SEQ ID NO: 967 |
| Bac1138 | YMKAFVSRW | SEQ ID NO: 968 |
| Bac1139 | MHAKVRIGL | SEQ ID NO: 969 |
| Bac1140 | YHVRSKWGW | SEQ ID NO: 970 |
| Bac1141 | ITAAKIKQK | SEQ ID NO: 971 |
| Bac1142 | LNYSRIFITR | SEQ ID NO: 972 |
| Bac1143 | WVQKRKKGR | SEQ ID NO: 973 |
| Bac1144 | WQAIRRIVG | SEQ ID NO: 974 |
| Bac1145 | RRLITWLVP | SEQ ID NO: 975 |
| Bac1146 | KVKRQNKKR | SEQ ID NO: 976 |
| Bac1147 | WSKTHIRRN | SEQ ID NO: 977 |
| Bac1148 | LQVKWWVKF | SEQ ID NO: 978 |
| Bac1149 | RKDNKKVVV | SEQ ID NO: 979 |
| Bac1150 | VRPWRGRIL | SEQ ID NO: 980 |
| Bac1151 | KWRAAQWVL | SEQ ID NO: 981 |
| Bac1152 | IKAGRGMVR | SEQ ID NO: 982 |
| Bac1153 | KIRLKVWRA | SEQ ID NO: 983 |
| Bac1154 | HWWLRPIVR | SEQ ID NO: 984 |
| Bac1155 | GMYNKRFKR | SEQ ID NO: 985 |
| Bac1156 | RLQIWTRGW | SEQ ID NO: 986 |
| Bac1157 | KTGLKSKVR | SEQ ID NO: 987 |
| Bac1158 | RKQQVWRIQ | SEQ ID NO: 988 |
| Bac1159 | LGKRGKHRW | SEQ ID NO: 989 |
| Bac1160 | GLRHGNYRW | SEQ ID NO: 990 |
| Bac1161 | RVRRMTRWM | SEQ ID NO: 991 |
| Bac1162 | KLARWTRGG | SEQ ID NO: 992 |
| Bac1163 | VVRKRYVRI | SEQ ID NO: 993 |
| Bac1164 | VHRYMPFGR | SEQ ID NO: 994 |
| Bac1165 | KITHTFRPR | SEQ ID NO: 995 |
| Bac1166 | ARRPAQWIQ | SEQ ID NO: 996 |
| Bac1167 | RVSSGKLTH | SEQ ID NO: 997 |
| Bac1168 | RWRTGPSIP | SEQ ID NO: 998 |
| Bac1169 | LRLRRYKKW | SEQ ID NO: 999 |
| Bac1170 | AKMTWIFRP | SEQ ID NO: 1000 |
| Bac1171 | ARWKRMWML | SEQ ID NO: 1001 |
| Bac1172 | GRRVAVHRR | SEQ ID NO: 1002 |
| Bac1173 | RRWWFPFYA | SEQ ID NO: 1003 |
| Bac1174 | KHHKGVWWA | SEQ ID NO: 1004 |
| Bad1175 | LLYWKRGIY | SEQ ID NO: 1005 |
| Bac1176 | PVNYRKKRP | SEQ ID NO: 1006 |
| Bac1177 | LRGGTGIFR | SEQ ID NO: 1007 |
| Bac1178 | HHGRFRHWW | SEQ ID NO: 1008 |
| Bac1179 | TNRHQQKRW | SEQ ID NO: 1009 |
| Bac1180 | VQQLTKWSK | SEQ ID NO: 1010 |
| Bac1181 | RRRPGQKKW | SEQ ID NO: 1011 |
| Bac1182 | RPGSWRWRV | SEQ ID NO: 1012 |
| Bac1183 | PRLWNRRQR | SEQ ID NO: 1013 |
| Bac1184 | RWIVWSRGK | SEQ ID NO: 1014 |
| Bac1185 | SPHLGWKRS | SEQ ID NO: 1015 |
| Bac1186 | NRRWQWRMI | SEQ ID NO: 1016 |
| Bac1187 | WPRRGYMVA | SEQ ID NO: 1017 |
| Bac1188 | RWWLWQPWR | SEQ ID NO: 1018 |
| Bac1189 | WRGILYRSH | SEQ ID NO: 1019 |
| Bac1190 | IGWQRNRKY | SEQ ID NO: 1020 |
| Bac1191 | WIHKFRRKS | SEQ ID NO: 1021 |
| Bac1192 | GKALHKNKI | SEQ ID NO: 1022 |
| Bac1193 | KTKKKGVRK | SEQ ID NO: 1023 |
| Bac1194 | WFIKWRLWA | SEQ ID NO: 1024 |
| Bac1195 | GRKVYRVKV | SEQ ID NO: 1025 |
| Bac1196 | NAKWHTWYR | SEQ ID NO: 1026 |
| Bac1197 | TRGKIQISM | SEQ ID NO: 1027 |
| Bac1198 | MTVIKRNLF | SEQ ID NO: 1028 |
| Bac1199 | KRMALNQRH | SEQ ID NO: 1029 |
| Bac1200 | RWVFNGSKV | SEQ ID NO: 1030 |
| Bac1201 | RQGRLMGMA | SEQ ID NO: 1031 |
| Bac1202 | IIYRRTPVG | SEQ ID NO: 1032 |
| Bac1203 | LRRWKIMTT | SEQ ID NO: 1033 |
| Bac1204 | YKKGNNWTA | SEQ ID NO: 1034 |
| Bac1205 | TRWRWKRVS | SEQ ID NO: 1035 |
| Bac1206 | RRAAPTGRG | SEQ ID NO: 1036 |
| Bac1207 | YIKRWYGIW | SEQ ID NO: 1037 |
| Bac1208 | PWGHIKKRK | SEQ ID NO: 1038 |
| Bac1209 | TVGFPTQKR | SEQ ID NO: 1039 |
| Bac1210 | KWVKGGWQV | SEQ ID NO: 1040 |
| Bac1211 | AFRIRKNID | SEQ ID NO: 1041 |
| Bac1212 | VWRKKMVLV | SEQ ID NO: 1042 |
| Bac1213 | FGYIKRGGP | SEQ ID NO: 1043 |
| Bac1214 | KLRMVKWQG | SEQ ID NO: 1044 |
| Bac1215 | KKWAAWQPR | SEQ ID NO: 1045 |
| Bac1216 | RKSLKQKHW | SEQ ID NO: 1046 |
| Bac1217 | HKRKQWQRG | SEQ ID NO: 1047 |
| Bac1218 | RAKIPKRIM | SEQ ID NO: 1048 |
| Bac1219 | VKQHHKNWR | SEQ ID NO: 1049 |
| Bac1220 | RRWIPQKRR | SEQ ID NO: 1050 |
| Bac1221 | AKRNFWKRW | SEQ ID NO: 1051 |
| Bac1222 | LKWMWNVKR | SEQ ID NO: 1052 |
| Bac1223 | KAGQWFGRM | SEQ ID NO: 1053 |
| Bac1224 | VKWANIIWK | SEQ ID NO: 1054 |
| Bac1225 | WWKKGLLAT | SEQ ID NO: 1055 |
| Bac1226 | STLSYRRKF | SEQ ID NO: 1056 |
| Bac1227 | RKSWWGVGR | SEQ ID NO: 1057 |
| Bac1228 | RRIPRIQWV | SEQ ID NO: 1058 |
| Bac1229 | WVARNRRWV | SEQ ID NO: 1059 |
| Bac1230 | FGHPFLRKV | SEQ ID NO: 1060 |
| Bac1231 | LLPQPRIFR | SEQ ID NO: 1061 |
| Bac1232 | MVITRYRRW | SEQ ID NO: 1062 |
| Bac1233 | GVKPKMLKL | SEQ ID NO: 1063 |
| Bac1234 | LKTKHWLNW | SEQ ID NO: 1064 |
| Bac1235 | FGHKFLMFR | SEQ ID NO: 1065 |
| Bac1236 | RLVWRQWLR | SEQ ID NO: 1066 |
| Bac1237 | QIRILKTRY | SEQ ID NO: 1067 |
| Bac1238 | VRHTPKRVR | SEQ ID NO: 1068 |
| Bac1239 | AGRSKRHPI | SEQ ID NO: 1069 |
| Bac1240 | HSRILRKNK | SEQ ID NO: 1070 |
| Bac1241 | KIQKYVANW | SEQ ID NO: 1071 |
| Bac1242 | WAHGIKYFK | SEQ ID NO: 1072 |
| Bac1243 | RSGHGRSYQ | SEQ ID NO: 1073 |
| Bac1244 | IFMSWKSRW | SEQ ID NO: 1074 |
| Bac1245 | HYSRKMAWR | SEQ ID NO: 1075 |
| Bac1246 | KRWIVKWVK | SEQ ID NO: 1076 |
| Bac1247 | WRNWPYKGK | SEQ ID NO: 1077 |
| Bac1248 | RPYKPGWGK | SEQ ID NO: 1078 |
| Bac1249 | WWAGPRLRI | SEQ ID NO: 1079 |
| Bac1250 | TFQIKKPTW | SEQ ID NO: 1080 |
| Bac1251 | GFAFKRTLR | SEQ ID NO: 1081 |
| Bac1252 | QPNGRRYMA | SEQ ID NO: 1082 |
| Bac1253 | HRNHWMNKW | SEQ ID NO: 1083 |
| Bac1254 | NKRRVLIFI | SEQ ID NO: 1084 |
| Bac1255 | WWAMKWIRV | SEQ ID NO: 1085 |
| Bac1256 | GAKKFQWFQ | SEQ ID NO: 1086 |
| Bac1257 | VAKTPTRNW | SEQ ID NO: 1087 |
| Bac1258 | RMRHLRKVR | SEQ ID NO: 1088 |
| Bac1259 | YGQRNMWRV | SEQ ID NO: 1089 |
| Bac1260 | IMVMLKTVK | SEQ ID NO: 1090 |
| Bac1261 | RLRRGISTK | SEQ ID NO: 1091 |
| Bac1262 | HRVWVKWPY | SEQ ID NO: 1092 |
| Bac1263 | MYIRGGNRF | SEQ ID NO: 1093 |
| Bac1264 | RIRWTGYGI | SEQ ID NO: 1094 |
| Bac1265 | PRRRTVRSM | SEQ ID NO: 1095 |
| Bac1266 | RIYYMGFRT | SEQ ID NO: 1096 |
| Bac1267 | YPPKFHKIK | SEQ ID NO: 1097 |
| Bac1268 | YWRGWRHGL | SEQ ID NO: 1098 |
| Bac1269 | RIKFFFNMW | SEQ ID NO: 1099 |
| Bac1270 | IRVLIIMRR | SEQ ID NO: 1100 |
| Bac1271 | HRRMVRLGV | SEQ ID NO: 1101 |
| Bac1272 | FRRYIMNWW | SEQ ID NO: 1102 |
| Bac1273 | HRHNRAPGS | SEQ ID NO: 1103 |
| Bac1274 | GHKHFQKGQ | SEQ ID NO: 1104 |
| Bac1275 | WNTPKFMLR | SEQ ID NO: 1105 |
| Bac1276 | FMVWWKRPI | SEQ ID NO: 1106 |
| Bac1277 | VKIKKRHQN | SEQ ID NO: 1107 |
| Bac1278 | IVSTKRNNP | SEQ ID NO: 1108 |
| Bac1279 | RMKTWKNWM | SEQ ID NO: 1109 |
| Bac1280 | RRNWIRGIK | SEQ ID NO: 1110 |
| Bac1281 | VWAKWWYAR | SEQ ID NO: 1111 |
| Bac1282 | TSKKTKQVR | SEQ ID NO: 1112 |
| Bac1283 | LIRALIFKW | SEQ ID NO: 1113 |
| Bac1284 | SLWQTKVYK | SEQ ID NO: 1114 |
| Bac1285 | NRVHRRVYW | SEQ ID NO: 1115 |
| Bac1286 | HLRIRIYQL | SEQ ID NO: 1116 |
| Bac1287 | PKKVRVNAH | SEQ ID NO: 1117 |
| Bac1288 | NRWRYWFAA | SEQ ID NO: 1118 |
| Bac1289 | WGQKRSRAF | SEQ ID NO: 1119 |
| Bac1290 | RLWPTWRTW | SEQ ID NO: 1120 |
| Bac1291 | IVGKKKMRM | SEQ ID NO: 1121 |
| Bac1292 | PWKVVIVRW | SEQ ID NO: 1122 |
| Bac1293 | WNFIGVIKR | SEQ ID NO: 1123 |
| Bac1294 | RFVPRVTYT | SEQ ID NO: 1124 |
| Bac1295 | RWGRHKRPQ | SEQ ID NO: 1125 |
| Bac1296 | WRRVPRKWE | SEQ ID NO: 1126 |
| Bac1297 | HGVRGFKHW | SEQ ID NO: 1127 |
| Bac1298 | KNKRSQLVW | SEQ ID NO: 1128 |
| Bac1299 | RIIPKYWWR | SEQ ID NO: 1129 |
| Bac1300 | ILRLKFWT | SEQ ID NO: 1130 |
| Bac1301 | GVRPQIRRQ | SEQ ID NO: 1131 |
| Bac1302 | QIHNRIRSF | SEQ ID NO: 1132 |
| Bac1303 | RSAIRFGTG | SEQ ID NO: 1133 |
| Bac1304 | RGRHNFVSI | SEQ ID NO: 1134 |
| Bac1305 | AWRVMIYRF | SEQ ID NO: 1135 |
| Bac1306 | KKNNGLWKH | SEQ ID NO: 1136 |
| Bac1307 | RMQMRWKRK | SEQ ID NO: 1137 |
| Bac1308 | VKTGRKWNN | SEQ ID NO: 1138 |
| Bac1309 | PLFGSRRIK | SEQ ID NO: 1139 |
| Bac1310 | RKWYIVQKK | SEQ ID NO: 1140 |
| Bac1311 | PIGFSRGMK | SEQ ID NO: 1141 |
| Bac1312 | KAKVKTIWA | SEQ ID NO: 1142 |
| Bac1313 | HKWRPVNRM | SEQ ID NO: 1143 |
| Bac1314 | RHRVWVRRR | SEQ ID NO: 1144 |
| Bac1315 | RPRTWAIRR | SEQ ID NO: 1145 |
| Bac1316 | KFRYLKLAL | SEQ ID NO: 1146 |
| Bac1317 | ASKMNPLYR | SEQ ID NO: 1147 |
| Bac1318 | RLHVGRVKH | SEQ ID NO: 1148 |
| Bac1319 | VVALQRRLW | SEQ ID NO: 1149 |
| Bac1320 | LPRKWATGA | SEQ ID NO: 1150 |
| Bac1321 | VWIHKVKGF | SEQ ID NO: 1151 |
| Bac1322 | WVAWRWTRS | SEQ ID NO: 1152 |
| Bac1323 | HNRKTFNGG | SEQ ID NO: 1153 |
| Bac1324 | KGWLRANPR | SEQ ID NO: 1154 |
| Bac1325 | SLNRKFHGK | SEQ ID NO: 1155 |
| Bac1326 | IRGWWLKQG | SEQ ID NO: 1156 |
| Bac1327 | KRRIRPRVR | SEQ ID NO: 1157 |
| Bac1328 | FWPRYGTKF | SEQ ID NO: 1158 |
| Bac1329 | IRTLRVFRT | SEQ ID NO: 1159 |
| Bac1330 | WRNTWIRWN | SEQ ID NO: 1160 |
| Bac1331 | RRRKYHTRD | SEQ ID NO: 1161 |
| Bac1332 | ERPAFRMWR | SEQ ID NO: 1162 |
| Bac1333 | LIVIRSKGR | SEQ ID NO: 1163 |
| Bac1334 | WVTVYVVKRF | SEQ ID NO: 1164 |
| Bac1335 | FGSANYRQK | SEQ ID NO: 1165 |
| Bac1336 | QTKYWQVAK | SEQ ID NO: 1166 |
| Bac1337 | MVVMVVWRR | SEQ ID NO: 1167 |
| Bac1338 | KWQTGKRTS | SEQ ID NO: 1168 |
| Bac1339 | KWYRWRNHR | SEQ ID NO: 1169 |
| Bac1340 | KTHWWRGRI | SEQ ID NO: 1170 |
| Bac1341 | PSARRGWIY | SEQ ID NO: 1171 |
| Bac1342 | KRAFKIRHI | SEQ ID NO: 1172 |
| Bac1343 | GWYNPTRKI | SEQ ID NO: 1173 |
| Bac1344 | VRNISFVRL | SEQ ID NO: 1174 |
| Bac1345 | RRGTKKERS | SEQ ID NO: 1175 |
| Bac1346 | ARRWKFIKT | SEQ ID NO: 1176 |
| Bac1347 | GPGRAGVRN | SEQ ID NO: 1177 |
| Bac1348 | PQIWGIKRK | SEQ ID NO: 1178 |
| Bac1349 | KKVWHWFTG | SEQ ID NO: 1179 |
| Bac1350 | RMRRRGKKW | SEQ ID NO: 1180 |
| Bac1351 | RIERVNRKP | SEQ ID NO: 1181 |
| Bac1352 | RTQYRYAHG | SEQ ID NO: 1182 |
| Bac1353 | NKPRMPWYV | SEQ ID NO: 1183 |
| Bac1354 | PHAYRVRFK | SEQ ID NO: 1184 |
| Bac1355 | KNVRQAKIW | SEQ ID NO: 1185 |
| Bac1356 | IWRGRVRAI | SEQ ID NO: 1186 |
| Bac1357 | IWYLRIYKW | SEQ ID NO: 1187 |
| Bac1358 | WRVRAGRWP | SEQ ID NO: 1188 |
| Bac1359 | HMKRWHRWG | SEQ ID NO: 1189 |
| Bac1360 | KGIYVWRRP | SEQ ID NO: 1190 |
| Bac1361 | QKQIGTRTH | SEQ ID NO: 1191 |
| Bac1362 | GMKVWRNLA | SEQ ID NO: 1192 |
| Bac1363 | RIAMWKVFR | SEQ ID NO: 1193 |
| Bac1364 | RKAGAIGAG | SEQ ID NO: 1194 |
| Bac1365 | YSWRKKFQP | SEQ ID NO: 1195 |
| Bac1366 | IRRIGGVGN | SEQ ID NO: 1196 |
| Bac1367 | KRPWYNRKI | SEQ ID NO: 1197 |
| Bac1368 | AHYNGYKRY | SEQ ID NO: 1198 |
| Bac1369 | AWGRYTKVA | SEQ ID NO: 1199 |
| Bac1370 | KRADAHRPI | SEQ ID NO: 1200 |
| Bac1371 | ASRSKWNVI | SEQ ID NO: 1201 |
| Bac1372 | RRGIPIKSR | SEQ ID NO: 1202 |
| Bac1373 | MKLVNSRHL | SEQ ID NO: 1203 |
| Bac1374 | HRIHRVTVF | SEQ ID NO: 1204 |
| Bac1375 | IRYIMNHGK | SEQ ID NO: 1205 |
| Bac1376 | RKRRRQGFI | SEQ ID NO: 1206 |
| Bac1377 | IKIRFAAQW | SEQ ID NO: 1207 |
| Bac1378 | WGKMRMRVW | SEQ ID NO: 1208 |
| Bac1379 | YRKINGGWY | SEQ ID NO: 1209 |
| Bac1380 | WQAQKMWWR | SEQ ID NO: 1210 |
| Bac1381 | RVHPFQKRL | SEQ ID NO: 1211 |
| Bac1382 | TRIYGVWAR | SEQ ID NO: 1212 |
| Bac1383 | RHRRKVKLI | SEQ ID NO: 1213 |
| Bac1384 | KWRWVGIFM | SEQ ID NO: 1214 |
| Bac1385 | WPKRFWNVW | SEQ ID NO: 1215 |
| Bac1386 | NIIQKKMMG | SEQ ID NO: 1216 |
| Bac1387 | KKWNRRRVK | SEQ ID NO: 1217 |
| Bac1388 | WWKGGYIMK | SEQ ID NO: 1218 |
| Bac1389 | NK1MAKRNW | SEQ ID NO: 1219 |
| Bac1390 | KFSRGGMWW | SEQ ID NO: 1220 |
| Bac1391 | GSHGWRRPP | SEQ ID NO: 1221 |
| Bac1392 | LRNIKIPRS | SEQ ID NO: 1222 |
| Bac1393 | KYFKARNSW | SEQ ID NO: 1223 |
| Bac1394 | FWRMRQWKG | SEQ ID NO: 1224 |
| Bac1395 | KLWDKRWMP | SEQ ID NO: 1225 |
| Bac1396 | KSYWWTRWT | SEQ ID NO: 1226 |
| Bac1397 | QRIRVVPYA | SEQ ID NO: 1227 |
| Bac1398 | RQRVRGRKW | SEQ ID NO: 1228 |
| Bac1399 | VPTRGRTQN | SEQ ID NO: 1229 |
| Bac1400 | VRVRVSRWW | SEQ ID NO: 1230 |
| Bac1401 | KQGNNRRYN | SEQ ID NO: 1231 |
| Bac1402 | SYHRRARPK | SEQ ID NO: 1232 |
| Bac1403 | WPYKHKRRI | SEQ ID NO: 1233 |
| Bac1404 | WKKHLLKIM | SEQ ID NO: 1234 |
| Bac1405 | ISGKRGSRR | SEQ ID NO: 1235 |
| Bac1406 | KVGRKQWWI | SEQ ID NO: 1236 |
| Bac1407 | RRAFRLQGK | SEQ ID NO: 1237 |
| Bac1408 | GISKGIIRI | SEQ ID NO: 1238 |
| Bac1409 | WWKNKHHWK | SEQ ID NO: 1239 |
| Bac1410 | RVIHRWHRG | SEQ ID NO: 1240 |
| Bac1411 | WRYWLVRNG | SEQ ID NO: 1241 |
| Bac1412 | KRVWISIQI | SEQ ID NO: 1242 |
| Bac1413 | GRWKVMNRT | SEQ ID NO: 1243 |
| Bac1414 | IAWRVIVKW | SEQ ID NO: 1244 |
| Bac1415 | NVWFVKRQQ | SEQ ID NO: 1245 |
| Bac1416 | PRISRRRPW | SEQ ID NO: 1246 |
| Bac1417 | TYWRRRPAV | SEQ ID NO: 1247 |
| Bac1418 | IKRSHIITN | SEQ ID NO: 1248 |
| Bac1419 | LKWWVGRAG | SEQ ID NO: 1249 |
| Bac1420 | NSHGGRTRV | SEQ ID NO: 1250 |
| Bac1421 | MRYAIWRTI | SEQ ID NO: 1251 |
| Bac1422 | QIKRTWRRT | SEQ ID NO: 1252 |
| Bac1423 | KMYIWKRKI | SEQ ID NO: 1253 |
| Bac1424 | IFKMRTWTM | SEQ ID NO: 1254 |
| Bac1425 | RAVWVRRMG | SEQ ID NO: 1255 |
| Bac1426 | YWRQKINAW | SEQ ID NO: 1256 |
| Bac1427 | GKYKWWRIR | SEQ ID NO: 1257 |
| Bac1428 | MQRGFRKRK | SEQ ID NO: 1258 |
| Bac1429 | WRRHWLPQN | SEQ ID NO: 1259 |
| Bac1430 | WHIRRWKFI | SEQ ID NO: 1260 |
| Bac1431 | WGSWRMRKH | SEQ ID NO: 1261 |
| Bac1432 | KGWTNYNGR | SEQ ID NO: 1262 |
| Bac1433 | VFIGKRTKS | SEQ ID NO: 1263 |
| Bac1434 | GKPIGRKTY | SEQ ID NO: 1264 |
| Bac1435 | RIRKWWSNH | SEQ ID NO: 1265 |
| Bac1436 | RWIHTMWRG | SEQ ID NO: 1266 |
| Bac1437 | WARKISNSW | SEQ ID NO: 1267 |
| Bac1438 | WSRKRVWKF | SEQ ID NO: 1268 |
| Bac1439 | WAWKLWIIK | SEQ ID NO: 1269 |
| Bac1440 | GWAIGRGRI | SEQ ID NO: 1270 |
| Bac1441 | YKIWHPKKV | SEQ ID NO: 1271 |
| Bac1442 | SGKGMRIHT | SEQ ID NO: 1272 |
| Bac1443 | WKQHNVKLG | SEQ ID NO: 1273 |
| Bac1444 | HYRVAYWPR | SEQ ID NO: 1274 |
| Bac1445 | RMTMHISIK | SEQ ID NO: 1275 |
| Bac1446 | KRMYKQAGI | SEQ ID NO: 1276 |
| Bac1447 | WIKIHRGLS | SEQ ID NO: 1277 |
| Bac1448 | PWLAHRRPR | SEQ ID NO: 1278 |
| Bac1449 | QWKVIFRVW | SEQ ID NO: 1279 |
| Bac1450 | RYIRRIVHG | SEQ ID NO: 1280 |
| Bac1451 | MAYKFLIKN | SEQ ID NO: 1281 |
| Bac1452 | PGSRYTRNW | SEQ ID NO: 1282 |
| Bac1453 | QWKGTYIRP | SEQ ID NO: 1283 |
| Bac1454 | KVWRKFQYF | SEQ ID NO: 1284 |
| Bac1455 | IIFRKHRIL | SEQ ID NO: 1285 |
| Bac1456 | WSGIWRRWF | SEQ ID NO: 1286 |
| Bac1457 | RKWLKVTMR | SEQ ID NO: 1287 |
| Bac1458 | VRQQWIIRW | SEQ ID NO: 1288 |
| Bac1459 | YYQQGRLRY | SEQ ID NO: 1289 |
| Bac1460 | LGTTFKRGT | SEQ ID NO: 1290 |
| Bac1461 | AKRVTRGMS | SEQ ID NO: 1291 |
| Bac1462 | VGRKGGWWL | SEQ ID NO: 1292 |
| Bac1463 | YRMQVKWVR | SEQ ID NO: 1293 |
| Bac1464 | RPGRWGRVW | SEQ ID NO: 1294 |
| Bac1465 | IGGITVVKR | SEQ ID NO: 1295 |
| Bac1466 | KSVMVVKGR | SEQ ID NO: 1296 |
| Bac1467 | QYIRRAQMF | SEQ ID NO: 1297 |
| Bac1468 | IKHWKWWAV | SEQ ID NO: 1298 |
| Bac1469 | KHPFSKQSR | SEQ ID NO: 1299 |
| Bac1470 | AISGKKRFW | SEQ ID NO: 1300 |
| Bac1471 | RLRVRIWIL | SEQ ID NO: 1301 |
| Bac1472 | KPQTRNWWV | SEQ ID NO: 1302 |
| Bac1473 | WTKRWTQVN | SEQ ID NO: 1303 |
| Bac1474 | RWRSVQILV | SEQ ID NO: 1304 |
| Bac1475 | RHRWGWISK | SEQ ID NO: 1305 |
| Bac1476 | GPGLGIVRR | SEQ ID NO: 1306 |
| Bac1477 | GARHRILYW | SEQ ID NO: 1307 |
| Bac1478 | VWLKGKHNN | SEQ ID NO: 1308 |
| Bac1479 | YWVLRNMKN | SEQ ID NO: 1309 |
| Bac1480 | YIVRRTLGV | SEQ ID NO: 1310 |
| Bac1481 | RGKGIWMWN | SEQ ID NO: 1311 |
| Bac1482 | IKNWPQIKT | SEQ ID NO: 1312 |
| Bac1483 | NGAFKRTQK | SEQ ID NO: 1313 |
| Bac1484 | KMWRWHGRW | SEQ ID NO: 1314 |
| Bac1485 | KVWHINAIR | SEQ ID NO: 1315 |
| Bac1486 | WWLQPKQWK | SEQ ID NO: 1316 |
| Bac1487 | IKARGNRMS | SEQ ID NO: 1317 |
| Bac1488 | KGTRMTAGW | SEQ ID NO: 1318 |
| Bac1489 | GSHRIKVKW | SEQ ID NO: 1319 |
| Bac1490 | TWRIDRIRR | SEQ ID NO: 1320 |
| Bac1491 | AKVHYWVKI | SEQ ID NO: 1321 |
| Bac1492 | RTWIMITKV | SEQ ID NO: 1322 |
| Bac1493 | GGKMPKIRG | SEQ ID NO: 1323 |
| Bac1494 | KIGRKWVYG | SEQ ID NO: 1324 |
| Bac1495 | KVKAMVGKM | SEQ ID NO: 1325 |
| Bac1496 | SWRSVHSRK | SEQ ID NO: 1326 |
| Bac1497 | VSRNSVVKK | SEQ ID NO: 1327 |
| Bac1498 | ARWWGIRRR | SEQ ID NO: 1328 |
| Bac1499 | SHRFRKHKR | SEQ ID NO: 1329 |
| Bac2000 | ARWRIVVIRVRR | SEQ ID NO: 1330 |
| Bac2001 | CRWRIVVIRVRR | SEQ ID NO: 1331 |
| Bac2002 | DRWRIVVIRVRR | SEQ ID NO: 1332 |
| Bac2003 | ERWRIVVIRVRR | SEQ ID NO: 1333 |
| Bac2004 | FRWRIVVIRVRR | SEQ ID NO: 1334 |
| Bac2005 | GRWRIVVIRVRR | SEQ ID NO: 1335 |
| Bac2006 | HRWRIVVIRVRR | SEQ ID NO: 1336 |
| Bac2007 | IRWRIVVIRVRR | SEQ ID NO: 1337 |
| Bac2008 | KRWRIVVIRVRR | SEQ ID NO: 1338 |
| Bac2009 | LRWRIVVIRVRR | SEQ ID NO: 1339 |
| Bac2010 | MRWRIVVIRVRR | SEQ ID NO: 1340 |
| Bac2011 | NRWRIVVIRVRR | SEQ ID NO: 1341 |
| Bac2012 | PRWRIVVIRVRR | SEQ ID NO: 1342 |
| Bac2013 | QRWRIVVIRVRR | SEQ ID NO: 1343 |
| Bac2014 | SRWRIVVIRVRR | SEQ ID NO: 1344 |
| Bac2015 | TRWRIVVIRVRR | SEQ ID NO: 1345 |
| Bac2016 | VRWRIVVIRVRR | SEQ ID NO: 1346 |
| Bac2017 | WRWRIVVIRVRR | SEQ ID NO: 1347 |
| Bac2018 | YRWRIVVIRVRR | SEQ ID NO: 1348 |
| Bac2019 | RAWRIVVIRVRR | SEQ ID NO: 1349 |
| Bac2020 | RCWRIVVIRVRR | SEQ ID NO: 1350 |
| Bac2021 | RDWRIVVIRVRR | SEQ ID NO: 1351 |
| Bac2022 | REWRIVVIRVRR | SEQ ID NO: 1352 |
| Bac2023 | RFWRIVVIRVRR | SEQ ID NO: 1353 |
| Bac2024 | RGWRIVVIRVRR | SEQ ID NO: 1354 |
| Bac2025 | RHWRIVVIRVRR | SEQ ID NO: 1355 |
| Bac2026 | RIWRIVVIRVRR | SEQ ID NO: 1356 |
| Bac2027 | RKWRIVVIRVRR | SEQ ID NO: 1357 |
| Bac2028 | RLWRIVVIRVRR | SEQ ID NO: 1358 |
| Bac2029 | RMWRIVVIRVRR | SEQ ID NO: 1359 |
| Bac2030 | RNWRIVVIRVRR | SEQ ID NO: 1360 |
| Bac2031 | RPWRIVVIRVRR | SEQ ID NO: 1361 |
| Bac2032 | RQWRIVVIRVRR | SEQ ID NO: 1362 |
| Bac2033 | RSWRIVVIRVRR | SEQ ID NO: 1363 |
| Bac2034 | RTWRIVVIRVRR | SEQ ID NO: 1364 |
| Bac2035 | RVWRIVVIRVRR | SEQ ID NO: 1365 |
| Bac2036 | RWWRIVVIRVRR | SEQ ID NO: 1366 |
| Bac2037 | RYWRIVVIRVRR | SEQ ID NO: 1367 |
| Bac2038 | RRARIVVIRVRR | SEQ ID NO: 1368 |
| Bac2039 | RRCRIVVIRVRR | SEQ ID NO: 1369 |
| Bac2040 | RRDRIVVIRVRR | SEQ ID NO: 1370 |
| Bac2041 | RRERIVVIRVRR | SEQ ID NO: 1371 |
| Bac2042 | RRFRIVVIRVRR | SEQ ID NO: 1372 |
| Bac2043 | RRGRIVVIRVRR | SEQ ID NO: 1373 |
| Bac2044 | RRHRIVVIRVRR | SEQ ID NO: 1374 |
| Bac2045 | RRIRIVVIRVRR | SEQ ID NO: 1375 |
| Bac2046 | RRKRIVVIRVRR | SEQ ID NO: 1376 |
| Bac2047 | RRLRIVVIRVRR | SEQ ID NO: 1377 |
| Bac2048 | RRMRIVVIRVRR | SEQ ID NO: 1378 |
| Bac2049 | RRNRIVVIRVRR | SEQ ID NO: 1379 |
| Bac2050 | RRPRIVVIRVRR | SEQ ID NO: 1380 |
| Bac2051 | RRQRIVVIRVRR | SEQ ID NO: 1381 |
| Bac2052 | RRRRIVVIRVRR | SEQ ID NO: 1382 |
| Bac2053 | RRSRIVVIRVRR | SEQ ID NO: 1383 |
| Bac2054 | RRTRIVVIRVRR | SEQ ID NO: 1384 |
| Bac2055 | RRVRIVVIRVRR | SEQ ID NO: 1385 |
| Bac2056 | RRYRIVVIRVRR | SEQ ID NO: 1386 |
| Bac2057 | RRWAIVVIRVRR | SEQ ID NO: 1387 |
| Bac2058 | RRWCIVVIRVRR | SEQ ID NO: 1388 |
| Bac2059 | RRWDIVVIRVRR | SEQ ID NO: 1389 |
| Bac2060 | RRWEIVVIRVRR | SEQ ID NO: 1390 |
| Bac2061 | RRWFIVVIRVRR | SEQ ID NO: 1391 |
| Bac2062 | RRWGIVVIRVRR | SEQ ID NO: 1392 |
| Bac2063 | RRWHIVVIRVRR | SEQ ID NO: 1393 |
| Bac2064 | RRWIIVVIRVRR | SEQ ID NO: 1394 |
| Bac2065 | RRWKIVVIRVRR | SEQ ID NO: 1395 |
| Bac2066 | RRWLIVVIRVRR | SEQ ID NO: 1396 |
| Bac2067 | RRWMIVVIRVRR | SEQ ID NO: 1397 |
| Bac2068 | RRWNIVVIRVRR | SEQ ID NO: 1398 |
| Bac2069 | RRWPIVVIRVRR | SEQ ID NO: 1399 |
| Bac2070 | RRWQIVVIRVRR | SEQ ID NO: 1400 |
| Bac2071 | RRWSIVVIRVRR | SEQ ID NO: 1401 |
| Bac2072 | RRWTIVVIRVRR | SEQ ID NO: 1402 |
| Bac2073 | RRWVIVVIRVRR | SEQ ID NO: 1403 |
| Bac2074 | RRWWIVVIRVRR | SEQ ID NO: 1404 |
| Bac2075 | RRWYIVVIRVRR | SEQ ID NO: 1405 |
| Bac2076 | RRWRAVVIRVRR | SEQ ID NO: 1406 |
| Bac2077 | RRWRCVVIRVRR | SEQ ID NO: 1407 |
| Bac2078 | RRWRDVVIRVRR | SEQ ID NO: 1408 |
| Bac2079 | RRWREVVIRVRR | SEQ ID NO: 1409 |
| Bac2080 | RRWRFVVIRVRR | SEQ ID NO: 1410 |
| Bac2081 | RRWRGVVIRVRR | SEQ ID NO: 1411 |
| Bac2082 | RRWRHVVIRVRR | SEQ ID NO: 1412 |
| Bac2083 | RRWRKVVIRVRR | SEQ ID NO: 1413 |
| Bac2084 | RRWRLVVIRVRR | SEQ ID NO: 1414 |
| Bac2085 | RRWRMVVIRVRR | SEQ ID NO: 1415 |
| Bac2086 | RRWRNVVIRVRR | SEQ ID NO: 1416 |
| Bac2087 | RRWRPVVIRVRR | SEQ ID NO: 1417 |
| Bac2088 | RRWRQVVIRVRR | SEQ ID NO: 1418 |
| Bac2089 | RRWRRVVIRVRR | SEQ ID NO: 1419 |
| Bac2090 | RRWRSVVIRVRR | SEQ ID NO: 1420 |
| Bac2091 | RRWRTVVIRVRR | SEQ ID NO: 1421 |
| Bac2092 | RRWRVVVIRVRR | SEQ ID NO: 1422 |
| Bac2093 | RRWRWVVIRVRR | SEQ ID NO: 1423 |
| B ac2094 | RRWRYVVIRVRR | SEQ ID NO: 1424 |
| Bac2095 | RRWRIAVIRVRR | SEQ ID NO: 1425 |
| Bac2096 | RRWRICVIRVRR | SEQ ID NO: 1426 |
| B ac2097 | RRWRIDVIRVRR | SEQ ID NO: 1427 |
| Bac2098 | RRWRIEVIRVRR | SEQ ID NO: 1428 |
| B ac2099 | RRWRIFVIRVRR | SEQ ID NO: 1429 |
| Bac2100 | RRWRIGVIRVRR | SEQ ID NO: 1430 |
| Bac2101 | RRWRIHVIRVRR | SEQ ID NO: 1431 |
| Bac2102 | RRWRIIVIRVRR | SEQ ID NO: 1432 |
| Bac2103 | RRWRIKVIRVRR | SEQ ID NO: 1433 |
| Bac2104 | RRWRILVIRVRR | SEQ ID NO: 1434 |
| Bac2105 | RRWRIMVIRVRR | SEQ ID NO: 1435 |
| Bac2106 | RRWRINVIRVRR | SEQ ID NO: 1436 |
| Bac2107 | RRWRIPVIRVRR | SEQ ID NO: 1437 |
| Bac2108 | RRWRIQVIRVRR | SEQ ID NO: 1438 |
| Bac2109 | RRWRIRVIRVRR | SEQ ID NO: 1439 |
| Bac2110 | RRWRISVIRVRR | SEQ ID NO: 1440 |
| Bac2111 | RRWRITVIRVRR | SEQ ID NO: 1441 |
| Bac2112 | RRWRIWVIRVRR | SEQ ID NO: 1442 |
| Bac2113 | RRWRIYVIRVRR | SEQ ID NO: 1443 |
| Bac2114 | RRWRIVAIRVRR | SEQ ID NO: 1444 |
| Bac2115 | RRWRIVCIRVRR | SEQ ID NO: 1445 |
| Bac2116 | RRWRIVDIRVRR | SEQ ID NO: 1446 |
| Bac2117 | RRWRIVEIRVRR | SEQ ID NO: 1447 |
| Bac2118 | RRWRIVFIRVRR | SEQ ID NO: 1448 |
| Bac2119 | RRWRIVGIRVRR | SEQ ID NO: 1449 |
| Bac2120 | RRWRIVHIRVRR | SEQ ID NO: 1450 |
| Bac2121 | RRWRIVIIRVRR | SEQ ID NO: 1451 |
| Bac2122 | RRWRIVKIRVRR | SEQ ID NO: 1452 |
| Bac2123 | RRWRIVLIRVRR | SEQ ID NO: 1453 |
| Bac2124 | RRWRIVMIRVRR | SEQ ID NO: 1454 |
| Bac2125 | RRWRIVNIRVRR | SEQ ID NO: 1455 |
| Bac2126 | RRWRIVPIRVRR | SEQ ID NO: 1456 |
| Bac2127 | RRWRIVQIRVRR | SEQ ID NO: 1457 |
| Bac2128 | RRWRIVRIRVRR | SEQ ID NO: 1458 |
| Bac2129 | RRWRIVSIRVRR | SEQ ID NO: 1459 |
| Bac2130 | RRWRIVTIRVRR | SEQ ID NO: 1460 |
| Bac2131 | RRWRIVWIRVRR | SEQ ID NO: 1461 |
| Bac2132 | RRWRIVYIRVRR | SEQ ID NO: 1462 |
| Bac2133 | RRWRIVVARVRR | SEQ ID NO: 1463 |
| Bac2134 | RRWRIVVCRVRR | SEQ ID NO: 1464 |
| Bac2135 | RRWRIVVDRVRR | SEQ ID NO: 1465 |
| Bac2136 | RRWRIVVERVRR | SEQ ID NO: 1466 |
| Bac2137 | RRWRIVVFRVRR | SEQ ID NO: 1467 |
| Bac2138 | RRWRIVVGRVRR | SEQ ID NO: 1468 |
| Bac2139 | RRWRIVVHRVRR | SEQ ID NO: 1469 |
| Bac2140 | RRWRIVVKRVRR | SEQ ID NO: 1470 |
| Bac2141 | RRWRIVVLRVRR | SEQ ID NO: 1471 |
| Bac2142 | RRWRIVVMRVRR | SEQ ID NO: 1472 |
| Bac2143 | RRWRIVVNRVRR | SEQ ID NO: 1473 |
| Bac2144 | RRWRIVVPRVRR | SEQ ID NO: 1474 |
| Bac2145 | RRWRIVVQRVRR | SEQ ID NO: 1475 |
| Bac2146 | RRWRIVVRRVRR | SEQ ID NO: 1476 |
| Bac2147 | RRWRIVVSRVRR | SEQ ID NO: 1477 |
| Bac2148 | RRWRIVVTRVRR | SEQ ID NO: 1478 |
| Bac2149 | RRWRIVVVRVRR | SEQ ID NO: 1479 |
| Bac2150 | RRWRIVVWRVRR | SEQ ID NO: 1480 |
| Bac2151 | RRWRIVVYRVRR | SEQ ID NO: 1481 |
| Bac2152 | RRWRIVVIAVRR | SEQ ID NO: 1482 |
| Bac2153 | RRWRIVVICVRR | SEQ ID NO: 1483 |
| Bac2154 | RRWRIVVIDVRR | SEQ ID NO: 1484 |
| Bac2155 | RRWRIVVEVRR | SEQ ID NO: 1485 |
| Bac2156 | RRWRIVVIFVRR | SEQ ID NO: 1486 |
| Bac2157 | RRWRIVVIGVRR | SEQ ID NO: 1487 |
| Bac2158 | RRWRIVVIHVRR | SEQ ID NO: 1488 |
| Bac2159 | RRWRIVVIIVRR | SEQ ID NO: 1489 |
| Bac2160 | RRWRIVVIKVRR | SEQ ID NO: 1490 |
| Bac2161 | RRWRIVVILVRR | SEQ ID NO: 1491 |
| Bac2162 | RRWRIVVIMVRR | SEQ ID NO: 1492 |
| Bac2163 | RRWRIVVINVRR | SEQ ID NO: 1493 |
| Bac2164 | RRWRIVVIPVRR | SEQ ID NO: 1494 |
| Bac2165 | RRWRIVVIQVRR | SEQ ID NO: 1495 |
| Bac2166 | RRWRIVVISVRR | SEQ ID NO: 1496 |
| Bac2167 | RRWRIVVITVRR | SEQ ID NO: 1497 |
| Bac2168 | RRWRIVVIVVRR | SEQ ID NO: 1498 |
| Bac2169 | RRWRIVVIWVRR | SEQ ID NO: 1499 |
| Bac2170 | RRWRIVVIYVRR | SEQ ID NO: 1500 |
| Bac2171 | RRWRIVVIRARR | SEQ ID NO: 1501 |
| Bac2172 | RRWRIVVIRCRR | SEQ ID NO: 1502 |
| Bac2173 | RRWRIVVIRDRR | SEQ ID NO: 1503 |
| Bac2174 | RRWRIVVIRERR | SEQ ID NO: 1504 |
| Bac2175 | RRWRIVVIRFRR | SEQ ID NO: 1505 |
| Bac2176 | RRWRIVVIRGRR | SEQ ID NO: 1506 |
| Bac2177 | RRWRIVVIRHRR | SEQ ID NO: 1507 |
| Bac2178 | RRWRIVVIRIRR | SEQ ID NO: 1508 |
| Bac2179 | RRWRIVVIRKRR | SEQ ID NO: 1509 |
| Bac2180 | RRWRIVVIRLRR | SEQ ID NO: 1510 |
| Bac2181 | RRWRIVVIRMRR | SEQ ID NO: 1511 |
| Bac2182 | RRWRIVVIRNRR | SEQ ID NO: 1512 |
| Bac2183 | RRWRIVVIRPRR | SEQ ID NO: 1513 |
| Bac2184 | RRWRIVVIRQRR | SEQ ID NO: 1514 |
| Bac2185 | RRWRIVVIRRRR | SEQ ID NO: 1515 |
| Bac2186 | RRWRIVVIRSRR | SEQ ID NO: 1516 |
| Bac2187 | RRWRIVVIRTRR | SEQ ID NO: 1517 |
| Bac2188 | RRWRIVVIRWRR | SEQ ID NO: 1518 |
| Bac2189 | RRWRIVVIRYRR | SEQ ID NO: 1519 |
| Bac2190 | RRWRIVVIRVAR | SEQ ID NO: 1520 |
| Bac2191 | RRWRIVVIRVCR | SEQ ID NO: 1521 |
| Bac2192 | RRWRIVVIRVDR | SEQ ID NO: 1522 |
| Bac2193 | RRWRIVVIRVER | SEQ ID NO: 1523 |
| Bac2194 | RRWRIVVIRVFR | SEQ ID NO: 1524 |
| Bac2195 | RRWRIVVIRVGR | SEQ ID NO: 1525 |
| Bac2196 | RRWRIVVIRVHR | SEQ ID NO: 1526 |
| Bac2197 | RRWRIVVIRVIR | SEQ ID NO: 1527 |
| Bac2198 | RRWRIVVIRVKR | SEQ ID NO: 1528 |
| Bac2199 | RRWRIVVIRVLR | SEQ ID NO: 1529 |
| B ac2200 | RRWRIVVIRVMR | SEQ ID NO: 1530 |
| Bac2201 | RRWRIVVIRVNR | SEQ ID NO: 1531 |
| Bac2202 | RRWRIVVIRVPR | SEQ ID NO: 1532 |
| Bac2203 | RRWRIVVIRVQR | SEQ ID NO: 1533 |
| Bac2204 | RRWRIVVIRVSR | SEQ ID NO: 1534 |
| Bac2205 | RRWRIVVIRVTR | SEQ ID NO: 1535 |
| Bac2206 | RRWRIVVIRVVR | SEQ ID NO: 1536 |
| Bac2207 | RRWRIVVIRVWR | SEQ ID NO: 1537 |
| Bac2208 | RRWRIVVIRVYR | SEQ ID NO: 1538 |
| Bac2209 | RRWRIVVIRVRA | SEQ ID NO: 1539 |
| Bac2210 | RRWRIVVIRVRC | SEQ ID NO: 1540 |
| Bac2211 | RRWRIVVIRVRD | SEQ ID NO: 1541 |
| Bac2212 | RRWRIVVIRVRE | SEQ ID NO: 1542 |
| Bac2213 | RRWRIVVIRVRF | SEQ ID NO: 1543 |
| Bac2214 | RRWRIVVIRVRG | SEQ ID NO: 1544 |
| Bac2215 | RRWRIVVIRVRH | SEQ ID NO: 1545 |
| Bac2216 | RRWRIVVIRVRI | SEQ ID NO: 1546 |
| Bac2217 | RRWRIVVIRVRK | SEQ ID NO: 1547 |
| Bac2218 | RRWRIVVIRVRL | SEQ ID NO: 1548 |
| Bac2219 | RRWRIVVIRVRM | SEQ ID NO: 1549 |
| Bac2220 | RRWRIVVIRVRN | SEQ ID NO: 1550 |
| Bac2221 | RRWRIVVIRVRP | SEQ ID NO: 1551 |
| Bac2222 | RRWRIVVIRVRQ | SEQ ID NO: 1552 |
| Bac2223 | RRWRIVVIRVRS | SEQ ID NO: 1553 |
| Bac2224 | RRWRIVVIRVRT | SEQ ID NO: 1554 |
| Bac2225 | RRWRIVVIRVRV | SEQ ID NO: 1555 |
| Bac2226 | RRWRIVVIRVRW | SEQ ID NO: 1556 |
| Bac2227 | RRWRIVVIRVRY | SEQ ID NO: 1557 |
| Bac2228 | AWWKIWVIRWWR | SEQ ID NO: 1558 |
| Bac2229 | CWWKIWVIRWWR | SEQ ID NO: 1559 |
| Bac2230 | DWWKIWVIRWWR | SEQ ID NO: 1560 |
| Bac2231 | EWWKIWVIRWWR | SEQ ID NO: 1561 |
| Bac2232 | FWWKIWVIRWWR | SEQ ID NO: 1562 |
| Bac2233 | GWWKIWVIRWWR | SEQ ID NO: 1563 |
| Bac2234 | HWWKIWVIRWWR | SEQ ID NO: 1564 |
| Bac2235 | IWWKIWVIRWWR | SEQ ID NO: 1565 |
| Bac2236 | KWWKIWVIRWWR | SEQ ID NO: 1566 |
| Bac2237 | LWWKIWVIRWWR | SEQ ID NO: 1567 |
| Bac2238 | MWWKIWVIRWWR | SEQ ID NO: 1568 |
| Bac2239 | NWWKIWVIRWWR | SEQ ID NO: 1569 |
| Bac2240 | PWWKIWVIRWWR | SEQ ID NO: 1570 |
| Bac2241 | QWWKIWVIRWWR | SEQ ID NO: 1571 |
| Bac2242 | SWWKIWVIRWWR | SEQ ID NO: 1572 |
| B ac2243 | TWWKIWVIRWWR | SEQ ID NO: 1573 |
| Bac2244 | VWWKIWVIRWWR | SEQ ID NO: 1574 |
| Bac2245 | WWWKIWVIRWWR | SEQ ID NO: 1575 |
| Bac2246 | YWWKIWVIRWWR | SEQ ID NO: 1576 |
| Bac2247 | RAWKIWVIRWWR | SEQ ID NO: 1577 |
| B ac2248 | RCWKIWVIRWWR | SEQ ID NO: 1578 |
| Bac2249 | RDWKIWVIRWWR | SEQ ID NO: 1579 |
| Bac2250 | REWKIWVIRWWR | SEQ ID NO: 1580 |
| Bac2251 | RFWKIWVIRWWR | SEQ ID NO: 1581 |
| Bac2252 | RGWKIWVIRWWR | SEQ ID NO: 1582 |
| Bac2253 | RHWKIWVIRWWR | SEQ ID NO: 1583 |
| Bac2254 | RIWKIWVIRWWR | SEQ ID NO: 1584 |
| Bac2255 | RKWKIWVIRWWR | SEQ ID NO: 1585 |
| Bac2256 | RLWKIWVIRWWR | SEQ ID NO: 1586 |
| Bac2257 | RMWKIWVIRWWR | SEQ ID NO: 1587 |
| Bac2258 | RNWKIWVIRWWR | SEQ ID NO: 1588 |
| Bac2259 | RPWKIWVIRWWR | SEQ ID NO: 1589 |
| B ac2260 | RQWKIWVIRWWR | SEQ ID NO: 1590 |
| Bac2261 | RRWKIWVIRWWR | SEQ ID NO: 1591 |
| Bac2262 | RSWKIWVIRWWR | SEQ ID NO: 1592 |
| Bac2263 | RTWKIWVIRWWR | SEQ ID NO: 1593 |
| Bac2264 | RVWKIWVIRWWR | SEQ ID NO: 1594 |
| Bac2265 | RYWKIWVIRWWR | SEQ ID NO: 1595 |
| B ac2266 | RWAKIWVIRWWR | SEQ ID NO: 1596 |
| Bac2267 | RWCKIWVIRWWR | SEQ ID NO: 1597 |
| Bac2268 | RWDKIWVIRWWR | SEQ ID NO: 1598 |
| Bac2269 | RWEKIWVIRWWR | SEQ ID NO: 1599 |
| Bac2270 | RWFKIWVIRWWR | SEQ ID NO: 1600 |
| Bac2271 | RWGKIWVIRWWR | SEQ ID NO: 1601 |
| Bac2272 | RWHKIWVIRWWR | SEQ ID NO: 1602 |
| Bac2273 | RWIKIWVIRWWR | SEQ ID NO: 1603 |
| Bac2274 | RWKKIWVIRWWR | SEQ ID NO: 1604 |
| Bac2275 | RWLKIWVIRWWR | SEQ ID NO: 1605 |
| Bac2276 | RWMKIWVIRWWR | SEQ ID NO: 1606 |
| Bac2277 | RWNKIWVIRWWR | SEQ ID NO: 1607 |
| Bac2278 | RWPKIWVIRWWR | SEQ ID NO: 1608 |
| Bac2279 | RWQKIWVIRWWR | SEQ ID NO: 1609 |
| Bac2280 | RWRKIWVIRWWR | SEQ ID NO: 1610 |
| Bac2281 | RWSKWVIRWWR | SEQ ID NO: 1611 |
| Bac2282 | RWTKIWVIRWWR | SEQ ID NO: 1612 |
| Bac2283 | RWVKIWVIRWWR | SEQ ID NO: 1613 |
| Bac2284 | RWYKIWVIRWWR | SEQ ID NO: 1614 |
| Bac2285 | RWWAIWVIRWWR | SEQ ID NO: 1615 |
| Bac2286 | RWWCIWVIRWWR | SEQ ID NO: 1616 |
| Bac2287 | RWWDIWVIRWWR | SEQ ID NO: 1617 |
| Bac2288 | RWWEIWVIRWWR | SEQ ID NO: 1618 |
| Bac2289 | RWWFIWVIRWWR | SEQ ID NO: 1619 |
| Bac2290 | RWWGIWVIRWWR | SEQ ID NO: 1620 |
| Bac2291 | RWWHIWVIRWWR | SEQ ID NO: 1621 |
| Bac2292 | RWWIIWVIRWWR | SEQ ID NO: 1622 |
| Bac2293 | RWWLIWVIRWWR | SEQ ID NO: 1623 |
| Bac2294 | RWWMIWVIRWWR | SEQ ID NO: 1624 |
| Bac2295 | RWWNIWVIRWWR | SEQ ID NO: 1625 |
| Bac2296 | RWWPIWVIRWWR | SEQ ID NO: 1626 |
| Bac2297 | RWWQIWVIRWWR | SEQ ID NO: 1627 |
| Bac2298 | RWWRIWVIRWWR | SEQ ID NO: 1628 |
| Bac2299 | RWWSIWVIRWWR | SEQ ID NO: 1629 |
| Bac2300 | RWWTIWVIRWWR | SEQ ID NO: 1630 |
| Bac2301 | RWWVIWVIRWWR | SEQ ID NO: 1631 |
| Bac2302 | RWWWIWVIRWWR | SEQ ID NO: 1632 |
| Bac2303 | RWWYIWVIRWWR | SEQ ID NO: 1633 |
| Bac2304 | RWWKAWVIRWWR | SEQ ID NO: 1634 |
| Bac2305 | RWWKCWVIRWWR | SEQ ID NO: 1635 |
| Bac2306 | RWWKDWVIRWWR | SEQ ID NO: 1636 |
| Bac2307 | RWWKEWVIRWWR | SEQ ID NO: 1637 |
| Bac2308 | RWWKFWVIRWWR | SEQ ID NO: 1638 |
| Bac2309 | RWWKGWVIRWWR | SEQ ID NO: 1639 |
| Bac2310 | RWWKHWVIRWWR | SEQ ID NO: 1640 |
| Bac2311 | RWWKKWVIRWWR | SEQ ID NO: 1641 |
| Bac2312 | RWWKLWVIRWWR | SEQ ID NO: 1642 |
| Bac2313 | RWWKMWVIRWWR | SEQ ID NO: 1643 |
| Bac2314 | RWWKNWVIRWWR | SEQ ID NO: 1644 |
| Bac2315 | RWWKPWVIRWWR | SEQ ID NO: 1645 |
| Bac2316 | RWWKQWVIRWWR | SEQ ID NO: 1646 |
| Bac2317 | RWWKRWVIRWWR | SEQ ID NO: 1647 |
| Bac2318 | RWWKSWVIRWWR | SEQ ID NO: 1648 |
| Bac2319 | RWWKTWVIRWWR | SEQ ID NO: 1649 |
| Bac2320 | RWWKVWVIRWWR | SEQ ID NO: 1650 |
| Bac2321 | RWWKWWVIRWWR | SEQ ID NO: 1651 |
| Bac2322 | RWWKYWVIRWWR | SEQ ID NO: 1652 |
| Bac2323 | RWWKIAVIRWWR | SEQ ID NO: 1653 |
| Bac2324 | RWWKICVIRWWR | SEQ ID NO: 1654 |
| Bac2325 | RWWKIDVIRWWR | SEQ ID NO: 1655 |
| Bac2326 | RWWKIEVIRWWR | SEQ ID NO: 1656 |
| Bac2327 | RWWKIFVIRWWR | SEQ ID NO: 1657 |
| Bac2328 | RWWKIGVIRWWR | SEQ ID NO: 1658 |
| Bac2329 | RWWKIHVIRWWR | SEQ ID NO: 1659 |
| Bac2330 | RWWKIIVIRWWR | SEQ ID NO: 1660 |
| Bac2331 | RWWKIKVIRWWR | SEQ ID NO: 1661 |
| Bac2332 | RWWKILVIRWWR | SEQ ID NO: 1662 |
| Bac2333 | RWWKIMVIRWWR | SEQ ID NO: 1663 |
| Bac2334 | RWWKINVIRWWR | SEQ ID NO: 1664 |
| Bac2335 | RWWKIPVIRWWR | SEQ ID NO: 1665 |
| Bac2336 | RWWKIQVIRWWR | SEQ ID NO: 1666 |
| Bac2337 | RWWKIRVIRWWR | SEQ ID NO: 1667 |
| Bac2338 | RWWKISVIRWWR | SEQ ID NO: 1668 |
| Bac2339 | RWWKITVIRWWR | SEQ ID NO: 1669 |
| Bac2340 | RWWKIVVIRWWR | SEQ ID NO: 1670 |
| Bac2341 | RWWKIYVIRWWR | SEQ ID NO: 1671 |
| Bac2342 | RWWKIWAIRWWR | SEQ ID NO: 1672 |
| Bac2343 | RWWKIWCIRWWR | SEQ ID NO: 1673 |
| Bac2344 | RWWKIWDIRWWR | SEQ ID NO: 1674 |
| Bac2345 | RWWKIWEIRWWR | SEQ ID NO: 1675 |
| Bac2346 | RWWKIWFIRWWR | SEQ ID NO: 1676 |
| Bac2347 | RWWKIWGIRWWR | SEQ ID NO: 1677 |
| Bac2348 | RWWKIWHIRWWR | SEQ ID NO: 1678 |
| Bac2349 | RWWKIWIIRWWR | SEQ ID NO: 1679 |
| Bac2350 | RWWKIWKIRWWR | SEQ ID NO: 1680 |
| Bac2351 | RWWKIWLIRWWR | SEQ ID NO: 1681 |
| Bac2352 | RWWKIWMIRWWR | SEQ ID NO: 1682 |
| Bac2353 | RWWKIWNIRWWR | SEQ ID NO: 1683 |
| Bac2354 | RWWKIWPIRWWR | SEQ ID NO: 1684 |
| Bac2355 | RWWKIWQIRWWR | SEQ ID NO: 1685 |
| Bac2356 | RWWKIWRIRWWR | SEQ ID NO: 1686 |
| Bac2357 | RWWKIWSIRWWR | SEQ ID NO: 1687 |
| Bac2358 | RWWKIWTIRWWR | SEQ ID NO: 1688 |
| Bac2359 | RWWKIWWIRWWR | SEQ ID NO: 1689 |
| Bac2360 | RWWKIWYIRWWR | SEQ ID NO: 1690 |
| Bac2361 | RWWKIWVARWWR | SEQ ID NO: 1691 |
| Bac2362 | RWWKIWVCRWWR | SEQ ID NO: 1692 |
| Bac2363 | RWWKIWVDRWWR | SEQ ID NO: 1693 |
| Bac2364 | RWWKIWVERWWR | SEQ ID NO: 1694 |
| Bac2365 | RWWKIWVFRWWR | SEQ ID NO: 1695 |
| Bac2366 | RWWKIWVGRWWR | SEQ ID NO: 1696 |
| Bac2367 | RWWKTWVHRWWR | SEQ ID NO: 1697 |
| Bac2368 | RWWKIWVKRWWR | SEQ ID NO: 1698 |
| Bac2369 | RWWKIWVLRWWR | SEQ ID NO: 1699 |
| Bac2370 | RWWKIWVMRWWR | SEQ ID NO: 1700 |
| Bac2371 | RWWKIWVNRWWR | SEQ ID NO: 1701 |
| Bac2372 | RWWKIWVPRWWR | SEQ ID NO: 1702 |
| Bac2373 | RWWKIVWQRWWR | SEQ ID NO: 1703 |
| Bac2374 | RWWKIWVRRWWR | SEQ ID NO: 1704 |
| Bac2375 | RWWKIWVSRWWR | SEQ ID NO: 1705 |
| Bac2376 | RWWKIWVTRWWR | SEQ ID NO: 1706 |
| Bac2377 | RWWKIWVVRWWR | SEQ ID NO: 1707 |
| Bac2378 | RWWKIWVWRWWR | SEQ ID NO: 1708 |
| Bac2379 | RWWKIWVYRWWR | SEQ ID NO: 1709 |
| Bac2380 | RWWKIWVIAWWR | SEQ ID NO: 1710 |
| Bac2381 | RWWKIWVICWWR | SEQ ID NO: 1711 |
| Bac2382 | RWWKIWVIDWWR | SEQ ID NO: 1712 |
| Bac2383 | RWWKIWVIEWWR | SEQ ID NO: 1713 |
| Bac2384 | RWWKIWVIFWWR | SEQ ID NO: 1714 |
| Bac2385 | RWWKIWVIGWWR | SEQ ID NO: 1715 |
| Bac2386 | RWWKIWVIHWWR | SEQ ID NO: 1716 |
| Bac2387 | RWWKIWVIIWWR | SEQ ID NO: 1717 |
| Bac2388 | RWWKIWVIKWWR | SEQ ID NO: 1718 |
| Bac2389 | RWWKIWVILWWR | SEQ ID NO: 1719 |
| Bac2390 | RWWKIWVIMWWR | SEQ ID NO: 1720 |
| Bac2391 | RWWKIWVINWWR | SEQ ID NO: 1721 |
| Bac2392 | RWWKIWVIPWWR | SEQ ID NO: 1722 |
| Bac2393 | RWWKIWVIQWWR | SEQ ID NO: 1723 |
| Bac2394 | RWWKIWVISWWR | SEQ ID NO: 1724 |
| Bac2395 | RWWKIWVITWWR | SEQ ID NO: 1725 |
| Bac2396 | RWWKIWVIVWWR | SEQ ID NO: 1726 |
| Bac2397 | RWWKIWVDVWWR | SEQ ID NO: 1727 |
| Bac2398 | RWWKIWVIYWWR | SEQ ID NO: 1728 |
| Bac2399 | RWWKIWVIRAWR | SEQ ID NO: 1729 |
| Bac2400 | RWWKIWVIRCWR | SEQ ID NO: 1730 |
| Bac2401 | RWWKIWVIRDWR | SEQ ID NO: 1731 |
| Bac2402 | RWWKIWVIREWR | SEQ ID NO: 1732 |
| Bac2403 | RWWKIWVIRFWR | SEQ ID NO: 1733 |
| Bac2404 | RWWKIWVIRGWR | SEQ ID NO: 1734 |
| Bac2405 | RWWKIWVIRHWR | SEQ ID NO: 1735 |
| Bac2406 | RWWKIWVIRIWR | SEQ ID NO: 1736 |
| Bac2407 | RWWKIWVIRKWR | SEQ ID NO: 1737 |
| Bac2408 | RWWKIWVIRLWR | SEQ ID NO: 1738 |
| Bac2409 | RWWKIWVIRMWR | SEQ ID NO: 1739 |
| Bac2410 | RWWKIWVIRNWR | SEQ ID NO: 1740 |
| Bac2411 | RWWKIWVIRPWR | SEQ ID NO: 1741 |
| Bac2412 | RWWKIWVIRQWR | SEQ ID NO: 1742 |
| Bac2413 | RWWKIWVIRRWR | SEQ ID NO: 1743 |
| Bac2414 | RWWKIWVIRSWR | SEQ ID NO: 1744 |
| Bac2415 | RWWKIWVIRTWR | SEQ ID NO: 1745 |
| Bac2416 | RWWKIWVIRVWR | SEQ ID NO: 1746 |
| Bac2417 | RWWKIWVIRYWR | SEQ ID NO: 1747 |
| Bac2418 | RWWKIWVIRWAR | SEQ ID NO: 1748 |
| Bac2419 | RWWKIWVIRWCR | SEQ ID NO: 1749 |
| Bac2420 | RWVVKIWVIRWDR | SEQ ID NO: 1750 |
| Bac2421 | RWWKIWVIRWER | SEQ ID NO: 1751 |
| Bac2422 | RWWKIWVIRWFR | SEQ ID NO: 1752 |
| Bac2423 | RWWKIWVIRWGR | SEQ ID NO: 1753 |
| Bac2424 | RWWKIWVIRWHR | SEQ ID NO: 1754 |
| Bac2425 | RWWKIWVIRWIR | SEQ ID NO: 1755 |
| Bac2426 | RWWKIWVIRWKR | SEQ ID NO: 1756 |
| Bac2427 | RWWKIWVIRWLR | SEQ ID NO: 1757 |
| Bac2428 | RWWKIWVIRWMR | SEQ ID NO: 1758 |
| Bac2429 | RWWKIWVIRWNR | SEQ ID NO: 1759 |
| Bac2430 | RWWKIWVIRWPR | SEQ ID NO: 1760 |
| Bac2431 | RWWKIWVIRWQR | SEQ ID NO: 1761 |
| Bac2432 | RWWKIWVIRWRR | SEQ ID NO: 1762 |
| Bac2433 | RWWKIWVIRWSR | SEQ ID NO: 1763 |
| Bac2434 | RWWKIWVIRWTR | SEQ ID NO: 1764 |
| Bac2435 | RWWKIWVIRWVR | SEQ ID NO: 1765 |
| Bac2436 | RWWKIWVIRWYR | SEQ ID NO: 1766 |
| Bac2437 | RWWKIWVIRWWA | SEQ ID NO: 1767 |
| Bac2438 | RWWKIWVIRWWC | SEQ ID NO: 1768 |
| Bac2439 | RWWKIWVIRWWD | SEQ ID NO: 1769 |
| Bac2440 | RWWKIWVIRWWE | SEQ ID NO: 1770 |
| Bac2441 | RWWKIWVIRWWF | SEQ ID NO: 1771 |
| Bac2442 | RWWKIWVIRWWG | SEQ ID NO: 1772 |
| Bac2443 | RWWKIWVIRWWH | SEQ ID NO: 1773 |
| Bac2444 | RWWKIWVIRWWI | SEQ ID NO: 1774 |
| Bac2445 | RWWKIWVIRWWK | SEQ ID NO: 1775 |
| Bac2446 | RWWKIWVIRWWL | SEQ ID NO: 1776 |
| Bac2447 | RWWKIVWIRWWM | SEQ ID NO: 1777 |
| Bac2448 | RWWKIWVIRWWN | SEQ ID NO: 1778 |
| Bac2449 | RWWKIWVIRWWP | SEQ ID NO: 1779 |
| Bac2450 | RWWKIWVIRWWQ | SEQ ID NO: 1780 |
| Bac2451 | RWWKIWVIRWWS | SEQ ID NO: 1781 |
| Bac2452 | RWWKIWVIRWWT | SEQ ID NO: 1782 |
| Bac2453 | RWWKIWVIRWWV | SEQ ID NO: 1783 |
| Bac2454 | RWWKIWVIRWWW | SEQ ID NO: 1784 |
| Bac2455 | RWWKIWVIRWWY | SEQ ID NO: 1785 |
| Bac2456 | ARAAVVLIVIRR | SEQ ID NO: 1786 |
| Bac2457 | CRAAVVLIVIRR | SEQ ID NO: 1787 |
| Bac2458 | DRAAVVLIVIRR | SEQ ID NO: 1788 |
| Bac2459 | ERAAVVLIVIRR | SEQ ID NO: 1789 |
| Bac2460 | FRAAVVLIVIRR | SEQ ID NO: 1790 |
| Bac2461 | GRAAVVLIVIRR | SEQ ID NO: 1791 |
| Bac2462 | HRAAVVLIVIRR | SEQ ID NO: 1792 |
| Bac2463 | IRAAVVLIVIRR | SEQ ID NO: 1793 |
| Bac2464 | KRAAVVLIVIRR | SEQ ID NO: 1794 |
| Bac2465 | LRAAWLIVIRR | SEQ ID NO: 1795 |
| Bac2466 | MRAAWLIVIRR | SEQ ID NO: 1796 |
| Bac2467 | NRAAVVLIVIRR | SEQ ID NO: 1797 |
| Bac2468 | PRAAVVLIVIRR | SEQ ID NO: 1798 |
| Bac2469 | QRAAVVLIVIRR | SEQ ID NO: 1799 |
| Bac2470 | SRAAVVLIVIRR | SEQ ID NO: 1800 |
| Bac2471 | TRAAVVLIVIRR | SEQ ID NO: 1801 |
| Bac2472 | VRAAVVLIVIRR | SEQ ID NO: 1802 |
| B ac2473 | WRAAVVLIVIRR | SEQ ID NO: 1803 |
| Bac2474 | YRAAVVLIVIRR | SEQ ID NO: 1804 |
| Bac2475 | RAAAVVLIVIRR | SEQ ID NO: 1805 |
| Bac2476 | RCAAVVLIVIRR | SEQ ID NO: 1806 |
| Bac2477 | RDAAVVLIVIRR | SEQ ID NO: 1807 |
| Bac2478 | REAAVVLIVIRR | SEQ ID NO: 1808 |
| Bac2479 | RFAAVVLIVIRR | SEQ ID NO: 1809 |
| Bac2480 | RGAAVVLIVIRR | SEQ ID NO: 1810 |
| Bac2481 | RHAAVVLIVIRR | SEQ ID NO: 1811 |
| Bac2482 | RIAAVVLIVIRR | SEQ ID NO: 1812 |
| Bac2483 | RKAAVVLIVIRR | SEQ ID NO: 1813 |
| Bac2484 | RLAAVVLIVIRR | SEQ ID NO: 1814 |
| Bac2485 | RMAAVVLIVIRR | SEQ ID NO: 1815 |
| Bac2486 | RNAAVVLIVIRR | SEQ ID NO: 1816 |
| Bac2487 | RPAAVVLIVIRR | SEQ ID NO: 1817 |
| Bac2488 | RQAAVVLIVIRR | SEQ ID NO: 1818 |
| Bac2489 | RSAAVVLIVIRR | SEQ ID NO: 1819 |
| Bac2490 | RTAAVVLIVIRR | SEQ ID NO: 1820 |
| Bac2491 | RVAAVVLIVIRR | SEQ ID NO: 1821 |
| Bac2492 | RWAAVVLIVIRR | SEQ ID NO: 1822 |
| Bac2493 | RYAAVVLIVIRR | SEQ ID NO: 1823 |
| Bac2494 | RRCAVVLIVIRR | SEQ ID NO: 1824 |
| Bac2495 | RRDAVVLIVIRR | SEQ ID NO: 1825 |
| Bac2496 | RREAVVLIVIRR | SEQ ID NO: 1826 |
| Bac2497 | RRFAVVLIVIRR | SEQ ID NO: 1827 |
| Bac2498 | RRGAVVLIVIRR | SEQ ID NO: 1828 |
| Bac2499 | RRHAVVLIVIRR | SEQ ID NO: 1829 |
| Bac2500 | RRIAVVLIVIRR | SEQ ID NO: 1830 |
| Bac2501 | RRKAVVLIVIRR | SEQ ID NO: 1831 |
| Bac2502 | RRLAVVLIVIRR | SEQ ID NO: 1832 |
| B ac2503 | RRMAVVLIVIRR | SEQ ID NO: 1833 |
| Bac2504 | RRNAVVLIVIRR | SEQ ID NO: 1834 |
| Bac2505 | RRPAVVLIVIRR | SEQ ID NO: 1835 |
| Bac2506 | RRQAVVLIVIRR | SEQ ID NO: 1836 |
| Bac2507 | RRRAVVLIVIRR | SEQ ID NO: 1837 |
| Bac2508 | RRSAVVLIVIRR | SEQ ID NO: 1838 |
| Bac2509 | RRTAVVLIVIRR | SEQ ID NO: 1839 |
| Bac2510 | RRVAVVLIVIRR | SEQ ID NO: 1840 |
| Bac2511 | RRWAVVLIVIRR | SEQ ID NO: 1841 |
| Bac2512 | RRYAVVLIVIRR | SEQ ID NO: 1842 |
| Bac2513 | RRACVVLIVIRR | SEQ ID NO: 1843 |
| Bac2514 | RRADVVLIVIRR | SEQ ID NO: 1844 |
| Bac2515 | RRAEVVLIVIRR | SEQ ID NO: 1845 |
| Bac2516 | RRAFVVLIVIRR | SEQ ID NO: 1846 |
| Bac2517 | RRAGVVLIVIRR | SEQ ID NO: 1847 |
| Bac2518 | RRAHVVLIVIRR | SEQ ID NO: 1848 |
| Bac2519 | RRAIVVLIVIRR | SEQ ID NO: 1849 |
| Bac2520 | RRAKVVLIVIRR | SEQ ID NO: 1850 |
| Bac2521 | RRALVVLIVIRR | SEQ ID NO: 1851 |
| Bac2522 | RRAMVVLIVIRR | SEQ ID NO: 1852 |
| Bac2523 | RRANVVLIVIRR | SEQ ID NO: 1853 |
| Bac2524 | RRAPVVLIVIRR | SEQ ID NO: 1854 |
| Bac2525 | RRAQVVLIVIRR | SEQ ID NO: 1855 |
| Bac2526 | RRARVVLIVIRR | SEQ ID NO: 1856 |
| Bac2527 | RRASVVLIVIRR | SEQ ID NO: 1857 |
| Bac2528 | RRATVVLIVIRR | SEQ ID NO: 1858 |
| Bac2529 | RRAVVVLIVIRR | SEQ ID NO: 1859 |
| Bac2530 | RRAWVVLIVIRR | SEQ ID NO: 1860 |
| Bac2531 | RRAYVVLIVIRR | SEQ ID NO: 1861 |
| Bac2532 | RRAAAVLIVIRR | SEQ ID NO: 1862 |
| Bac2533 | RRAACVLIVIRR | SEQ ID NO: 1863 |
| Bac2534 | RRAADVLIVIRR | SEQ ID NO: 1864 |
| Bac2535 | RRAAEVLIVIRR | SEQ ID NO: 1865 |
| Bac2536 | RRAAFVLIVIRR | SEQ ID NO: 1866 |
| Bac2537 | RRAAGVLIVIRR | SEQ ID NO: 1867 |
| Bac2538 | RRAAHVLIVIRR | SEQ ID NO: 1868 |
| Bac2539 | RRAAIVLIVIRR | SEQ ID NO: 1869 |
| Bac2540 | RRAAKVLIVIRR | SEQ ID NO: 1870 |
| Bac2541 | RRAALVLIVIRR | SEQ ID NO: 1871 |
| Bac2542 | RRAAMVLIVIRR | SEQ ID NO: 1872 |
| Bac2543 | RRAANVLIVIRR | SEQ ID NO: 1873 |
| Bac2544 | RRAAPVLIVIRR | SEQ ID NO: 1874 |
| Bac2545 | RRAAQVLIVIRR | SEQ ID NO: 1875 |
| B ac2546 | RRAARVLIVIRR | SEQ ID NO: 1876 |
| Bac2547 | RRAASVLIVIRR | SEQ ID NO: 1877 |
| Bac2548 | RRAATVLIVIRR | SEQ ID NO: 1878 |
| Bac2549 | RRAAWVLIVIRR | SEQ ID NO: 1879 |
| Bac2550 | RRAAYVLIVIRR | SEQ ID NO: 1880 |
| Bac2551 | RRAAVALIVIRR | SEQ ID NO: 1881 |
| Bac2552 | RRAAVCLIVIRR | SEQ ID NO: 1882 |
| Bac2553 | RRAAVDLIVIRR | SEQ ID NO: 1883 |
| Bac2554 | RRAAVELIVIRR | SEQ ID NO: 1884 |
| Bac2555 | RRAAVFLIVIRR | SEQ ID NO: 1885 |
| Bac2556 | RRAAVGLIVIRR | SEQ ID NO: 1886 |
| Bac2557 | RRAAVHLIVIRR | SEQ ID NO: 1887 |
| Bac2558 | RRAAVILIVIRR | SEQ ID NO: 1888 |
| Bac2559 | RRAAVKLIVIRR | SEQ ID NO: 1889 |
| Bac2560 | RRAAVLLIVIRR | SEQ ID NO: 1890 |
| Bac2561 | RRAAVMLIVIRR | SEQ ID NO: 1891 |
| Bac2562 | RRAAVNLIVIRR | SEQ ID NO: 1892 |
| Bac2563 | RRAAVPLIVIRR | SEQ ID NO: 1893 |
| Bac2564 | RRAAVQLIVIRR | SEQ ID NO: 1894 |
| Bac2565 | RRAAVRLIVIRR | SEQ ID NO: 1895 |
| Bac2566 | RRAAVSLIVIRR | SEQ ID NO: 1896 |
| Bac2567 | RRAAVTLIVIRR | SEQ ID NO: 1897 |
| Bac2568 | RRAAVWLIVIRR | SEQ ID NO: 1898 |
| Bac2569 | RRAAVYLIVIRR | SEQ ID NO: 1899 |
| Bac2570 | RRAAVVAIVIRR | SEQ ID NO: 1900 |
| Bac2571 | RRAAVVCIVIRR | SEQ ID NO: 1901 |
| Bac2572 | RRAAVVDIVIRR | SEQ ID NO: 1902 |
| Bac2573 | RRAAVVEIVIRR | SEQ ID NO: 1903 |
| Bac2574 | RRAAVVFIVIRR | SEQ ID NO: 1904 |
| Bac2575 | RRAAVVGIVIRR | SEQ ID NO: 1905 |
| Bac2576 | RRAAVVHIVIRR | SEQ ID NO: 1906 |
| Bac2577 | RRAAVVIIVIRR | SEQ ID NO: 1907 |
| Bac2578 | RRAAVVKIVIRR | SEQ ID NO: 1908 |
| Bac2579 | RRAAVVMIVIRR | SEQ ID NO: 1909 |
| Bac2580 | RRAAVVNIVIRR | SEQ ID NO: 1910 |
| Bac2581 | RRAAVVPIVIRR | SEQ ID NO: 1911 |
| Bac2582 | RRAAVVQIVIRR | SEQ ID NO: 1912 |
| Bac2583 | RRAAVVRIVIRR | SEQ ID NO: 1913 |
| Bac2584 | RRAAVVSIVIRR | SEQ ID NO: 1914 |
| Bac2585 | RRAAVVTIVIRR | SEQ ID NO: 1915 |
| Bac2586 | RRAAVVVIVIRR | SEQ ID NO: 1916 |
| Bac2587 | RRAAVVWIVIRR | SEQ ID NO: 1917 |
| Bac2588 | RRAAVVYIVIRR | SEQ ID NO: 1918 |
| Bac2589 | RRAAVVLAVIRR | SEQ ID NO: 1919 |
| Bac2590 | RRAAVVLCVIRR | SEQ ID NO: 1920 |
| Bac2591 | RRAAVVLDVIRR | SEQ ID NO: 1921 |
| Bac2592 | RRAAVVLEVIRR | SEQ ID NO: 1922 |
| Bac2593 | RRAAVVLFVIRR | SEQ ID NO: 1923 |
| Bac2594 | RRAAVVLGVIRR | SEQ ID NO: 1924 |
| Bac2595 | RRAAVVLHVIRR | SEQ ID NO: 1925 |
| Bac2596 | RRAAVVLKVIRR | SEQ ID NO: 1926 |
| Bac2597 | RRAAVVLLVIRR | SEQ ID NO: 1927 |
| Bac2598 | RRAAVVLMVIRR | SEQ ID NO: 1928 |
| Bac2599 | RRAAVVLNVIRR | SEQ ID NO: 1929 |
| Bac2600 | RRAAVVLPVIRR | SEQ ID NO: 1930 |
| Bac2601 | RRAAVVLQVIRR | SEQ ID NO: 1931 |
| B ac2602 | RRAAVVLRVIRR | SEQ ID NO: 1932 |
| Bac2603 | RRAAVVLSVIRR | SEQ ID NO: 1933 |
| B ac2604 | RRAAVVLTVIRR | SEQ ID NO: 1934 |
| Bac2605 | RRAAVVLVVIRR | SEQ ID NO: 1935 |
| B ac2606 | RRAAVVLWVIRR | SEQ ID NO: 1936 |
| Bac2607 | RRAAVVLYVIRR | SEQ ID NO: 1937 |
| Bac2608 | RRAAVVLIAIRR | SEQ ID NO: 1938 |
| Bac2609 | RRAAVVLICIRR | SEQ ID NO: 1939 |
| Bac2610 | RRAAVVLIDIRR | SEQ ID NO: 1940 |
| Bac2611 | RRAAVVLIEIRR | SEQ ID NO: 1941 |
| Bac2612 | RRAAVVLIFIRR | SEQ ID NO: 1942 |
| Bac2613 | RRAAVVLIGIRR | SEQ ID NO: 1943 |
| Bac2614 | RRAAVVLIHIRR | SEQ ID NO: 1944 |
| Bac2615 | RRAAVVLIIIRR | SEQ ID NO: 1945 |
| Bac2616 | RRAAVVLIKIRR | SEQ ID NO: 1946 |
| Bac2617 | RRAAVVLILIRR | SEQ ID NO: 1947 |
| Bac2618 | RRAAVVLIMIRR | SEQ ID NO: 1948 |
| Bac2619 | RRAAVVLINIRR | SEQ ID NO: 1949 |
| Bac2620 | RRAAVVLIPIRR | SEQ ID NO: 1950 |
| Bac2621 | RRAAVVLIQIRR | SEQ ID NO: 1951 |
| Bac2622 | RRAAVVLIRIRR | SEQ ID NO: 1952 |
| Bac2623 | RRAAVVLISIRR | SEQ ID NO: 1953 |
| Bac2624 | RRAAVVLITIRR | SEQ ID NO: 1954 |
| Bac2625 | RRAAVVLIWIRR | SEQ ID NO: 1955 |
| Bac2626 | RRAAVVLIYIRR | SEQ ID NO: 1956 |
| Bac2627 | RRAAVVLIVARR | SEQ ID NO: 1957 |
| Bac2628 | RRAAVVLIVCRR | SEQ ID NO: 1958 |
| Bac2629 | RRAAVVLIVDRR | SEQ ID NO: 1959 |
| Bac2630 | RRAAVVLIVERR | SEQ ID NO: 1960 |
| Bac2631 | RRAAVVLIVFRR | SEQ ID NO: 1961 |
| Bac2632 | RRAAVVLIVGRR | SEQ ID NO: 1962 |
| Bac2633 | RRAAVVLIVHRR | SEQ ID NO: 1963 |
| Bac2634 | RRAAVVLIVKRR | SEQ ID NO: 1964 |
| Bac2635 | RRAAVVLIVLRR | SEQ ID NO: 1965 |
| Bac2636 | RRAAVVLIVMRR | SEQ ID NO: 1966 |
| Bac2637 | RRAAVVLIVNRR | SEQ ID NO: 1967 |
| Bac2638 | RRAAVVLIVPRR | SEQ ID NO: 1968 |
| Bac2639 | RRAAVVLIVQRR | SEQ ID NO: 1969 |
| Bac2640 | RRAAVVLIVRRR | SEQ ID NO: 1970 |
| Bac2641 | RRAAVVLIVSRR | SEQ ID NO: 1971 |
| Bac2642 | RRAAVVLIVTRR | SEQ ID NO: 1972 |
| Bac2643 | RRAAVVLIVVRR | SEQ ID NO: 1973 |
| Bac2644 | RRAAVVLIVWRR | SEQ ID NO: 1974 |
| Bac2645 | RRAAVVLIVYRR | SEQ ID NO: 1975 |
| Bac2646 | RRAAVVLIVIAR | SEQ ID NO: 1976 |
| Bac2647 | RRAAVVLIVICR | SEQ ID NO: 1977 |
| Bac2648 | RRAAVVLIVIDR | SEQ ID NO: 1978 |
| Bac2649 | RRAAVVLIVIER | SEQ ID NO: 1979 |
| Bac2650 | RRAAVVLIVIFR | SEQ ID NO: 1980 |
| Bac2651 | RRAAVVLIVIGR | SEQ ID NO: 1981 |
| Bac2652 | RRAAVVLIVIHR | SEQ ID NO: 1982 |
| Bac2653 | RRAAVVLIVIIR | SEQ ID NO: 1983 |
| Bac2654 | RRAAVVLIVIKR | SEQ ID NO: 1984 |
| Bac2655 | RRAAVVLIVILR | SEQ ID NO: 1985 |
| Bac2656 | RRAAVVLIVIMR | SEQ ID NO: 1986 |
| Bac2657 | RRAAVVLIVINR | SEQ ID NO: 1987 |
| Bac2658 | RRAAVVLIVIPR | SEQ ID NO: 1988 |
| Bac2659 | RRAAVVLIVIQR | SEQ ID NO: 1989 |
| Bac2660 | RRAAVVLIVISR | SEQ ID NO: 1990 |
| Bac2661 | RRAAVVLIVITR | SEQ ID NO: 1991 |
| Bac2662 | RRAAVVLIVIVR | SEQ ID NO: 1992 |
| Bac2663 | RRAAVVLIVIWR | SEQ ID NO: 1993 |
| Bac2664 | RRAAVVLIVIYR | SEQ ID NO: 1994 |
| Bac2665 | RRAAVVLIVIRA | SEQ ID NO: 1995 |
| Bac2666 | RRAAVVLIVIRC | SEQ ID NO: 1996 |
| Bac2667 | RRAAVVLIVIRD | SEQ ID NO: 1997 |
| Bac2668 | RRAAVVLIVIRE | SEQ ID NO: 1998 |
| Bac2669 | RRAAVVLIVIRF | SEQ ID NO: 1999 |
| Bac2670 | RRAAVVLIVIRG | SEQ ID NO: 2000 |
| Bac2671 | RRAAVVLIVIRH | SEQ ID NO: 2001 |
| Bac2672 | RRAAVVLIVIRI | SEQ ID NO: 2002 |
| Bac2673 | RRAAVVLIVIRK | SEQ ID NO: 2003 |
| Bac2674 | RRAAVVLIVIRL | SEQ ID NO: 2004 |
| Bac2675 | RRAAVVLIVIRM | SEQ ID NO: 2005 |
| Bac2676 | RRAAVVLIVIRN | SEQ ID NO: 2006 |
| Bac2677 | RRAAVVLIVIRP | SEQ ID NO: 2007 |
| Bac2678 | RRAAVVLIVIRQ | SEQ ID NO: 2008 |
| Bac2679 | RRAAVVLIVIRS | SEQ ID NO: 2009 |
| Bac2680 | RRAAVVLIVIRT | SEQ ID NO: 2010 |
| Bac2681 | RRAAVVLIVIRV | SEQ ID NO: 2011 |
| Bac2682 | RRAAVVLIVIRW | SEQ ID NO: 2012 |
| Bac2683 | RRAAVVLIVIRY | SEQ ID NO: 2013 |
| Bac2684 | AIWVIWRR | SEQ ID NO: 2014 |
| Bac2685 | CIWVIWRR | SEQ ID NO: 2015 |
| Bac2686 | DIWVIWRR | SEQ ID NO: 2016 |
| Bac2687 | EIWVIWRR | SEQ ID NO: 2017 |
| Bac2688 | FIWVIWRR | SEQ ID NO: 2018 |
| Bac2689 | GIWVIWRR | SEQ ID NO: 2019 |
| Bac2690 | HIWVIWRR | SEQ ID NO: 2020 |
| Bac2691 | IIWVIWRR | SEQ ID NO: 2021 |
| Bac2692 | KIWVIWRR | SEQ ID NO: 2022 |
| Bac2693 | LIWVIWRR | SEQ ID NO: 2023 |
| Bac2694 | MIWVIWRR | SEQ ID NO: 2024 |
| Bac2695 | NIWVIWRR | SEQ ID NO: 2025 |
| Bac2696 | PIWVIWRR | SEQ ID NO: 2026 |
| B ac2697 | QIWVIWRR | SEQ ID NO: 2027 |
| Bac2698 | SIWVIWRR | SEQ ID NO: 2028 |
| B ac2699 | TIWVIWRR | SEQ ID NO: 2029 |
| Bac2700 | VIWVIWRR | SEQ ID NO: 2030 |
| Bac2701 | WIWVIWRR | SEQ ID NO: 2031 |
| Bac2702 | YIWVIWRR | SEQ ID NO: 2032 |
| Bac2703 | RAWVIWRR | SEQ ID NO: 2033 |
| Bac2704 | RCWVIWRR | SEQ ID NO: 2034 |
| Bac2705 | RDWVIWRR | SEQ ID NO: 2035 |
| Bac2706 | REWVIWRR | SEQ ID NO: 2036 |
| Bac2707 | RFWVIWRR | SEQ ID NO: 2037 |
| Bac2708 | RGWVIWRR | SEQ ID NO: 2038 |
| Bac2709 | RHWVIWRR | SEQ ID NO: 2039 |
| Bac2710 | RKWVIWRR | SEQ ID NO: 2040 |
| Bac2711 | RLWVIWRR | SEQ ID NO: 2041 |
| Bac2712 | RMWVIWRR | SEQ ID NO: 2042 |
| Bac2713 | RNWVIWRR | SEQ ID NO: 2043 |
| Bac2714 | RPWVIWRR | SEQ ID NO: 2044 |
| Bac2715 | RQWVIWRR | SEQ ID NO: 2045 |
| Bac2716 | RRWVIWRR | SEQ ID NO: 2046 |
| Bac2717 | RSWVIWRR | SEQ ID NO: 2047 |
| Bac2718 | RTWVIWRR | SEQ ID NO: 2048 |
| Bac2719 | RVWVIWRR | SEQ ID NO: 2049 |
| Bac2720 | RWWVIWRR | SEQ ID NO: 2050 |
| Bac2721 | RYWVIWRR | SEQ ID NO: 2051 |
| Bac2722 | RIAVIWRR | SEQ ID NO: 2052 |
| Bac2723 | RICVIWRR | SEQ ID NO: 2053 |
| Bac2724 | RIDVIWRR | SEQ ID NO: 2054 |
| Bac2725 | RIEVIWRR | SEQ ID NO: 2055 |
| Bac2726 | RIFVIWRR | SEQ ID NO: 2056 |
| Bac2727 | RIGVIWRR | SEQ ID NO: 2057 |
| Bac2728 | RIHVIWRR | SEQ ID NO: 2058 |
| Bac2729 | RIIVIWRR | SEQ ID NO: 2059 |
| Bac2730 | RIKVIWRR | SEQ ID NO: 2060 |
| Bac2731 | RILVIWRR | SEQ ID NO: 2061 |
| Bac2732 | RIMVIWRR | SEQ ID NO: 2062 |
| Bac2733 | RINVIWRR | SEQ ID NO: 2063 |
| Bac2734 | RIPVIWRR | SEQ ID NO: 2064 |
| Bac2735 | RIQVIWRR | SEQ ID NO: 2065 |
| Bac2736 | RIRVIWRR | SEQ ID NO: 2066 |
| Bac2737 | RISVIWRR | SEQ ID NO: 2067 |
| Bac2738 | RITVIWRR | SEQ ID NO: 2068 |
| Bac2739 | RIVVIWRR | SEQ ID NO: 2069 |
| Bac2740 | RIYVIWRR | SEQ ID NO: 2070 |
| Bac2741 | RIWAIWRR | SEQ ID NO: 2071 |
| Bac2742 | RIWCIWRR | SEQ ID NO: 2072 |
| Bac2743 | RIWDIWRR | SEQ ID NO: 2073 |
| Bac2744 | RIWEIWRR | SEQ ID NO: 2074 |
| Bac2745 | RIWFIWRR | SEQ ID NO: 2075 |
| Bac2746 | RIWGIWRR | SEQ ID NO: 2076 |
| Bac2747 | RIWHIWRR | SEQ ID NO: 2077 |
| Bac2748 | RIWIIWRR | SEQ ID NO: 2078 |
| Bac2749 | RIWKIWRR | SEQ ID NO: 2079 |
| Bac2750 | RIWLIWRR | SEQ ID NO: 2080 |
| Bac2751 | RIWMIWRR | SEQ ID NO: 2081 |
| Bac2752 | RIWNIWRR | SEQ ID NO: 2082 |
| Bac2753 | RIWPIWRR | SEQ ID NO: 2083 |
| Bac2754 | RIWQIWRR | SEQ ID NO: 2084 |
| Bac2755 | RIWRIWRR | SEQ ID NO: 2085 |
| Bac2756 | RIWSIWRR | SEQ ID NO: 2086 |
| Bac2757 | RIWTIWRR | SEQ ID NO: 2087 |
| Bac2758 | RIWWIWRR | SEQ ID NO: 2088 |
| Bac2759 | RIWYIWRR | SEQ ID NO: 2089 |
| Bac2760 | RIWVAWRR | SEQ ID NO: 2090 |
| Bac2761 | RIWVCWRR | SEQ ID NO: 2091 |
| Bac2762 | RIWVDWRR | SEQ ID NO: 2092 |
| Bac2763 | RIWVEWRR | SEQ ID NO: 2093 |
| Bac2764 | RIWVFWRR | SEQ ID NO: 2094 |
| Bac2765 | RIWVGWRR | SEQ ID NO: 2095 |
| Bac2766 | RIWVHWRR | SEQ ID NO: 2096 |
| Bac2767 | RIWVKWRR | SEQ ID NO: 2097 |
| Bac2768 | RIWVLWRR | SEQ ID NO: 2098 |
| Bac2769 | RIWVMWRR | SEQ ID NO: 2099 |
| Bac2770 | RIWVNWRR | SEQ ID NO: 2100 |
| Bac2771 | RIWVPWRR | SEQ ID NO: 2101 |
| Bac2772 | RIWVQWRR | SEQ ID NO: 2102 |
| Bac2773 | RIWVRWRR | SEQ ID NO: 2103 |
| Bac2774 | RIWVSWRR | SEQ ID NO: 2104 |
| Bac2775 | RIWVTWRR | SEQ ID NO: 2105 |
| Bac2776 | RIWVVWRR | SEQ ID NO: 2106 |
| Bac2777 | RIWVWWRR | SEQ ID NO: 2107 |
| Bac2778 | RIWVYWRR | SEQ ID NO: 2108 |
| Bac2779 | RIWVIARR | SEQ ID NO: 2109 |
| Bac2780 | RIWVICRR | SEQ ID NO: 2110 |
| Bac2781 | RIWVIDRR | SEQ ID NO: 2111 |
| Bac2782 | RIWVIERR | SEQ ID NO: 2112 |
| Bac2783 | RIWVIFRR | SEQ ID NO: 2113 |
| Bac2784 | RIWVIGRR | SEQ ID NO: 2114 |
| Bac2785 | RIWVIHRR | SEQ ID NO: 2115 |
| Bac2786 | RIWVIIRR | SEQ ID NO: 2116 |
| Bac2787 | RIWVIKRR | SEQ ID NO: 2117 |
| Bac2788 | RIWVILRR | SEQ ID NO: 2118 |
| Bac2789 | RIWVIMRR | SEQ ID NO: 2119 |
| Bac2790 | RIWVINRR | SEQ ID NO: 2120 |
| Bac2791 | RIWVIPRR | SEQ ID NO: 2121 |
| Bac2792 | RIWVIQRR | SEQ ID NO: 2122 |
| Bac2793 | RIWVIRRR | SEQ ID NO: 2123 |
| Bac2794 | RIWVISRR | SEQ ID NO: 2124 |
| Bac2795 | RIWVITRR | SEQ ID NO: 2125 |
| Bac2796 | RIWVIVRR | SEQ ID NO: 2126 |
| Bac2797 | RIWVIYRR | SEQ ID NO: 2127 |
| Bac2798 | RIWVIWAR | SEQ ID NO: 2128 |
| Bac2799 | RIWVIWCR | SEQ ID NO: 2129 |
| Bac2800 | RIWVIWDR | SEQ ID NO: 2130 |
| Bac2801 | RIWVIWER | SEQ ID NO: 2131 |
| Bac2802 | RIWVIWFR | SEQ ID NO: 2132 |
| Bac2803 | RIWVIWGR | SEQ ID NO: 2133 |
| Bac2804 | RIWVIWHR | SEQ ID NO: 2134 |
| Bac2805 | RIWVIWIR | SEQ ID NO: 2135 |
| Bac2806 | RIWVIWKR | SEQ ID NO: 2136 |
| Bac2807 | RIWVIWLR | SEQ ID NO: 2137 |
| Bac2808 | RIWVIWMR | SEQ ID NO: 2138 |
| Bac2809 | RIWVIWNR | SEQ ID NO: 2139 |
| Bac2810 | RIWVIWPR | SEQ ID NO: 2140 |
| Bac2811 | RIWVIWQR | SEQ ID NO: 2141 |
| Bac2812 | RIWVIWSR | SEQ ID NO: 2142 |
| Bac2813 | RIWVIWTR | SEQ ID NO: 2143 |
| Bac2814 | RIWVIWVR | SEQ ID NO: 2144 |
| Bac2815 | RIWVIWWR | SEQ ID NO: 2145 |
| Bac2816 | RIWVIWYR | SEQ ID NO: 2146 |
| Bac2817 | RIWVIWRA | SEQ ID NO: 2147 |
| Bac2818 | RIWVIWRC | SEQ ID NO: 2148 |
| Bac2819 | RIWVIWRD | SEQ ID NO: 2149 |
| Bac2820 | RIWVIWRE | SEQ ID NO: 2150 |
| Bac2821 | RIWVIWRF | SEQ ID NO: 2151 |
| Bac2822 | RIWVIWRG | SEQ ID NO: 2152 |
| Bac2823 | RIWVIWRH | SEQ ID NO: 2153 |
| Bac2824 | RIWVIWRI | SEQ ID NO: 2154 |
| Bac2825 | RIWVIWRK | SEQ ID NO: 2155 |
| Bac2826 | RIWVIWRL | SEQ ID NO: 2156 |
| Bac2827 | RIWVIWRM | SEQ ID NO: 2157 |
| Bac2828 | RIWVIWRN | SEQ ID NO: 2158 |
| Bac2829 | RIWVIWRP | SEQ ID NO: 2159 |
| Bac2830 | RIWVIWRQ | SEQ ID NO: 2160 |
| Bac2831 | RIWVIWRS | SEQ ID NO: 2161 |
| Bac2832 | RIWVIWRT | SEQ ID NO: 2162 |
| Bac2833 | RIWVIWRV | SEQ ID NO: 2163 |
| Bac2834 | RIWVIWRW | SEQ ID NO: 2164 |
| Bac2835 | RIWVIWRY | SEQ ID NO: 2165 |

Circular dichroism (CD) spectroscopy. CD spectra were recorded using a Jasco J-810 spectropolarimeter (Japan). Spectra were measured using a quartz cell with 1 mm path length at room temperature between 190 and 250 nm at a scan speed of 10nm/min and a total of 10 scans per sample. The data were collected and processed by the manufacturer's software. The concentration for each peptide was 70 µM. Spectra were recorded in 100 mM Tris buffer, pH 7.3, 10mM sodium dodecyl sulphate (SDS), or liposomes made by extrusion of 1:1 mixtures of 1-palmitoyl-2-oleoyl-sn-glycero-3-phosphocholine (POPC) and 1-1-palmitoyl-2-oleoyl-sn-glycero-3-phosphoglycerol (POPG). In all cases, the spectrum of the buffer only was subtracted from the peptide spectra.

Minimal inhibitory concentration (MIC) determination. The MICs of the peptides were measured using a modified broth microdilution method in Mueller Hinton (MH) medium. Wu *et al., J. Biol. Chem*. **274**: 29-35, 1999. Briefly, the peptides were dissolved and stored in glass vials. The assay was performed in sterile 96-well polypropylene microtitre plates were used. Serial dilutions of the peptides to be assayed were performed in 0.01 % acetic acid (Fisher) containing 0.2% bovine serum albumin (Boehringer Mannheim GmbH) at 10X the desired final concentration. Ten microlitres of the 10X peptides were added to each well of a 96-well polypropylene plate containing 90 µl of MH media per well. Bacteria were added to the plate from an overnight culture at 2 - 7 x 105 colony forming units/ml and incubated over night at 37°C. The MIC was taken as the concentration at which no growth was observed.

Luminescence-based MIC assay for the non-cleaved peptides on cellulose membranes. The peptide spots were punched out and transferred to a 96 well microtiter plate with a clear polystyrene bottom and opaque white polypropylene sides (PerkinElmer, Boston, USA). The spots were washed two times with 100% ethanol for 5 min and afterwards equilibrated five times with 100 mM Tris buffer pH 7.3 for 5 min. An overnight culture of *Pseudomonas aeruginosa* strain H1001 *fliC::luxCDABE* was diluted 1:50 in new MH medium and incubated at 37°C to an OD600 of 0.35. This bacterial culture was diluted 1:25 into 100mM Tris-HCl buffer, pH 7.3 containing 20 mM glucose. Fifty µl of this culture was added to all wells of the microtiter plate and incubated at 37°C. The luminescence of the strain produced by the ATP-dependent luciferase system was detected in a time dependent manner using a Tecan Spectra Fluor plus (Tecan, Austria). At the end of the experiment, the membranes were cleaned by washing the spots two times with 100% ethanol for five minutes. After removing the ethanol the membrane was air-dried.

Assay for the cleaved peptides from cellulose support. The peptides were cleaved from the dried membrane in an ammonia atmosphere overnight, resulting in free peptides with an amidated C-terminus. The free peptides contained two β-alanines at the C-terminus, in addition to being amidated due to the linker between the cellulose membrane and the peptide sequence. The peptide spots were punched out and transferred in a 96-well microtiter plate. Serial dilutions were carried out from the membrane spots. Four rows were filled with four controls including two with Bac2A and two with of an unrelated peptide. The other eight rows were used for serial dilution steps of the peptide variants. An overnight culture of *Pseudomonas aeruginosa* strain H1001 was diluted 1:500 using either LB media or 100mM Tris buffer pH 7.3, 20mM glucose and was added to the wells (100 µl/well) containing the peptide spots. In all other wells 50 µl were added. The microtiter plate was incubated for 30 min at 37°C to release the peptides from the membrane. Subsequently, a dilution series were performed and the plate was incubated at 37°C. The luminescence produced by the ATP dependent luciferase system were detected in a time dependent manner using a Tecan Spectra Fluor plus.

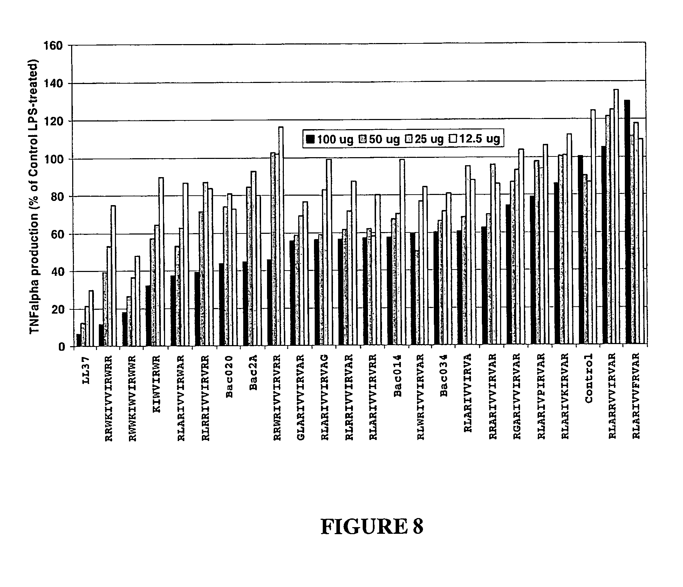

LPS Binding and Cytotoxicity assay. Peptides are known to bind to bacterial lipopolysaccharide as part of their mechanism of action and this binding can be revealed through the ability of the peptide to inhibit the ability of the peptide to suppress the production of TNFα by monocytic cells such as the human monocyte cell line THP-1 cells (Fig. 8). THP1 cells were cultured in RPMI 1640 medium (supplemented with 10% (v/v) FCS, 1% L-glutamine, and 1 nM sodium pyruvate) in E-toxa-clean (Sigma-Aldrich, Oakville, Ontario, Canada)-washed, endotoxin-free bottle. THP1 cells were counted and 250µl per well of 2-4 x 104 cells/ml were transferred into a 96 well tissue cultured treated polystyrene microtiterplate (Beckton Dickinson, Franklin Lakes, USA). In addition PMA was added (1.3 nM) and the cells were incubated for three days. After three days the medium were exchanged and *Pseudomonas aeruginosa* LPS and the peptides were added. The incubation time was four hours and the supernatant was frozen at -20°C. The cells were stained with Tryphan Blue for 2 minutes and washed with PBS two times. The viability of the cells was determined by counting the stained cells over the unstained. The supernatant was used to measure the TNFα production by an ELISA (eBioscience, SanDiego, USA) following the manufactures protocol (Fig. 8).

Strains. For the killing assay a mini-Tn5-lux mutant in *Pseudornonas aeruginosa* H103 was used. The strain is called H1001 and contains a fliC::luxCDABE transcriptional fusion resulting in constitutive expression of luciferase. The bacterial strains used for the antimicrobial activity assays included *Escherichia coli* UB 1005 (F-, nalA37, metB1), a wild-type *Salmonella enterica* ssp. Typhimurium (S. Typhimurium), wild-type *Pseudomonas aeruginos*a PAO1 strain H103, *Enterococcus faecalis* ATCC29212, *Staphylococcus aureus* ATCC25923, and a clinical isolate of *Staphylococcus epidermidis* obtained from Dr. D. Speert (Department of Medicine, University of British Columbia). Antifungal activity was tested using a lab isolate of *Candida albicans* obtained from Dr. B. Dill (Department of Microbiology and Immunology, University of British Columbia).

### EXAMPLE 2

### Scrambled Peptides

Bac2A is a short peptide of 12 amino acids with a maximal length of about 40Å. This peptide can kill both Gram positive and Gram negative bacteria. To learn more about the relationships between structure and activity, 49 scrambled variants of Bac2A were synthesized. All these peptides have the same length, same net charge and same amount of hydrophilic and hydrophobic amino acids.

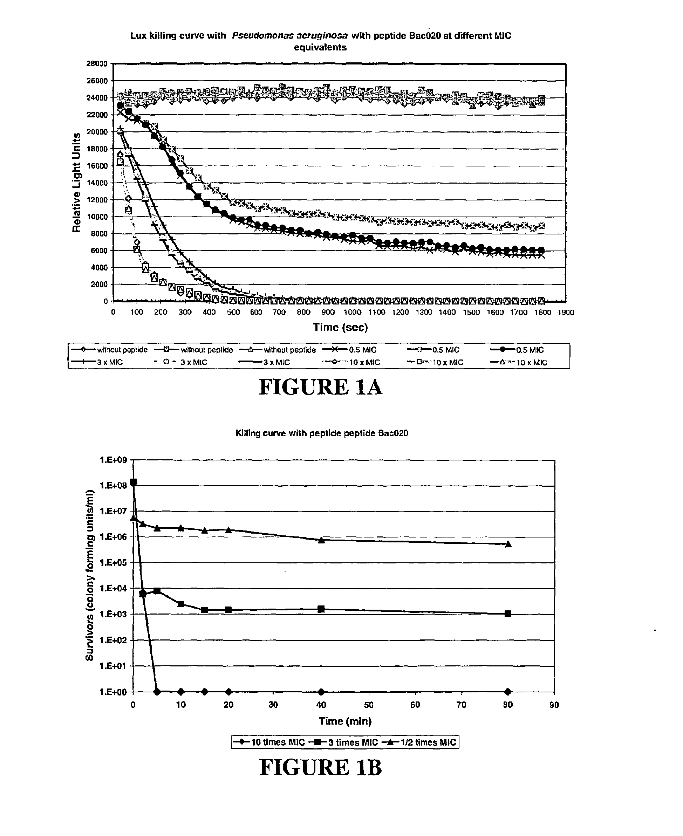

Peptide synthesis on cellulose is a very effective and inexpensive way to investigate many different peptide variants for one particular activity. However, one of the problems of this technique is the low peptide density synthesized on the membrane, about 280 nmol per cm2. An average peptide spot used for the experiments presented is about 0.3 cm2. Therefore, an assay had to be developed that was sensitive enough to show activity with this amount of peptides. *P. aeruginosa* H1001 has a reporter system (*e*.*g*., a luciferase gene cassette) incorporated into the bacterial chromosome in the *fliC* gene (involved in flagellar biosynthesis) that is constitutively expressed. It will thus produce light if ATP is present. When this strain is killed, e.g. by peptides, the amount of light produced will decrease due to a decrease in ATP levels in the killed cells. This action can thus be monitored by detecting luminescence in a microtiter plate luminescence reader over time. The volume and amount of cells per well were optimized for this assay. After screening, the killing action was monitored with small amounts of the parent peptide Bac2A (2 µg/ml of free peptide). Control experiments demonstrated that the decrease in luminescence (Fig. 1A) reflected bacterial death as assessed by the loss of colony forming ability (Fig. 1B)

Forty-nine scrambled peptides (Bac002-Bac050) were synthesized on the cellulose support and then cleaved by ammonia gas resulting in free peptides with an amidated C-terminus including the linker. The peptide spots were transferred to a 96-well microtiter plate and incubated for 30 min to dissociate the peptides from the cellulose. After serial dilution and addition of strain H1001, luminescence was recorded in a time dependent manner (Fig. 1). All 50 scrambled peptides were compared to Bac2A (RLARIVVIRVAR-NH2; SEQ ID NO: 1) by this assay (see Table 2). Each of the peptides showed some killing activity against P. aeruginosa strain H1001 at the highest concentration, in contrast to the unrelated negative-control peptide (GATPEDLNQKLS; SEQ ID NO: 321). Only two peptides showed decreased activity at the highest concentration. This data indicates that the ability of the short peptides to kill Pseudomonas aeruginosa strain is determined by the general properties of amino acid domains rather than the composition of the amino acids that was the same for each peptide. Further since all scrambled peptides had some activity, a sequence-specific interaction of the peptides with a receptor or target could be excluded.

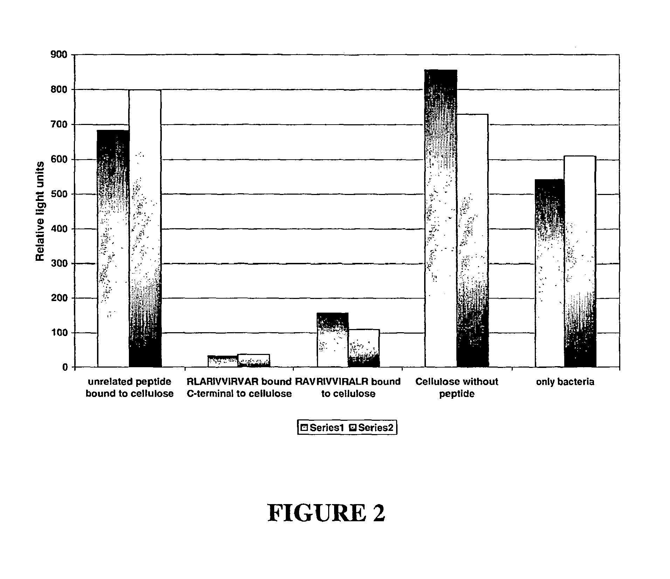

The same set of peptides, as well as the reversed sequence of Bac2A-R (RAVRIVVIRALR; SEQ ID NO: 2166), were synthesized on cellulose and left uncleaved. The production of light was initially compared in wells containing spots of Bac2A, Bac2A-R (with the reversed sequence of Bac2A), the unrelated negative-control peptide, or, as additional controls, only cellulose, or only bacteria (Fig. 2). The reversed Bac2A sequence showed only a slightly decreased ability to inhibit light production, while the control wells demonstrated no activity in decreasing luminescence.

To rule out the possibility that the non-cleaved peptides were being released from the membrane during the assay, a similar assessment of killing with four separate peptide spots (two containing Bac2A and two containing the unrelated peptide) was reiterated 10 times. Thus the peptide spots, after addition to the wells of the microtiter plate, were incubated through 10 consecutive identical cycles comprising exposure for 40 h to bacteria, followed by 14 h of exposure to 96% ethanol and 4 h of washing with Tris-HCl, pH 7.5 buffer. For each cycle, very similar inhibition of light production (killing) was observed. This is strong evidence that the observed killing is not a result of cleavage of the peptides from the membrane.

The complete set of scrambled peptides was measured in one experiment in a 96-well plate with the results shown in Table 3. The data shows that uncleaved cellulose bound peptides are able to kill P. aeruginosa strain H1001. As a control, the negative control (unrelated) peptide Bac429 and parent peptide Bac2A (= Bac001) were used in several different wells of the microtiter plate. Luminescence (relative light units; RLU) was measured at 14 different time points, 9 of which are shown. To permit comparison of the data with the free peptide data set, the result for each peptide was classified into six different groups, like the data set with the free peptides (Table 2). As shown in Table 2, measurement of the killing activity of scrambled peptides with soluble and bound peptides is listed according to relative activity classes of the free peptides and compared to Bac2. For the soluble peptides the following classes were established:

Class 1: Peptides showing stronger killing than Bac2A at a 4 to 8-fold higher dilution;

Class 2: Peptides showing stronger killing compared to that of Bac2A at a 4-fold higher dilution;

Class 3: Peptides similar to Bac2A;

Class 4: Peptides similar to Bac2A at the highest concentration but demonstrating weaker killing at a 2-fold dilution;

Class 5: Peptides giving very weak or no killing at a 2-fold dilution; and

Class 6: Peptides with weak killing activity at the highest concentration.

For the uncleaved peptides the following classes were established:

Class 1: Peptides reducing luminescence (lux) values to less than 500 after 40 min.;

Class 2: Peptides reducing lux values to between 500 and 1000 after 40 min.;

Class 3: Peptides similar to Bac2A resulting in lux values of less than 500 after 240 min.;

Class 4: Peptides reducing luminescence (lux) values to between 500 to 1499 after 240 min.;

Class 5: Peptides reducing luminescence (lux) values to between 1500 to 3000 after 240 min.; and

Class 6: Peptides having residual luminescence (lux) values greater than 3000 after 240 min.

The correlation of the numbers within the classification groups of bound and soluble peptides variants was strong with only 14 peptides (28%) demonstrating differences of 2 classes or more between the bound and soluble forms. However there was a general trend amongst all of the peptides towards decreasing activity for the bound versions. Only two free peptides were classified into group 6 compared to 10 bound peptides. The opposite was true in classes 1 and 2 for which there were reduced numbers of bound peptides. Only three bound peptides were more active than Bac2A in contrast to 10 free peptides, possibly due to restrictions on the conformational freedom of these C-terminally bound peptides.

An analysis was performed in an attempt to understand the basis for differing activity of the scrambled peptides. No correlation was observed between the sequence and the activity, and no sub-sequences corresponding to improved activity were observed. This result strengthens the concept that there are no sequence-specific interactions involved in killing bacteria. Computational analysis was therefore performed to identify any general structural features which might correlate with activity, using a series of descriptors of peptide structure. There was no correlation between the position of arginines in the peptides and the activity. A good correlation between activity and the existence of a hydrophobic patch of 5, or more, consecutive hydrophobic amino acid residues was determined.

Five of the most active Class 1 (free) peptides and two of the least active Class 6 (free) peptides were chosen to confirm the screening methods and to permit more detailed investigations. In addition, the parent peptide Bac2A was included in these studies as a standard. The minimal inhibitory concentrations (MIC) were determined for the important medical pathogens *Pseudomonas aeruginosa, Escherichia coli, Salmonella typhimurium, Staphylococcus aureus, Staphylococcus epidennidis, Enterococcus faecaelis*, and the yeast *Candida albicans* in Mueller-Hinton media. The measured MIC values (averages of three independent measurements are shown in Tables 4a and 4b) for this broader spectrum of organisms reflected very well the classification of the free peptides as recorded in Table 2. All class 1 peptides showed lower MICs (better activity) than Bac2A, with the best being about seven times more active. The two class 6 peptides showed an MIC that was at least 5 times higher (worse) than that for Bac2A. Two of the peptides from class 1 demonstrated low solubility in water and were not used for further investigations. The MICs for *P. aeruginosa* exposed to high and low Mg2+ conditions demonstrated the same 4-fold difference in MIC for both Bac2A and the class 1 peptides (Table 5).

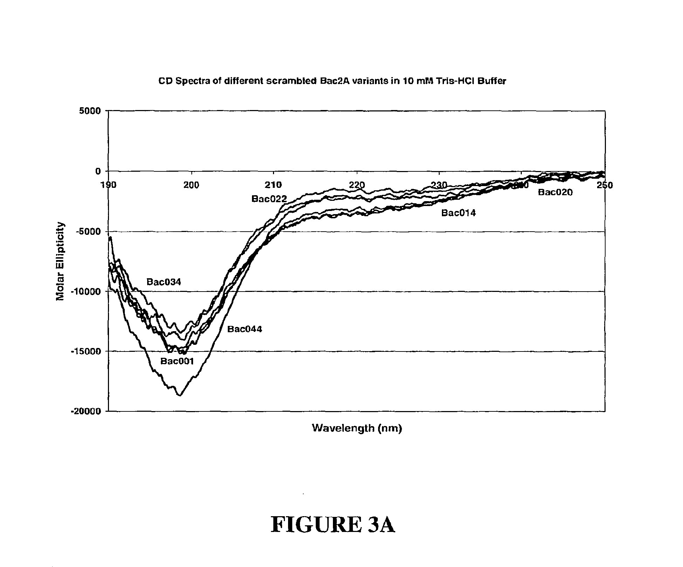

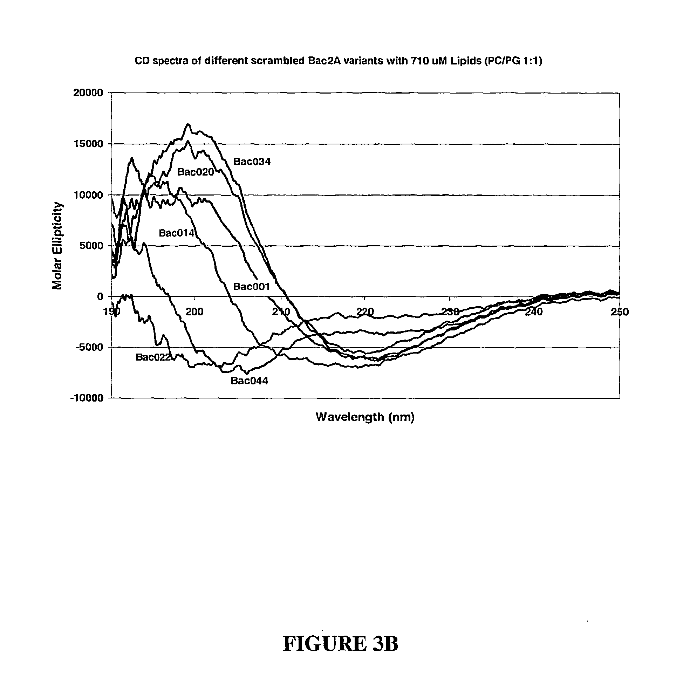

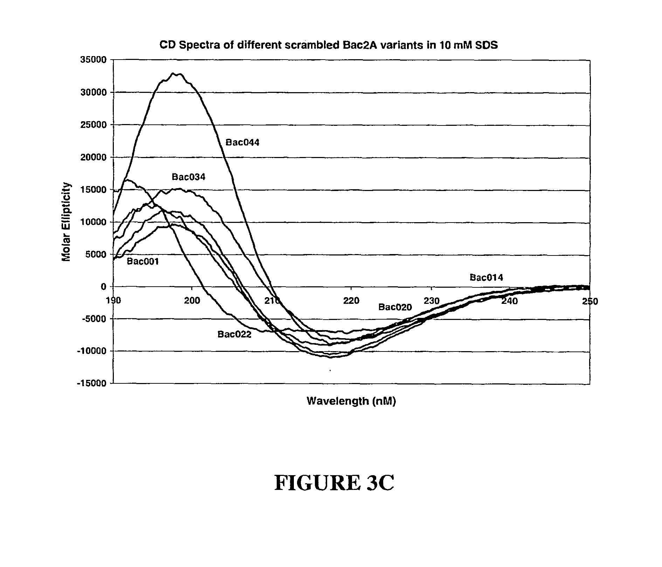

To determine if the activity of the scrambled peptide variants reflected structural differences, CD spectroscopic measurements were performed in Tris buffer, with SDS and with liposomes (Fig. 3A-C). All of the peptides had a typical random structure in Tris buffer. In the membrane-mimicking detergent SDS, the peptides showed spectra with characteristics of β-sheet conformations. The only exception was peptide 44 (class 6), which displayed a spectra that was more helical in nature. As SDS is an artificial membrane mimetic experiments were performed using liposomes, to directly assess the influence of lipids on structure. In liposomes, the Class 1 peptides again demonstrated spectra typical of β-structures, whereas both Class 6 peptides displayed more unstructured spectral characteristics. This suggests that those peptides with low antimicrobial activity are less able to interact with membranes resulting in structural changes. This strongly suggests that these structural attributes are correlated with the antimicrobial activity.

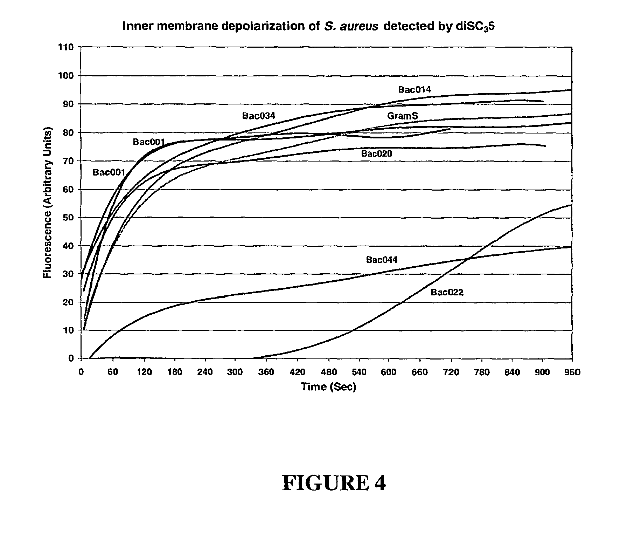

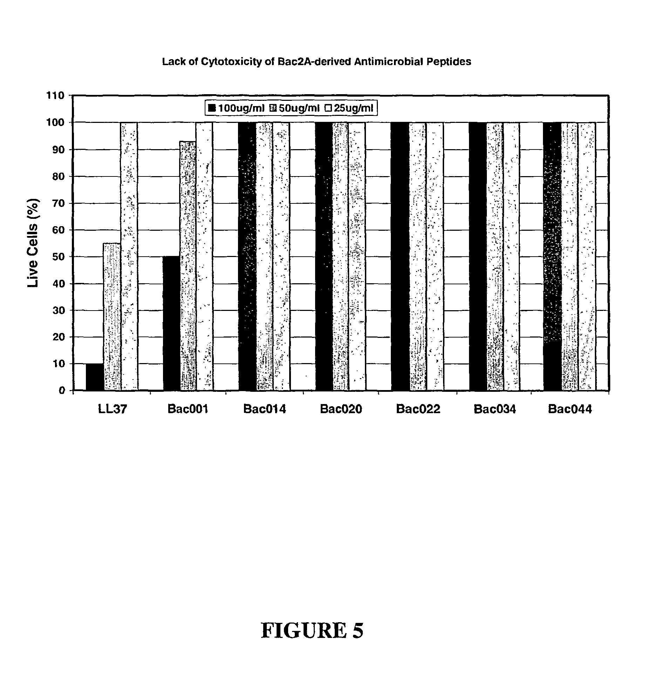

The ability to depolarize the membrane of Gram-positive bacteria can be monitored by the release of the fluorescent dye diSC35 (Fig. 4). The peptide Bac2A and all Class 1 peptides were able to depolarize the membrane of *S. aureus* very quickly, with similar rates as gramicidin S, see Fig. 4. In contrast, the Class 6 peptides showed a lower ability to depolarize the membrane. Cytotoxicity was assessed using PMA-treated, human macrophage THP-1 cell line (Fig. 5). All tested scrambled peptides showed no cytotoxicity and indeed a lower cytotoxic effect on THP-1 cells than either Bac2A or the human host defence peptide LL-37.

The best peptide variant, Bac020, was found to have a very low MIC (7 µg/ml) against P. aeruginosa and other bacteria, compared to the parent molecule Bac2A and had no cytotoxic effect at 100 µg/ml against a human macrophage cell line. The discovery of this and other improved peptide variants shows the advantage of using high-throughput solid-phase methodology in the discovery of antimicrobial peptides with improved activity.

**TABLE 2**

| **Peptide** | **Sequence** | **Activity Class** | | **Difference in Class** |
|---|---|---|---|---|
| | | **Soluble** | **bound** | **Soluble - Bound** |
| Bac006 | VRIRARRVILVA | 1 | 3 | -2 |
| Bac010 | AAVRRRVRLVII | 1 | 3 | -2 |
| Bac014 | RAVAVIIRLRRV | 1 | nd | |
| Bac020 | RRAAVVLIVIRR | 1 | 1 | 0 |
| Bac034 | VRLRIRVAVIRA | 1 | 3 | -2 |
| Bac043 | RVLIVIRARRVA | 1 | 3 | -2 |
| Bac008 | VLIRIRRVARAV | 2 | 5 | -3 |
| Bac016 | RARIVRVRVILA | 2 | 3 | -1 |
| Bac018 | RRVAIVVIARLR | 2 | 2 | 0 |
| Bac029 | IILAVRAVRRVR | 2.5 | 4 | -1.5 |
| Bac001 | RLARIVVIRVAR | 3 | 3 | 0 |
| Bac002 | RRIARVIVAVLR | 3 | 3 | 0 |
| Bac004 | IRARIAVRRVVL | 3 | 5 | -2 |
| Bac005 | IVRVAVALRRIR | 3 | 5 | -2 |
| Bac012 | IAARRLIRVVRV | 3 | 6 | -3 |
| Bac013 | VARIVVRLIRAR | 3 | 3 | 0 |
| Bac015 | AVRAIRVLRVIR | 3 | 4 | -1 |
| Bac025 | ARAILIRVVRRV | 3 | 3 | 0 |
| Bac026 | IARRIVAVRLRV | 3 | 3 | 0 |
| Bac042 | ILVIVRRRARAV | 3 | 3 | 0 |
| Bac045 | VIALRIAVRRVR | 3 | 3 | 0 |
| Bac046 | RRRVIVAVLARI | 3 | 3 | 0 |
| Bac047 | RVLIAARVIRRV | 3 | 5 | -2 |
| Bac049 | VIALVRARVRRI | 3 | 5 | -2 |
| Bac050 | RRVIAIAVRRLV | 3 | 6 | -3 |
| Bac007 | RRLVAIVAVRRI | 4 | 3 | 1 |
| Bac011 | AVRVRRRAILVI | 4 | 3 | 1 |
| Bac021 | ARIARRVRILVV | 4 | 3 | 1 |
| Bac024 | RAIIRRVLVRVA | 4 | 4 | 0 |
| Bac032 | ARRARIRILVVV | 4 | 3 | 1 |
| Bac036 | RARRVRVLIAIV | 4 | 3 | 1 |
| Bac037 | RAIRVRRIVLAV | 4 | 3 | 1 |
| Bac041 | RLRVAIVAIVRR | 4 | 2 | 2 |
| Bac048 | VVRALRRIIARV | 4 | 6 | -2 |
| Bac023 | RRRAIVRVVAIL | 4.5 | 3 | 1.5 |
| Bac030 | IVVRRRRAALVI | 4.5 | 6 | -1.5 |
| Bac003 | ARRLIVRVRVIA | 5 | 3 | 2 |
| Bac009 | IIRAALRRVRVV | 5 | 6 | -1 |
| Bac017 | VILARRRVRIAV | 5 | 3 | 2 |
| Bac019 | ILVARVIRRRVA | 5 | 5 | 0 |
| Bac028 | VIVRLAARRVRI | 5 | 6 | -1 |
| Bac031 | LAIVRRARVRIV | 5 | 6 | -1 |
| Bac033 | IRVRRLVAAVIR | 5 | 5 | 0 |
| Bac035 | RVLRVVRAAIRI | 5 | 5 | 0 |
| Bac038 | VVIRAAIRRVRL | 5 | 5 | 0 |
| Bac039 | RIVLRRAAVIRV | 5 | 6 | -1 |
| Bac040 | VLARVVARRIRI | 5 | 5 | 0 |
| Bac027 | RVLIARVVRAIR | 5.5 | 6 | -0.5 |
| Bac022 | ILRRVRVRAVAI | 6 | 5 | 1 |
| Bac044 | VIRRRRILAAVV | 6 | 6 | 0 |

**TABLE 3**

| **Peptide** | **Luminescence at the given time in minutes** | | | | | | | | |
|---|---|---|---|---|---|---|---|---|---|
| | **0** | **20** | **40** | **60** | **80** | **100** | **120** | **180** | **240** |
| **Bac429** | 4537 | 7275 | 6459 | 6149 | 6092 | 5799 | 5611 | 5656 | 5881 |
| **Bac001** | 4530 | 6411 | 1840 | 163 | 88 | 57 | 84 | 73 | 52 |
| **Bac002** | 4340 | 6090 | 1631 | 135 | 58 | 48 | 35 | 59 | 48 |
| **Bac003** | 4138 | 5992 | 3320 | 906 | 192 | 74 | 83 | 76 | 36 |
| **Bac004** | 3773 | 6547 | 5008 | 4405 | 3805 | 3125 | 2753 | 2459 | 2607 |
| **Bac005** | 3853 | 6844 | 5163 | 4287 | 3443 | 2254 | 1891 | 1665 | 1791 |
| **Bac006** | 3878 | 6829 | 4619 | 2393 | 867 | 469 | 277 | 286 | 306 |
| **Bac007** | 3943 | 7157 | 4403 | 2010 | 679 | 205 | 90 | 89 | 79 |
| **Bac008** | 3718 | 6695 | 5154 | 4426 | 3671 | 3164 | 2786 | 2703 | 2890 |
| **Bac009** | 3935 | 6553 | 4936 | 4512 | 3977 | 3488 | 3216 | 3270 | 3104 |
| **Bac010** | 4380 | 6719 | 4170 | 1628 | 562 | 129 | 121 | 99 | 56 |
| **Bac011** | 4516 | 7019 | 5176 | 3694 | 2076 | 1222 | 816 | 494 | 447 |
| **Bac429** | 4295 | 7478 | 6031 | 5770 | 5449 | 4779 | 4438 | 3939 | 3689 |
| **Bac001** | 4374 | 6729 | 2607 | 341 | 96 | 48 | 112 | 43 | 45 |
| **Bac012** | 4045 | 7171 | 5701 | 5037 | 4631 | 4418 | 4182 | 4344 | 5087 |
| **Bac013** | 3890 | 6850 | 3930 | 1584 | 494 | 193 | 111 | 100 | 66 |
| **Bac014** | 5233 | 8217 | 7835 | 7867 | 8100 | 8931 | 8956 | 10738 | 12876 |
| **Bac015** | 4309 | 7112 | 5227 | 3783 | 2656 | 1785 | 1398 | 1244 | 1320 |
| **Bac016** | 4172 | 6362 | 2916 | 678 | 142 | 80 | 60 | 68 | 22 |
| **Bac017** | 4219 | 7259 | 5041 | 3136 | 1907 | 1047 | 578 | 321 | 214 |
| **Bac018** | 4966 | 6080 | 981 | 82 | 44 | 60 | 62 | 50 | 39 |
| **Bac019** | 5239 | 7569 | 5731 | 4741 | 3822 | 3000 | 2373 | 1908 | 1570 |
| **Bac020** | 5285 | 4935 | 145 | 54 | 34 | 46 | 48 | 67 | 33 |
| **Bac021** | 5470 | 7247 | 4581 | 1967 | 683 | 278 | 196 | 129 | 128 |
| **Bac429** | 4593 | 7525 | 6900 | 6732 | 6701 | 6812 | 6872 | 7674 | 9094 |
| **Bac001** | 4041 | 6352 | 2249 | 244 | 118 | 35 | 59 | 34 | 52 |
| **Bac022** | 4336 | 7179 | 5180 | 4505 | 3675 | 3365 | 3044 | 2912 | 2966 |
| **Bac023** | 3958 | 7783 | 5656 | 3751 | 1827 | 761 | 329 | 123 | 79 |
| **Bac024** | 4164 | 7630 | 5586 | 4358 | 3256 | 2398 | 1727 | 1384 | 1373 |
| **Bac025** | 4354 | 6728 | 3660 | 1127 | 343 | 113 | 115 | 114 | 80 |
| **Bac026** | 4873 | 6389 | 4439 | 2341 | 1080 | 518 | 302 | 201 | 208 |
| **Bac027** | 4437 | 6695 | 4991 | 4421 | 4011 | 3545 | 3100 | 3182 | 3513 |
| **Bac028** | 5001 | 6828 | 5315 | 5037 | 4555 | 4143 | 3762 | 3646 | 4268 |
| **Bac029** | 4538 | 6664 | 5062 | 3963 | 2745 | 1799 | 1279 | 1012 | 942 |
| **Bac030** | 4618 | 7325 | 5921 | 5326 | 4981 | 4418 | 4087 | 3861 | 3679 |
| **Bac031** | 4646 | 7357 | 5808 | 5146 | 4145 | 3815 | 3472 | 3130 | 3247 |
| **Bac429** | 4337 | 7118 | 6593 | 6716 | 6273 | 6453 | 6210 | 6656 | 7935 |
| **Bac001** | 4234 | 6373 | 1840 | 169 | 73 | 50 | 48 | 42 | 20 |
| **Bac032** | 4649 | 6342 | 4584 | 2088 | 595 | 148 | 107 | 54 | 33 |
| **Bac033** | 4795 | 6539 | 4785 | 4155 | 3565 | 3095 | 2639 | 2613 | 2962 |
| **Bac034** | 5305 | 6449 | 4160 | 1454 | 390 | 157 | 64 | 60 | 63 |
| **Bac035** | 5199 | 6461 | 4989 | 4389 | 4001 | 3439 | 3162 | 2456 | 2577 |
| **Bac036** | 5208 | 7062 | 5114 | 3233 | 1182 | 394 | 215 | 129 | 107 |
| **Bac037** | 4908 | 6480 | 4105 | 1624 | 366 | 120 | 54 | 59 | 46 |
| **Bac038** | 5024 | 7158 | 5307 | 4676 | 4112 | 3323 | 2739 | 2466 | 2576 |
| **Bac039** | 4727 | 7074 | 5814 | 5262 | 4994 | 4717 | 4569 | 4524 | 5392 |
| **Bac040** | 4220 | 6241 | 4269 | 3951 | 3368 | 2880 | 2761 | 2300 | 2415 |
| **Bac041** | 4620 | 5674 | 615 | 54 | 68 | 37 | 77 | 53 | 37 |
| **Bac429** | 4761 | 7536 | 6318 | 6102 | 5996 | 6086 | 5899 | 6384 | 6991 |
| **Bac001** | 4425 | 6323 | 2721 | 413 | 151 | 53 | 75 | 85 | 40 |
| **Bac042** | 5171 | 6703 | 4453 | 2128 | 746 | 244 | 86 | 49 | 52 |
| **Bac043** | 5254 | 6385 | 3722 | 1189 | 313 | 159 | 67 | 84 | 52 |
| **Bac044** | 5592 | 7069 | 5932 | 5652 | 5206 | 5008 | 4999 | 5060 | 5195 |
| **Bac045** | 4873 | 6105 | 2726 | 554 | 83 | 61 | 37 | 57 | 35 |
| **Bac046** | 4408 | 5904 | 2345 | 233 | 76 | 70 | 102 | 48 | 53 |
| **Bac047** | 4733 | 6747 | 5100 | 4770 | 4031 | 3383 | 3012 | 2829 | 2615 |
| **Bac048** | 5001 | 6505 | 5163 | 4406 | 3824 | 3470 | 3423 | 3268 | 3694 |
| **Bac049** | 4926 | 7239 | 5219 | 4106 | 3188 | 2524 | 2059 | 1687 | 1794 |
| **Bac050** | 4803 | 6715 | 5157 | 4430 | 3915 | 3428 | 3246 | 3206 | 3590 |
| **None** | 5322 | 7699 | 6664 | 6607 | 6523 | 6427 | 6273 | 6902 | 6960 |

**TABLE 4a**

| **Peptide** | **MIC (mg/ml)** | | | |
|---|---|---|---|---|
| | **Gram Negative Bacteria** | | | **Yeast** |
| | ***P.aeruginosa*** | ***E. coli*** | ***S. typhimurium*** | ***C. albicans*** |
| Bac2A | 50 | 17 | 34 | 9 |
| Bac006 | 28 | 12 | N.D. | N.D. |
| Bac014 | 33 | 4 | 11 | 8 |
| Bac020 | 7 | 2 | 18 | 11 |
| Bac022 | 250 | 38 | 272 | 272 |
| Bac034 | 10 | 1 | 6 | 8 |
| Bac043 | 40 | 7 | N.D. | N.D. |
| Bac044 | >250 | 97 | 113 | 136 |

**TABLE 4b**

| **Peptide** | **MIC (mg/ml)** | | |
|---|---|---|---|
| | **Gram Positive Bacteria** | | |
| | ***S. aureus*** | ***S. epidermidis*** | ***E. faecalis*** |
| Bac2A | 17 | 4 | 17 |
| Bac006 | 28 | N.D. | N.D. |
| Bac014 | 13 | 8 | 11 |
| Bac020 | 5 | 1.5 | 8 |
| Bac022 | 125 | 136 | 272 |
| Bac034 | 5 | 4 | 11 |
| Bac043 | 23 | N.D. | N.D. |
| Bac044 | 250 | 113 | >272 |

**TABLE 5**

| Peptide | Soluble Class | MIC (µg/ml) *P. aeruginosa* | | |
|---|---|---|---|---|
| | | Muller Hinton Media | With 10 µM Mg | With 2 mM Mg |
| Bac020 | 1 | 7 | 2 | 8 |
| Bac034 | 1 | 10 | 4 | 16 |
| Bac014 | 1 | 33 | 16 | 63 |
| Bac2A | 3 | 54 | 18 | 73 |
| Bac022 | 6 | 250 | 250 | >250 |
| Bac044 | 6 | >250 | 250 | >250 |

### EXAMPLE 3

### Truncation Experiments

Although the specific set of active peptides differed among the free and bound peptides, it is apparent that activity can be correlated with the presence of a "hydrophobic patch". The specific location of this patch (e.g., N-terminal, C-terminal or central to the sequence) was not important in either the free or bound peptide activity models, but N-terminal hydrophobicity was of central importance in the explaining the difference in activity between the soluble and bound peptides.

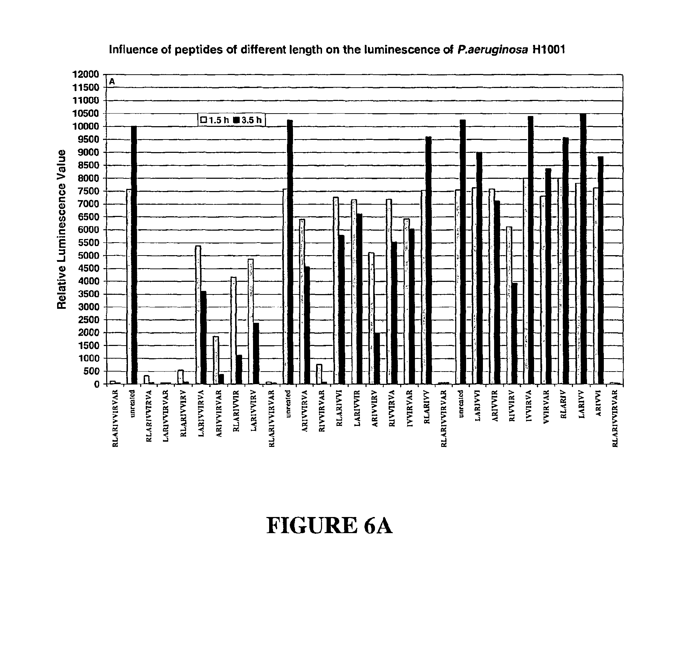

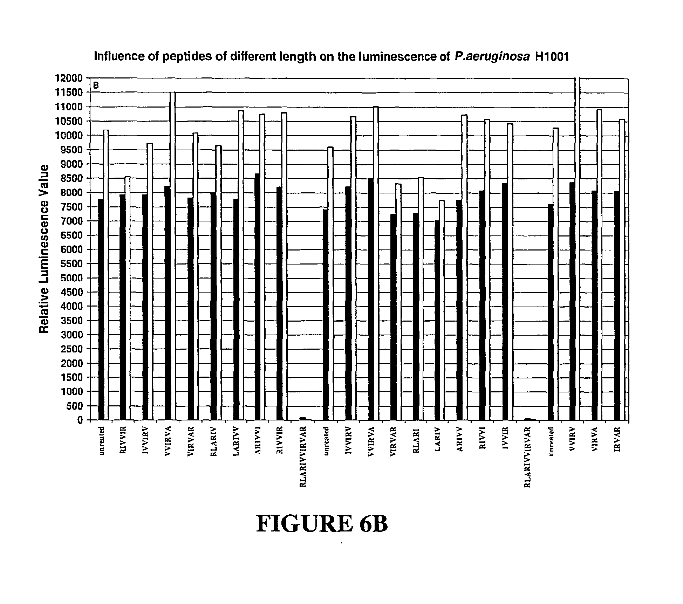

These conclusions were further evaluated in the context of another experiment in which the Bac2A peptide was gradually truncated and activity similarly assessed (Fig. 6). In this case, the primary predictor of activity across all the truncated enzymes (sequence length 5-12 amino acids), was the length of the peptide. No peptides of less than 8 amino acids were active in this assay (Figs. 6A and 6B). Further analysis of the peptides > 8 amino acids (10 peptides) revealed a dependence on the presence of arginine within the peptide. Measurement of MICs of these peptides against our panel of strains (Table 6) revealed moderate MICs that were generally higher (demonstrating weaker activity) than that of the parent peptide Bac2A.

### EXAMPLE 4

### Complete Substitution Analysis

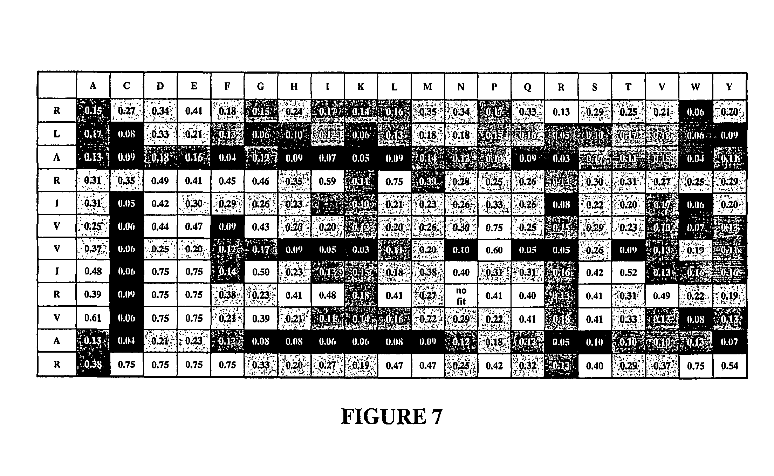

To analyze the positional importance of the specific amino acids in Bac2A, each amino acid was changed to the other 19 amino acids one-by-one, creating a total of 229 unique peptides. Activity was assessed in the *lux*-based assay (Fig. 7). The results revealed definite positional specificity of particular amino acids and many substitutions that improved the activity of Bac2A. Referring to the grid shown in Fig. 7, the top row provides the amino acid substituted for the natural residue of the parent peptide given at the start of each row. Thus, for example, the peptide in the upper left hand corner (row 1 column 1) is ALARIVVIRVAR (SEQ ID NO: 93), and the next peptide in row 1, column 2 is RCARIVVIRVAR (SEQ ID NO: 113), and so forth, whereas the peptide in row 2, column 1 is RAARIVVIRVAR (SEQ ID NO: 112), then in row 3, column 1 the unmutated Bac2A peptide RLARIVVIRVAR (SEQ ID NO: 1), in row 4, column 1 the peptide is RLAAIVVIRVAR (SEQ ID NO: 150), and so forth. The results are the residual luminescence after treatment of the *lux* reporter strain H1001 with peptide for 1 minute. Results are color coded as black = superior activity to the parent peptide Bac2A = Bac001; dark grey with white lettering = equivalent activity to the parent peptide Bac001; light grey with black lettering = inferior activity to the parent peptide Bac2A = Bac001; white = very little activity.

The most favored residues were:
AA1 = W
AA2 = C, G, H, K, R, S, W, Y
AA3 = C, F, H, I, K, L, Q, R, W
AA4 = no improvement on R
AA5 = C, R, W
AA6 = C, F, W
AA7 = C, H, I, K, N, Q, R, T
AA8=C
AA9 = C
AA 10 = C, W
AA11 = C, G, H, I, K, L, M, R, S, Y
AA12 = no improvement on R

It is clear that some amino acids particularly C>W>R>H,K are often preferred to the parent residue. In contrast, some residues never led to an improvement in activity, namely A, D, E, P, and V. Overall substitutions were rarely conservative and predictable just from the obvious substitution of e.g. one hydrophobic residue for another. Some positions were particularly rich candidates for substitution, namely positions 2,3,7, and 11.

Regarding unfavorable substitutions, the least favorable substitutions were:
AA1 = E
AA2 = none
AA3 = none
AA4 = D, E, F, G, I, L
AA5 = D
AA6 = D, E, G, P
AA7 = P
AA8 = 1, D, E, G, N, S, T
AA9 = I, D, E, H, I, L, N, P, Q, S, V
AA10 = I, D, E, G, Q, S
AA11 = none
AA12 = C, D, E, F, L, M, P, S, W, Y

Generally speaking those positions with the most favorable substitutions (AA2, AA3, AA7, and AA11) were the most flexible and had the least number of unfavorable substitutions. Five positions did not readily accept substitutions namely AA9, AA12, AA8, AA10 and AA4, and for these the parent (Bac2A) amino acid was often the best residue.

A number of these peptides were then synthesized and tested against a panel of strains (Table 6). These MIC measurements bore out the data from the spot synthesis results. Peptides shown to be better in the lux assay generally had superior MICs against the whole panel of bacteria although for 3 peptides lack of solubility was an issue. Conversely predicted poor substitutions led to peptides that had a lower effectiveness against all bacteria (e.g., the V7P and I9F substitutions).

To see if this information could be used for improved design of peptides, new peptides were made that combined 2 to 6 of the favorable amino acid substitutions (Table 6). Dramatic improvements in activity were observed over all other peptides studied, particularly in two of the peptides that developed very broad spectrum activity.

Interestingly, the same information could be used for the truncated peptides, in this case starting from an insoluble (n.s.) 8-mer truncation derivative (SEQ ID NO: 68), substitution of 3-4 residues led to peptides with activity equal to or better than Bac2A (Table 6). Activity of substitution peptides and truncation variants for the bacteria *P. aeruginosa* (*P.aerug*), *E. coli,* S. Typhimurium, *S. Typhim, S. aureus, S. epidermidis* (*S. epi*), and *E. faecalis*, and the yeast *C. albicans* is shown in Table 6.

**TABLE 6**

| **Peptide** | **MIC (**µ**g/ml)** | | | | | | |
|---|---|---|---|---|---|---|---|
| | ***P. aerug*** | ***E. coli*** | ***S. Typhim.*** | ***S. aureus*** | **S. *epi.*** | ***E.faecalis*** | ***C. albicans*** |
| **Bac2A** | 50 | 17 | 34 | 17 | 4 | 17 | 9 |
| **Bac206** | 125 | 16 | 63 | 63 | 16 | 31 | 125 |
| **Bac225** | 31 | 8 | 31 | 31 | 16 | 31 | 16 |
| **Bac233** | 8 | 4 | 8 | 16 | 2 | 16 | 8 |
| **Bac252** | 8 | 4 | 8 | 8 | 2 | 8 | 16 |
| **Bac241** | n.s. | n.s. | n.s. | n.s. | n.s. | n.s. | n.s. |
| **Bac256** | 8 | 2 | 8 | 2 | 0.5 | 2 | 8 |
| **Bac263** | n.s. | n.s. | n.s. | n.s. | n.s. | n.s. | n.s. |
| **Bac290** | 63 | 16 | 31 | 63 | 16 | 125 | 125 |
| **Bac297** | n.s. | n.s. | n.s. | n.s. | n.s. | n.s. | n.s. |
| **Bac327** | >250 | 250 | >250 | >250 | >250 | >250 | >250 |
| **Bac323** | 31 | 16 | 63 | 125 | 31 | 125 | 31 |
| **Bac338** | >250 | >250 | >250 | >250 | >250 | >250 | >250 |
| **Bac289** | 16 | 4 | 8 | 8 | 2 | 4 | 8 |
| **Bac404** | 16 | 8 | 8 | 8 | 2 | 8 | 8 |
| **Bac415** | 63 | 16 | 63 | 63 | 31 | 31 | 63 |
| **Bac430** | 2 | 0.5 | 4 | 2 | 0.2 | 4 | 4 |
| **Bac431** | 2 | 4 | 8 | 2 | 0.5 | 2 | 4 |
| **Bac432** | 31 | 4 | 16 | 8 | 1 | 8 | 31 |
| **Bac433** | 8 | 2 | 8 | 8 | 2 | 4 | 31 |
| **Bac101** | 63 | 8 | 31 | 63 | 16 | 31 | 63 |
| **Bac102** | 83 | 42 | >83 | 83 | 11 | 46 | 83 |
| **Bac103** | 46 | 23 | 46 | 46 | 11 | 23 | 23 |
| **Bac109** | n.s. | n.s. | n.s. | n.s. | n.s. | n.s. | n.s. |
| **Bac112** | n.s. | n.s. | n.s. | n.s. | n.s. | n.s. | n.s. |
| **Bac118** | n.s. | n.s. | n.s. | n.s. | n.s. | n.s. | n.s. |
| **Bac434** | 16 | 8 | 31 | 16 | 4 | 16 | 16 |
| **Bac435** | 16 | 4 | 16 | 4 | 4 | 8 | 16 |
| **Bac436** | 8 | 2 | 8 | 2 | 2 | 2 | 8 |
| **Bac437** | 250 | 16 | 31 | 16 | 8 | 32 | 63 |

From the foregoing description, various modifications and changes in the compositions and methods will occur to those skilled in the art. All such modifications coming within the scope of the appended claims are intended to be included therein. Each recited range includes all combinations and sub-combinations of ranges, as well as specific numerals contained therein.

All publications and patent documents cited above are hereby incorporated by reference in their entirety for all purposes to the same extent as if each were so individually denoted.

Although the foregoing invention has been described in detail by way of example for purposes of clarity of understanding, it will be apparent to the artisan that certain changes and modifications are comprehended by the disclosure and can be practiced without undue experimentation within the scope of the appended claims, which are presented by way of illustration not limitation.

### Preferred embodiments

1. An isolated antimicrobial peptide having 8 to 12 amino acids, wherein the peptide has an amino acid sequence of SEQ ID NOS: 1-2166, or analogs, derivatives, amidated variations and conservative variations thereof.
2. An isolated polynucleotide that encodes a peptide of embodiment 1.
3. The peptide of embodiment 1 comprising any contiguous sequence of amino acids having the formula: R₁-L₂-A₃-R₄-I₅-V₆-V₇-I₈-R₉-V₁₀-A₁₁-R₁₂, wherein R₁ = R or W; L₂ = L, C, G, H, K, R, S, W, or Y; A₃ = A, C, F, H, I, K, L, Q, R, or W; I₅ =1, C, R, or W; V₆ =V, C, F, or W; V₇ = V, C, H, I, K, N, Q, R, or T; I₈ = I or C; R₉ =R or C; V₁₀ = V, C, or W; A₁₁ = A, C, G, H, I, K, L, M, R, S, or Y, and derivatives, substitutions, deletions and additions thereof.
4. The peptide of embodiment 1, wherein the peptide has an amino acid sequence having the formula: AA₁ - AA₂ - AA₃ - AA₄ - AA₅ - AA₆ - AA₇ - AA₈ - AA₉ - AA₁₀ - AA₁₁ - AA₁₂, wherein AA₁ = A, G, I, K, L, P, R, or W; AA₂ = any residue except D, E, M, or N; AA₃ = any residue; AA₄ = K, M, or R; AA₅ = C, I, K, R, V, or W; AA₆ = C, F, K, R, V, W, or Y; AA₇ = C, F, G, H, I, K, L, N, Q, R, T, V, or Y; AA₈ = C, F, I, K, R, V, W, or Y; AA₉ = C, K, or R; AA₁₀ = C, I, K, L, R, V, W, or Y; AA₁₁ = any residue except D, E, or P; AA₁₂ = A, or R, and derivatives, substitutions, deletions and additions thereof.
5. The peptide of embodiment 1, wherein the peptide has a sequence of 8 amino acids having the formula: AA₁ AA2 - AA₃ - V - I - AA₆ - AA₇ - R, wherein AA₁ = K or R; AA₂ = I or R; AA₃ = W or V; AA₆ = R or W; and AA₇ = R or W.
6. A polypeptide X₁- A -X₂ or a functional variant or mimetic thereof, wherein A represents at least one peptide having an amino acid sequence of SEQ ID NOS: 1-2166 or analogs, derivatives, amidated variations and conservative variations thereof; and wherein each X₁ and X₂ independently of one another represents any amino acid sequence of n amino acids, n varying from 0 to 50, and n being identical or different in X₁ and X₂.
7. The polypeptide of embodiment 6 wherein the functional variant or mimetic is a conservative amino acid substitution or peptide mimetic substitution.
8. The polypeptide of embodiment 7 wherein the functional variant has about 70% or greater amino acid identity to X₁ - A - X₂.
9. The polypeptide of embodiment 6 wherein n is zero.
10. A method of inhibiting the growth of bacteria comprising contacting the bacteria with an inhibiting effective amount of a peptide having an amino acid sequence of SEQ ID NOS: 2-2166, or any combination thereof, or analogs, derivatives, amidated variations and conservative variations thereof, with the proviso that the peptide having an amino acid sequence of SEQ ID NO: 1 is only used in combination with any peptide having an amino acid sequence of SEQ ID NO: 2-2166.
11. The method of embodiment 10, wherein the bacteria is Gram positive.
12. The method of embodiment 11, wherein the bacteria is *Staphylococcus aureus, Staphylococcus epidermidis*, or *Enterococcus faecaelis*.
13. The method of embodiment 10, wherein the bacteria Gram negative.
14. The peptide of embodiment 13, wherein the bacteria is *Pseudomonas aeruginosa, Escherichia coli*, or *Salmonella enteritidis* ssp Typhimurium.
15. The method of embodiment 10, wherein the contacting comprises a peptide in combination with at least one antibiotic or lysozome.
16. The method of embodiment 15, wherein the antibiotic is selected from the group consisting of aminoglycosides, penicillins, cephalosporins, carbapenems, monobactams, quinolones, tetracyclines, and glycopeptides.
17. The method of embodiment 16, wherein the antibiotic is selected from the group consisting of amikacin, gentamicin, kanamycin, netilmicin, tobramycin, streptomycin, azithromycin, clarithromycin, erythromycin, erythromycin estolate/ethyl- succinate/gluceptate/lactobionate/stearate, penicillin G, penicillin V, methicillin, nafcillin, oxacillin, cloxacillin, dicloxacillin, ampicillin, amoxicillin, ticarcillin, carbenicillin, mezlocillin, azlocillin, piperacillin, cephalothin, cefazolin, cefaclor, cefamandole, cefoxitin, cefuroxime, cefonicid, cefmetazole, cefotetan, cefprozil, loracarbef, cefetamet, cefoperazone, cefotaxime, ceftizoxime, ceftriaxone, ceftazidime, cefepime, cefixime, cefpodoxime, cefsulodin, imipenem, aztreonam, fleroxacin, nalidixic acid, norfloxacin, ciprofloxacin, ofloxacin, enoxacin, lomefloxacin, cinoxacin, doxycycline, minocycline, tetracycline, vancomycin, chloramphenicol, clindamycin, trimethoprim, sulfamethoxazole, nitrofurantoin, rifampin and mupirocin and teicoplanin.
18. The method of embodiment 10, wherein the peptide is covalently bound to a solid support.
19. The method of embodiment 18, wherein the solid support is a medical device.
20. A method for identifying an antimicrobial peptide having 8 to 12 amino acids comprising contacting a test peptide with a microbe under conditions sufficient for antimicrobial activity, and detecting a change in growth or proliferation of the microbe as compared to the growth or proliferation of the microbe prior to the contacting.
21. The method of embodiment 20, wherein the test peptide is synthesized in a multi-spot format on a solid support.
22. The method of embodiment 20, wherein the test peptide is covalently bound to a solid support.
23. The method of embodiment 22, wherein the test peptide retains antimicrobial activity when cleaved from the solid support.
24. The method of embodiment 20, wherein the test peptide has a sequence of 12 amino acids including a consecutive stretch of 5 or more hydrophobic amino acid residues.
25. The method of embodiment 20, wherein the microbe is a Gram negative bacteria.
26. The method of embodiment 25, wherein the Gram negative bacteria is *Pseudomonas aeruginosa, Escherichia coli*, or *Salmonella enteritidis ssp* Typhimurium.
27. The method of embodiment 20, wherein the microbe is a Gram positive bacteria.
28. The method of embodiment 27, wherein the bacteria is *Staphylococcus aureus, Staphylococcus epidermidis*, or *Enterococcus faecaelis*.
29. The method of embodiment 20, wherein the microbe is a yeast.
30. The method of embodiment 29, wherein the yeast is *Candida albicans*.
31. The method of embodiment 20, wherein the microbe contains a reporter system.
32. The method of embodiment 31, wherein the reporter system is a bacterial luciferase construct inserted into the chromosome.
33. The method of embodiment 32, wherein the reporter system is inserted into *fliC* gene in *Pseudomonas aeruginosa.*
34. A pharmaceutical composition comprising a peptide of embodiment 1 or a polypeptide of embodiment 6 and a pharmaceutically acceptable carrier.
35. A method of modulating microbial activity in a subject, comprising administering to the subject a therapeutically effective amount of a pharmaceutical composition comprising the pharmaceutical composition of embodiment 34.
36. A method of treating a disease or disorder associated with microbial activity in a subject comprising administering to the subject a therapeutically effective amount of the pharmaceutical composition of embodiment 34.

## Claims

1. An isolated antimicrobial peptide comprising any contiguous sequence of amino acids having the sequence of amino acids having the formula: AA1 - AA2 - AA3 - AA4 - AA5 - AA6 - AA7 - AA8 - AA9 - AA10 - AA11 - AA12, wherein AA1 = A, G, I, K, L, P, R, or W; AA2 = any residue except D, E, M, or N; AA3 = any residue; AA4 = K, M, or R; AA5 = C, I, K, R, V, or W; AA6 = C, F, K, R, V, W, or Y; AA7 = C, F, G, H, I, K, L, N, Q, R, T, V, or Y; AA8 = C, F, I, K, R, V, W, or Y; AA9 = C, K, or R; AA10 = C, I, K, L, R, V, W, or Y; AA11 = any residue except D, E, or P; AA12 = A, or R, and derivatives, substitutions, deletions and additions thereof.

2. The peptide of claim 1, wherein the peptide is selected from the group consisting of RRARIVVIRVAR, RLRRIVVIRVAR, RLWRIVVIRVAR, RLARIVVIRVRR, RRWRIVVIRVRR, RRWKIVVIRWRR and RLRRIVVIRVRR.

3. The peptide of any preceding claim, wherein the peptide has an amino acid sequence of SEQ ID NO: 125, 144, 148, 296, 322, 323 or 325, or analogs, derivatives, amidated variations and conservative variations thereof.

4. A method of inhibiting the growth of bacteria comprising contacting the bacteria with an inhibiting effective amount of a peptide according to any one of the preceding claims, or any combination thereof, or analogs, derivatives, amidated variations and conservative variations thereof.

5. The method of claim 4, wherein the contacting comprises a peptide in combination with at least one antibiotic or lysozome.

6. The method of claim 5, wherein the antibiotic is selected from the group consisting of aminoglycosides, penicillins, cephalosporins, carbapenems, monobactams, quinolones, tetracyclines, and glycopeptides.

7. The method of claim 6, wherein the antibiotic is selected from the group consisting of amikacin, gentamicin, kanamycin, netilmicin, tobramycin, streptomycin, azithromycin, clarithromycin, erythromycin, erythromycin estolate/ethylsuccinate/gluceptate/lactobionate/stearate, penicillin G, penicillin V, methicillin, nafcillin, oxacillin, cloxacillin, dicloxacillin, ampicillin, amoxicillin, ticarcillin, carbenicillin, mezlocillin, azlocillin, piperacillin, cephalothin, cefazolin, cefaclor, cefamandole, cefoxitin, cefuroxime, cefonicid, cefmetazole, cefotetan, cefprozil, loracarbef, cefetamet, cefoperazone, cefotaxime, ceftizoxime, ceftriaxone, ceftazidime, cefepime, cefixime, cefpodoxime, cefsulodin, imipenem, aztreonam, fleroxacin, nalidixic acid, norfloxacin, ciprofloxacin, ofloxacin, enoxacin, lomefloxacin, cinoxacin, doxycycline, minocycline, tetracycline, vancomycin, chloramphenicol, clindamycin, trimethoprim, sulfamethoxazole, nitrofurantoin, rifampin and mupirocin and teicoplanin.

8. The method of claim 4, wherein the peptide is covalently bound to a solid support.

9. The method of claim 8, wherein the solid support is a medical device.

10. The method of any one of claims 4 to 9, wherein the microbe is a Gram negative bacteria.

11. The method of claim 10, wherein the Gram negative bacteria is *Pseudomonas aeruginosa, Escherichia coli, or Salmonella enteritidis ssp Typhimurium.*

12. The method of any one of claims 4 to 9, wherein the microbe is a Gram positive bacteria.

13. The method of claim 12, wherein the bacteria is *Staphylococcus aureus, Staphylococcus epidermidis, or Enterococcus faecaelis.*

14. A pharmaceutical composition comprising a peptide of any one of claims 1 to 3 and a pharmaceutically acceptable carrier.

15. The pharmaceutical composition of claim 14 for modulating microbial activity in a subject or treating a disease or disorder associated with microbial activity.
